# EUROPEAN PATENT APPLICATION

(11) **EP 1 561 818 A1**
(43) Date of publication of application: **10.08.2005**
(21) Application number: 05006787.5
(22) Date of filing: 28.12.2000
(51) Int. Cl.: C12N 15/12, C07K 14/705, G01N 33/68, A01K 67/027

(54) **Nucleic acids and polypeptides of drosophila melanogaster SNF sodium-neurotransmitter symporter family cell surface receptors and methods of use**

(30) Priority: 30.12.1999 US 173929 P; 15.03.2000 US 189399 P; 23.03.2000 US 191688 P; 23.03.2000 US 191687 P; 23.03.2000 US 191686 P; 23.03.2000 US 191695 P
(62) Divisional of application: 00989575.6
(71) Applicant: Genoptera, LLC, South San Francisco, CA 94083-0511 (US)
(72) Inventor: KELLERMAN, Kathryn A., Pacifica, CA 94044 (US); KEEGAN, Kevin Patrick, San Leandro, CA 94579 (US); EBENS, Allen James, Jr., San Francisco, CA 94122 (US)
(74) Representative: Grünecker, Kinkeldey, Stockmair & Schwanhäusser Anwaltssozietät

(57) **Abstract**

The invention provides isolated invertebrate symporter cell surface receptor nucleic acid molecules of the sodium/neurotransmitter family (SNF), and proteins encoded thereby. The subject nucleic acid and protein can be used to genetically modify metazoan invertebrate organisms, such as insects and worms, or cultured cells, resulting in expression or mis-expression of a subject protein. The genetically modified organisms or cells can be sed in screening assays to identify candidate compounds which are potential pesticidal agents or therapeutics that interact with a subject protein. They can also be used in methods for studying activity of a subject protein and identifying other genes that modulate the function of, or interact with, a subject gene.

## Description

### BACKGROUND OF THE INVENTION

The sodium/neurotransmitter family (SNF) of symporter cell surface receptors is implicated in neuroregulation and brain development. These molecules are targets of antidepressant medicines and other drugs *(e.g.* amphetamines, cocaine). Most members of this family function in the brain to terminate neurotransmitter activity by mediating neurotransmitter re-uptake into pre-synaptic terminals (Reizer *et al*., Biochim. Biophys. Acta (1994) 1197:133-136). The SNF family also includes members that function outside the brain, orphan transporters of unknown function, and hypothetical transporters from bacteria. Solutes transported by members of this family include serotonin, GABA, noradrenaline, dopamine, glycine, possibly proline, acetylcholine, choline, taurine, betaine and creatine.

The NTT4 subclass of SNFs is comprised of orphan receptors most closely related to serotonin porters. In rat, the timing and localizatian of NTT4 expression suggests a role in central nervous system maturation (Jursky and Nelson, J. Neurosci. Res. (1999) 55(1):24-35). Others members of the SNF family include K⁺-coupled amino acid transporters from insects. These transporters are related to members of the sodium/neurotransmitter symporter family (SNF), but transport is driven by K⁺ and H⁺ gradients rather than Na⁺ and H⁺ gradients. Lepidopteran insect larvae have a high K⁺ and a low Na⁺ content, and their midgut cells lack Na⁺/K⁺ ATPase. Instead, an H⁺ translocating, vacuolar-type ATPase generates a voltage of approximately -240 mV across the apical membrane of the gut goblet cells, which drives H⁺ back into the cells in exchange for K⁺, resulting in net K⁺ secretion into the lumen. The resulting inwardly directed K⁺ electrochemical gradient serves as a driving force for active amino acid uptake into adjacent columnar cells. The KAAT1 gene was cloned from a *Manduca sexta* larval midgut library using expression in *Xenopus laevis* oocytes. It is expressed in absorptive columnar cells of the midgut and in labial glands (Castagna *et al*., Proc Natl Acad Sci USA (1998) 95:5395-5400).

In addition to synaptic transport, the transporters are responsible for re-uptake of neurotransmitters. For example, the *Manduca sexta* GABA transporter, MasGAT, has been shown to be pharmacologically distinct from known mammalian GABA transporters (Mbungu et al., *Arch Biochem Biophys* 1995, 318:489-497). A similar gene, TmGAT, has been cloned from the Cabbage looper, *Trichoplusia ni* (Gao,X. et al., *Insect Biochem Mol Biol* 1999 Jul;29(7):609-23).

Members of the SNF family are 600-730 amino acid proteins sharing a common structure: 12 putative transmembrane (TM) domains with a hydrophillic extracellular loop between the third and fourth TM domains. They share significant sequence similarity, particularly in the putative membrane-spanning domains.

Pesticide development has traditionally focused on the chemical and physical properties of the pesticide itself, a relatively time-consuming and expensive process. As a consequence, efforts have been concentrated on the modification of pre-existing, well-validated compounds, rather than on the development of new pesticides. There is a need in the art for new pesticidal compounds that are safer, more selective, and more efficient than currently available pesticides. The present invention addresses this need by providing novel pesticide targets from invertebrates such as the fruit fly *Drosophila melanogaster*, and by providing methods of identifying compounds that bind to and modulate the activity of such targets.

### SUMMARY OF THE INVENTION

It is an object of the invention to provide isolated insect nucleic acids and proteins that are targets for pesticides. The isolated insect nucleic acid molecules provided herein are useful for producing insect proteins encoded thereby. The insect proteins are useful in assays to identify compounds that modulate a biological activity of the proteins, which assays identify compounds that may have utility as pesticides. It is an object of the present invention to provide invertebrate homologs of SNF genes that can be used in genetic screening methods to characterize pathways that such genes may be involved in, as well as other interacting genetic pathways. It is also an object of the invention to provide methods for screening compounds that interact with a subject SNF. Compounds that interact with a subject SNF may have utility as therapeutics or pesticides. In some embodiments, the subject proteins are SNFs from *Drosophila melanogaster*; particular embodiments are referred to herein as dmSNF, dmSNF2, and dmSNF3. In some embodiments, the subject proteins are members of the NTT4 subclass of SNFs. In particular embodiments, the subject proteins are NTT4 proteins from *Drosophila melanogaster*, referred to herein as dmNTT4. In other embodiments, the subject proteins are K⁺-coupled amino acid transporters. In particular embodiments, the subject proteins are K⁺-coupled amino acid transporters from *Drosophila melanogaster*, referred to herein as dmKSNF. In other embodiments, the subject proteins are γ-amino butyric acid (GABA) transporters (GAT). In particular embodiments, the subject proteins are GAT from *Drosophila melanogaster*, referred to herein as dmGAT.

These and other objects are provided by the present invention which concerns the identification and characterization of novel pesticide targets in insects, e.g., *Drosophila melanogaster*. Isolated nucleic acid molecules are provided that comprise nucleic acid sequences encoding subject proteins as well as novel fragments and derivatives thereof.

The present application provides an isolated nucleic acid molecule of less than about 15 kb in size comprising a nucleic acid sequence that encodes an invertebrate receptor polypeptide and that shares at least about 75% nucleotide sequence identity with the sequence set forth in any one of SEQ ID NOS : 1, 3, 5, 7, 9, and 11, or the complement thereof. The present application further provides an isolated nucleic acid molecule comprising a nucleotide sequence that encodes a polypeptide having 100% sequence identity with any one of SEQ ID NOS : 2, 4, 6, 8, 10, and 12.

Methods of using the isolated nucleic acid molecules and fragments of the invention as biopesticides are described, such as use of RNA interference methods that block a biological activity of a subject protein. Vectors and host cells comprising the subject nucleic acid molecules are also described, as well as metazoan invertebrate organisms *(e.g.* insects, coelomates and pseudocoelomates) that are genetically modified to express or mis-express a subject protein.

The present application provides a process for producing an invertebrate symporter cell surface receptor protein, comprising culturing a host cell comprising an expression vector comprising the nucleic acid molecules of the present invention under conditions suitable for expression of said protein ; and recovering said protein.

An important utility of the subject nucleic acids and proteins is that they can be used in screening assays to identify candidate compounds which are potential pesticidal agents or therapeutics that interact with subject proteins. Such assays typically comprise contacting a subject protein or fragment with one or more candidate molecules, and detecting any interaction between the candidate compound and the subject protein. The assays may comprise adding the candidate molecules to cultures of cells genetically engineered to express subject proteins, or alternatively, administering the candidate compound to a metazoan invertebrate organism genetically engineered to express a subject protein.

The genetically engineered metazoan invertebrate animals of the invention can also be used in methods for studying a biological activity of a subject protein. These methods typically involve detecting the phenotype caused by the expression or mis-expression of the subject protein. The methods may additionally comprise observing a second animal that has the same genetic modification as the first animal and, additionally has a mutation in a gene of interest. Any difference between the phenotypes of the two animals identifies the gene of interest as capable of modifying the function of the gene encoding the subject protein. The methods may additionally comprise administering one or more candidate compounds to said animal or its progeny and observing any changes in invertebrate symporter cell surface receptor protein activity of said animal or its progeny.

### DETAILED DESCRIPTION OF THE INVENTION

### Overview

The use of invertebrate model organism genetics and related technologies can greatly facilitate the elucidation of biological pathways (Scangos, Nat. Biotechnol. (1997) 15:1220-1221; Margolis and Duyk, *supra*). Of particular use is the insect model organism, *Drosophila melanogaster* (hereinafter referred to generally as "*Drosophila*"). An extensive search for sodium neurotransmitter family (SNF) of cell surface receptor nucleic acids and their encoded proteins in *Drosophila* was conducted in an attempt to identify new and useful tools for probing the function and regulation of the SNF genes, and for use as targets in pesticide and drug discovery.

Novel SNF nucleic acids, and the encoded proteins are identified herein. The newly identified subject nucleic acid can be used for the generation of mutant phenotypes in animal models or in living cells that can be used to study regulation of genes encoding the subject proteins, and the use of subject proteins as a pesticide or drug target Due to the ability to rapidly carry out large-scale, systematic genetic screens, the use of invertebrate model organisms such as *Drosophila* has great utility for analyzing the expression and mis-expression of a subject protein. Thus, the invention provides a superior approach for identifying other components involved in the synthesis, activity, and regulation of subject proteins. Systematic genetic analysis of SNFs using invertebrate model organisms can lead to the identification and validation of pesticide targets directed to components of a pathway in which a subject protein is involved. Model organisms or cultured cells that have been genetically engineered to express a subject protein can be used to screen candidate compounds for their ability to modulate expression or activity of a subject nucleic acid and/or protein, and thus are useful in the identification of new drug targets, therapeutic agents, diagnostics and prognostics useful in the treatment of disorders associated with ion channels. Additionally, these invertebrate model organisms can be used for the identification and screening of pesticide targets directed to components of pathways involving subject proteins.

The details of the conditions used for the identification and/or isolation of novel nucleic acids and proteins of the invention are described in the Examples section below. Various non-limiting embodiments of the invention, applications and uses of these novel subject genes and proteins are discussed in the following sections. The entire contents of all references, including patent applications, cited herein are incorporated by reference in their entireties for all purposes. Additionally, the citation of a reference in the preceding background section is not an admission of prior art against the claims appended hereto.

For the purposes of the present application, singular forms "a", "and", and "the" include plural referents unless the context clearly indicates otherwise. Thus, for example, reference to "an invertebrate receptor" includes large numbers of receptors, reference to "an agent" includes large numbers of agents and mixtures thereof, reference to "the method" includes one or more methods or steps of the type described herein.

### Definitions

As used herein the term "isolated" is meant to describe a polynucleotide, a polypeptide, an antibody, or a host cell that is in an environment different from that in which the polynucleotide, the polypeptide, the antibody, or the host cell naturally occurs. As used herein, the term "substantially purified" refers to a compound (e.g., either a polynucleotide or a polypeptide or an antibody) that is removed from its natural environment and is at least 60% free, preferably 75% free, and most preferably 90% free from other components with which it is naturally associated.

The terms "polypeptide" and "protein", used interchangeably herein, refer to a polymeric form of amino acids of any length, which can include coded and non-coded amino acids, chemically or biochemically modified or derivatized amino acids, and polypeptides having modified peptide backbones. The term includes fusion proteins, including, but not limited to, fusion proteins with a heterologous amino acid sequence, fusions with heterologous and homologous leader sequences, with or without N-terminal methionine residues; immunologically tagged proteins; and the like.

A "host cell", as used herein, denotes microorganisms or eukaryotic cells or cell lines cultured as unicellular entities which can be, or have been, used as recipients for recombinant vectors or other transfer polynucleotides, and include the progeny of the original cell which has been transfected. It is understood that the progeny of a single cell may not necessarily be completely identical in morphology or in genomic or total DNA complement as the original parent, due to natural, accidental, or deliberate mutation.

By "transformation" is meant a permanent or transient genetic change induced in a cell following incorporation of new DNA (i.e., DNA exogenous to the cell). Genetic change can be accomplished either by incorporation of the new DNA into the genome of the host cell, or by transient or stable maintenance of the new DNA as an episomal element. Where the cell is a eukaryotic cell, a permanent genetic change is generally achieved by introduction of the DNA into the genome of the cell.

### ISOLATED NUCLEIC ACID MOLECULES OF THE INVENTION

The invention provides isolated insect nucleic acid molecules comprising nucleotide sequences of SNFs, and more particularly SNF nucleic acid sequences *of Drosophila,* and methods of using these nucleic acid molecules.

The present invention provides isolated nucleic acid molecules that comprise nucleotide sequences encoding insect proteins that are potential pesticide targets. The isolated nucleic acid molecules have a variety of uses, e.g., as hybridization probes, e.g., to identify nucleic acid molecules that share nucleotide sequence identity; in expression vectors to produce the polypeptides encoded by the nucleic acid molecules; and to modify a host cell or animal for use in assays described hereinbelow.

Thus, the term "isolated nucleic acid sequence", as used herein, includes the reverse complement, RNA equivalent, DNA or RNA single- or double-stranded sequences, and DNA/RNA hybrids of the sequence being described, unless otherwise indicated.

The terms "polynucleotide" and "nucleic acid molecule", used interchangeably herein, refer to a polymeric forms of nucleotides of any length, either ribonucleotides or deoxynucleotides. Thus, this tem includes, but is not limited to, single-, double-, or multi-stranded DNA or RNA, genomic DNA, cDNA, DNA-RNA hybrids, or a polymer comprising purine and pyrimidine bases or other natural, chemically or biochemically modified, non-natural, or derivatized nucleotide bases. The backbone of the polynucleotide can comprise sugars and phosphate groups (as may typically be found in RNA or DNA), or modified or substituted sugar or phosphate groups. Alternatively, the backbone of the polynucleotide can comprise a polymer of synthetic subunits such as phosphoramidites and thus can be an oligodeoxynucleoside phosphoramidate or a mixed phosphoramidate-phosphodiester oligomer. Peyrottes et al. (1996) *Nucl. Acids Res.* 24:1841-1848; Chaturvedi et al. (1996) *Nucl. Acids Res.* 24:2318-2323. A polynucleotide may comprise modified nucleotides, such as methylated nucleotides and nucleotide analogs, uracyl, other sugars, and linking groups such as fluororibose and thioate, and nucleotide branches. The sequence of nucleotides may be interrupted by non-nucleotide components. A polynucleotide may be further modified after polymerization, such as by conjugation with a labeling component. Other types of modifications included in this definition are caps, substitution of one or more of the naturally occurring nucleotides with an analog, and introduction of means for attaching the polynucleotide to proteins, metal ions, labeling components, other polynucleotides, or a solid support.

For hybridization probes, it may be desirable to use nucleic acid analogs, in order to improve the stability and binding affinity. A number of modifications have been described that alter the chemistry of the phosphodiester backbone, sugars or heterocyclic bases.

Among useful changes in the backbone chemistry are phosphorothioates; phosphorodithioates, where both of the non-bridging oxygens are substituted with sulfur; phosphoroamidites; alkyl phosphotriesters and boranophosphates. Achiral phosphate derivatives include 3=-O=-5=-S-phosphorothioate, 3=-S-5=-O-phosphorothioate, 3=-CH2-5=-O-phosphonate and 3=-NH-5=-O-phosphoroamidate. Peptide nucleic acids replace the entire phosphodiester backbone with a peptide linkage.

Sugar modifications are also used to enhance stability and affinity. The α-anomer of deoxyribose may be used, where the base is inverted with respect to the natural β-anomer. The 2'-OH of the ribose sugar may be altered to form 2'-O-methyl or 2'-O-allyl sugars, which provides resistance to degradation without compromising affinity. Modification of the heterocyclic bases must maintain proper base pairing. Some useful substitutions include deoxyuridine for deoxythymidine; 5-methyl-2'-deoxycytidine and 5-bromo-2'-deoxycytidine for deoxycytidine. 5- propynyl-2'-deoxyuridine and 5-propynyl-2'-deoxycytidine have been shown to increase affinity and biological activity when substituted for deoxythymidine and deoxycytidine, respectively.

In addition to the fragments and derivatives of SEQ ID NOS:1, 3, 5, 7, 9, and 11, as described in detail below, the invention includes the reverse complements thereof. Also, the subject nucleic acid sequences, derivatives and fragments thereof may be RNA molecules comprising the nucleotide sequence of SEQ ID NOS:1, 3, 5, 7, 9, and 11 (or derivative or fragment thereof) wherein the base U (uracil) is substituted for the base T (thymine). The DNA and RNA sequences of the invention can be single- or double-stranded. Thus, the term "isolated nucleic acid sequence", as used herein, includes the reverse complement, RNA equivalent, DNA or RNA single- or double-stranded sequences, and DNA/RNA hybrids of the sequence being described, unless otherwise indicated.

Fragments of the subject nucleic acid molecules can be used for a variety of purposes. Interfering RNA (RNAi) fragments, particularly double-stranded (ds) RNAi, can be used to generate loss-of-function phenotypes, or to formulate biopesticides (discussed further below). The subject nucleic acid fragments are also useful as nucleic acid hybridization probes and replication/amplification primers. Certain "antisense" fragments, i.e. that are reverse complements of portions of the coding sequence of any one of SEQ ID NOS:1, 3, 5, 7, 9, and 11 have utility in inhibiting the function of a subject protein. The fragments are of length sufficient to specifically hybridize with the corresponding any one of SEQ ID NOS:1, 3, 5, 7, 9, and 11. The fragments consist of or comprise at least 12, preferably at least 24, more preferably at least 36, and more preferably at least 96 contiguous nucleotides of any one of SEQ ID NOS:1, 3, 5, 7, 9, and 11. When the fragments are flanked by other nucleic acid sequences, the total length of the combined nucleic acid sequence is less than 15 kb, preferably less than 10 kb or less than 5kb, and more preferably less than 2 kb.

The subject nucleic acid sequences may consist solely of any one of SEQ ID NOS:1, 3, 5, 7, 9, and 11 or fragments thereof. Alternatively, the subject nucleic acid sequences and fragments thereof may be joined to other components such as labels, peptides, agents that facilitate transport across cell membranes, hybridization-triggered cleavage agents or intercalating agents. The subject nucleic acid sequences and fragments thereof may also be joined to other nucleic acid sequences (i.e. they may comprise part of larger sequences) and are of synthetic/non-natural sequences and/or are isolated and/or are purified, i.e. unaccompanied by at least some of the material with which it is associated in its natural state. Preferably, the isolated nucleic acids constitute at least about 0.5%, and more preferably at least about 5% by weight of the total nucleic acid present in a given fraction, and are preferably recombinant, meaning that they comprise a non-natural sequence or a natural sequence joined to nucleotide(s) other than that which it is joined to on a natural chromosome.

Derivative nucleic acid molecules of the subject nucleic acid molecules include sequences that hybridize to the nucleic acid sequence of any one of SEQ ID NOS:1, 3, 5, 7, 9, and 11 under stringency conditions such that the hybridizing derivative nucleic acid is related to the subject nucleic acid by a certain degree of sequence identity. A nucleic acid molecule is "hybridizable" to another nucleic acid molecule, such as a cDNA, genomic DNA, or RNA, when a single stranded form of the nucleic acid molecule can anneal to the other nucleic acid molecule. Stringency of hybridization refers to conditions under which nucleic acids are hybridizable. The degree of stringency can be controlled by temperature, ionic strength, pH, and the presence of denaturing agents such as formamide during hybridization and washing. As used herein, the term "stringent hybridization conditions" are those normally used by one of skill in the art to establish at least a 90% sequence identity between complementary pieces of DNA or DNA and RNA. "Moderately stringent hybridization conditions" are used to find derivatives having at least 70% sequence identity. Finally, "low-stringency hybridization conditions" are used to isolate derivative nucleic acid molecules that share at least about 50% sequence identity with the subject nucleic acid sequence.

The ultimate hybridization stringency reflects both the actual hybridization conditions as well as the washing conditions following the hybridization, and it is well known in the art how to vary the conditions to obtain the desired result. Conditions routinely used are set out in readily available procedure texts (*e*.*g*., Current Protocol in Molecular Biology, Vol. 1, Chap. 2.10, John Wiley & Sons, Publishers (1994); Sambrook *et al*., Molecular Cloning, Cold Spring Harbor (1989)). A preferred derivative nucleic acid is capable of hybridizing to any one of SEQ ID NOS:1, 3, 5, 7, 9, and 11 under stringent hybridization conditions that comprise: prehybridization of filters containing nucleic acid for 8 hours to overnight at 65° C in a solution comprising 6X single strength citrate (S SC) (1X SS C is 0.15 M NaCl, 0.015 M Na citrate; pH 7.0), 5X Denhardt's solution, 0.05% sodium pyrophosphate and 100 µg/ml herring sperm DNA; hybridization for 18-20 hours at 65° C in a solution containing 6X SSC, 1X Denhardt's solution, 100 µg/ml yeast tRNA and 0.05% sodium pyrophosphate; and washing of filters at 65° C for 1 h in a solution containing 0.2X SSC and 0.1% SDS (sodium dodecyl sulfate).

Derivative nucleic acid sequences that have at least about 70% sequence identity with any one of SEQ ID NOS:1, 3, 5, 7, 9, and 11 are capable of hybridizing to any one of SEQ ID NOS:1, 3, 5, 7, 9, and 11 under moderately stringent conditions that comprise: pretreatment of filters containing nucleic acid for 6 h at 40° C in a solution containing 35% formamide, 5X SSC, 50 mM Tris-HCl (pH 7.5), 5 mM EDTA, 0.1% PVP, 0.1% Ficoll, 1% BSA, and 500 µg/ml denatured salmon sperm DNA; hybridization for 18-20 h at 40° C in a solution containing 35% formamide, 5X SSC, 50 mM Tris-HCl (pH 7.5), 5 mM EDTA, 0.02% PVP, 0.02% Ficoll, 0.2% BSA, 100 µg/ml salmon sperm DNA, and 10% (wt/vol) dextran sulfate; followed by washing twice for 1 hour at 55° C in a solution containing 2X SSC and 0.1% SDS.

Other preferred derivative nucleic acid sequences are capable of hybridizing to any one of SEQ ID NOS:1, 3, 5, 7, 9, and 11 under low stringency conditions that comprise: incubation for 8 hours to overnight at 37° C in a solution comprising 20% formamide, 5 x SSC, 50 mM sodium phosphate (pH 7.6), 5X Denhardt's solution, 10% dextran sulfate, and 20 µg/ml denatured sheared salmon sperm DNA; hybridization in the same buffer for 18 to 20 hours; and washing of filters in 1 x SSC at about 37° C for 1 hour.

As used herein, "percent (%) nucleic acid sequence identity" with respect to a subject sequence, or a specified portion of a subject sequence, is defined as the percentage of nucleotides in the candidate derivative nucleic acid sequence identical with the nucleotides in the subject sequence (or specified portion thereof), after aligning the sequences and introducing gaps, if necessary to achieve the maximum percent sequence identity, as generated by the program WU-BLAST-2.0a19 (Altschul *et al*., J. Mol. Biol. (1997) 215:403-410; http://blast.wustl.edu/blast/README.html; hereinafter referred to generally as "BLAST") with all the search parameters set to default values. The HSP S and HSP S2 parameters are dynamic values and are established by the program itself depending upon the composition of the particular sequence and composition of the particular database against which the sequence of interest is being searched. A percent (%) nucleic acid sequence identity value is determined by the number of matching identical nucleotides divided by the sequence length for which the percent identity is being reported.

Derivatives of a subj ect nucleic acid molecule usually have at least 70% sequence identity, preferably at least 80% sequence identity, more preferably at least 85% sequence identity, still more preferably at least 90% sequence identity, and most preferably at least 95% sequence identity with any one of SEQ ID NOS:1, 3, 5, 7, 9, and 11, or domain-encoding regions thereof.

In one preferred embodiment, the derivative nucleic acid encodes a polypeptide comprising an amino acid sequence set forth in any one of SEQ ID NOS:2, 4, 6, 8, 10, and 12, or a fragment or derivative thereof as described further below. A derivative of a subject nucleic acid molecule, or fragment thereof, may comprise 100% sequence identity with any one of SEQ ID NOS:1, 3, 5, 7, 9, and 11, but be a derivative thereof in the sense that it has one or more modifications at the base or sugar moiety, or phosphate backbone. Examples of modifications are well known in the art (Bailey, Ullmann's Encyclopedia of Industrial Chemistry (1998), 6th ed. Wiley and Sons). Such derivatives may be used to provide modified stability or any other desired property.

Another type of derivative of the subject nucleic acid sequences includes corresponding humanized sequences. A humanized nucleic acid sequence is one in which one or more codons has been substituted with a codon that is more commonly used in human genes. Preferably, a sufficient number of codons have been substituted such that a higher level expression is achieved in mammalian cells than what would otherwise be achieved without the substitutions. Tables are available in the art that show, for each amino acid, the calculated codon frequency in humans genes for 1000 codons (Wada *et al*., Nucleic Acids Research (1990) 18(Suppl.):2367-2411). Similarly, other nucleic acid derivatives can be generated with codon usage optimized for expression in other organisms, such as yeasts, bacteria, and plants, where it is desired to engineer the expression of receptor proteins by using specific codons chosen according to the preferred codons used in highly expressed genes in each organism. Thus, a subject nucleic acid molecule in which the glutamic acid codon, GAA has been replaced with the codon GAG, which is more commonly used in human genes, is an example of a humanized nucleic acid molecule. A detailed discussion of the humanization of nucleic acid sequences is provided in U.S. Pat No. 5,874,304 to Zolotukhin *et al*.

Specific nucleic acid molecules of the invention are discussed in detail below.

### dmNTT4 Nucleic Acid Molecules

In some embodiments, the invention provides isolated nucleic acid molecules comprising nucleotide sequences encoding a dmNTT4 SNF. As described in the Examples below, a nucleic acid sequence (SEQ ID NO:1) was isolated from *Drosophila* that encodes an SNF homolog, hereinafter referred to as dmNTT4.

In some embodiments, a dmNTT4 nucleic acid molecule comprises a nucleotide sequence of at least about 50, at least about 75, at least about 100, at least about 150, at least about 200, at least about 300, at least about 400, at least about 500, at least about 600, at least about 700, at least about 800, at least about 900, at least about 1000, at least about 1100, at least about 1200, at least about 1300, at least about 1400, at least about 1500, at least about 1600, at least about 1700, at least about at least about 1800, at least about 1900, at least about 2000, at least about 2100, at least about 2200, at least about 2300 at least about 2400, at least about 2500, at least about 2600, at least about 2700, at least about 2800, at least about 2900, at least about 3000, at least about 3100, at least about 3200, at least about 3300, at least about 3400, at least about 3500, at least about 3600, at least about 3700, at least about 3800, or at least about 3900 contiguous nucleotides of the sequence set forth in SEQ ID NO:1, up to the entire sequence set forth in SEQ ID NO:1.

In other embodiments, a dmNTT4 nucleic acid molecule of the invention comprises a nucleotide sequence that encodes a polypeptide comprising at least about 6, at least about 10, at least about 20, at least about 50, at least about 75, at least about 100, at least about 150, at least about 200, at least about 250, at least about 300, at least about 350, at least about 400, at least about 450, at least about 500, at least about 550, at least about 600, at least about 650, or at least about 670 contiguous amino acids of the sequence set forth in SEQ ID NO:2, up to the entire amino acid sequence as set forth in SEQ ID NO:2.

Additional preferred fragments of SEQ ID NO:1 encode intracellular or extracellular domains, which are located at approximately nucleotides 239-412, 476-508, 572-631, 695-886, 950-967, 1031-1117, 1181-1222, 1286-1519, 1583-1657, 1721-1753, 1817-1891, 1955-2023, 2087-2263, respectively. Further additional preferred fragments encode the SNF domains, located at approximately nucleotides 403-1294, and 1423-2089. Another preferred fragment is the ORF located at approximately nucleotides 239-2263 of SEQ ID NO:1.

More specific embodiments of preferred dmNTT4 protein fragments and derivatives are discussed further below in connection with specific dmNTT4 proteins.

### dmKSNF Nucleic Acid Molecules

In some embodiments, the invention provides isolated nucleic acid molecules comprising nucleotide sequence encoding K⁺ coupled amino acid transporters of the SNF family of cell surface transporters, and more particularly SNF nucleic acid sequences *of Drosophila,* and methods of using these sequences. As described in the Examples below, a nucleic acid sequence (SEQ ID NO:3) was isolated from *Drosophila* that encodes an SNF homolog, hereinafter referred to as dmKSNF.

In some embodiments, a dmKSNF nucleic acid molecule comprises a nucleotide sequence of at least about 50, at least about 75, at least about 100, at least about 150, at least about 200, at least about 300, at least about 400, at least about 500, at least about 600, at least about 700, at least about 800, at least about 900, at least about 1000, at least about 1100, at least about 1200, at least about 1300, at least about 1400, at least about 1500, at least about 1600, at least about 1700, at least about at least about 1800, at least about 1900, at least about 2000, at least about 2100, at least about 2200, at least about 2300, or at least about 2400 contiguous nucleotides of the sequence set forth in SEQ ID NO:3, up to the entire sequence set forth in SEQ ID NO:3.

In other embodiments, a dmKSNF nucleic acid molecule of the invention comprises a nucleotide sequence that encodes a polypeptide comprising at least about 6, at least about 10, at least about 20, at least about 50, at least about 75, at least about 100, at least about 150, at least about 200, at least about 250, at least about 300, at least about 350, at least about 400, at least about 450, at least about 500, at least about 550, at least about 600, or at least about 630 contiguous amino acids of the sequence set forth in SEQ ID NO:4, up to the entire amino acid sequence as set forth in SEQ ID NO:4.

Additional preferred fragments of SEQ ID NO:3 encode extracellular or intracellular domains, which are located at approximately nucleotides 249-371, 435-461, 522-617, 681-938,1002-1019, 1083-1269,1233-1283,1347-1448,1512-1568,1632-1664,1730-1790,1854-1901, and 1965-2173.

More specific embodiments of preferred dmKSNF protein fragments and derivatives are discussed further below in connection with specific dmKSNF proteins.

### dmSNF Nucleic Acid Molecules

In some embodiments, the invention provides isolated nucleic acid molecules comprising nucleotide sequences of SNF transporters, and more particularly SNF transporter nucleic acid sequences of *Drosophila*, and methods of using these sequences. As described in the Examples below, a nucleic acid sequence (SEQ ID NO:5) was isolated from *Drosophila* that encodes a sodium neurotransmitter symporter (SNF) family homolog, hereinafter referred to as dmSNF.

In some embodiments, a dmSNF nucleic acid molecule comprises a nucleotide sequence of at least about 50, at least about 75, at least about 100, at least about 150, at least about 200, at least about 300, at least about 400, at least about 500, at least about 600, at least about 700, at least about 800, at least about 900, at least about 1000, at least about 1100, at least about 1200, at least about 1300, at least about 1400, at least about 1500, at least about 1600, at least about 1700, at least about at least about 1800, or at least about 1900, contiguous nucleotides of the sequence set forth in SEQ ID NO:5, up to the entire sequence set forth in SEQ ID NO:5.

In other embodiments, a dmSNF nucleic acid molecule of the invention comprises a nucleotide sequence that encodes a polypeptide comprising at least about 6, at least about 10, at least about 20, at least about 50, at least about 75, at least about 100, at least about 150, at least about 200, at least about 250, at least about 300, at least about 350, or at least about 400 contiguous amino acids of the sequence set forth in SEQ ID NO:6, up to the entire amino acid sequence as set forth in SEQ ID NO:6.

Additional preferred fragments of SEQ ID NO:5 encode the signature motif of the sodium neurotransmitter symporter (SNF) family which are located at approximately nucleotides 864-1139.

More specific embodiments of preferred dmSNF protein fragments and derivatives are discussed further below in connection with specific dmSNF proteins.

### dmSNF2 Nucleic Acid Molecules

In some embodiments, the invention provides isolated nucleic acid molecules comprising nucleotide sequences of SNF transporters , and more particularly SNF transporter nucleic acid sequences *of Drosophila,* and methods of using these sequences. As described in the Examples below, a nucleic acid sequence (SEQ ID NO:7) was isolated from *Drosophila* that encodes a sodium neurotransmitter symporter (SNF) family homolog, hereinafter referred to as dmSNF2.

In some embodiments, a dmSNF2 nucleic acid molecule comprises a nucleotide sequence of at least about 50, at least about 75, at least about 100, at least about 150, at least about 200, at least about 300, at least about 400, at least about 500, at least about 600, at least about 700, at least about 800, at least about 900, at least about 1000, at least about 1100, at least about 1200, at least about 1300, at least about 1400, at least about 1500, at least about 1600, at least about 1700, at least about at least about 1800, at least about 1900, or at least about 2000 contiguous nucleotides of the sequence set forth in SEQ ID NO:7, up to the entire sequence set forth in SEQ ID NO:7.

In other embodiments, a dmSNF2 nucleic acid molecule of the invention comprises a nucleotide sequence that encodes a polypeptide comprising at least about 6, at least about 10, at least about 20, at least about 50, at least about 75, at least about 100, at least about 150, at least about 200, at least about 250, at least about 300, at least about 350, at least about 400, at least about 500, at least about 550, or at least about 575 contiguous amino acids of the sequence set forth in SEQ ID NO:8, up to the entire amino acid sequence as set forth in SEQ ID NO:8.

Additional preferred fragments of SEQ ID NO:7 encode the signature motif of the sodium neurotransmitter symporter (SNF) family which is located at approximately nucleotides 99-1700. Other preferred fragments encode the transmembrane domains, which are located at approximately nucleotides 120 - 170, 210 - 260, 357 - 407, 624 - 674, 702 - 752, 849 - 899, 969 -1019, 1128 - 1178, 1242 - 1292, 1350 - 1400, 1482 - 1532, and 1563 - 1613.

More specific embodiments of preferred dmSNF2 protein fragments and derivatives are discussed further below in connection with specific dmSNF2 proteins.

### dmSNF3 Nucleic Acid Molecules

In some embodiments, the invention provides isolated nucleic acid molecules comprising nucleotide sequences of dmSNF3s, and more particularly dmSNF3 nucleic acid sequences of *Drosophila,* and methods of using these sequences. As described in the Examples below, a nucleic acid sequence (SEQ ID NO:9) was isolated from *Drosophila* that encodes a dmSNF3 homolog.

In some embodiments, a dmSNF3 nucleic acid molecule comprises a nucleotide sequence of at least about 50, at least about 75, at least about 100, at least about 150, at least about 200, at least about 300, at least about 400, at least about 500, at least about 600, at least about 700, at least about 800, at least about 900, at least about 1000, at least about 1100, at least about 1200, at least about 13 00, at least about 1400, at least about 1500, at least about 1600, at least about 1700, at least about at least about 1800, at least about 1900, at least about 2000, or at least about 2100 contiguous nucleotides of the sequence set forth in SEQ ID NO:9, up to the entire sequence set forth in SEQ ID NO:9.

In other embodiments, a dmSNF3 nucleic acid molecule of the invention comprises a nucleotide sequence that encodes a polypeptide comprising at least about 6, at least about 10, at least about 20, at least about 5 0, at least about 75, at least about 100, at least about 150, at least about 200, at least about 250, at least about 300, at least about 350, at least about 400, at least about 500, at least about 550, at least about 600, or at least about 625 contiguous amino acids of the sequence set forth in SEQ ID NO:10, up to the entire amino acid sequence as set forth in SEQ ID NO:10.

Additional preferred fragments of SEQ ID NO:9 encode the signature motif of the sodium neurotransmitter symporter (SNF) family which is located at approximately nucleotides 197-1843. Other preferred fragments encode the transmembrane domains, which are located at approximately nucleotides 208 - 268; 308 - 368; 458 - 518; 764 - 824; 854 - 914; 1112 - 1172; 1274 - 1334; 1391 - 1451; 1469 - 1529; 1613 - 1673; and 1739 - 1799.

More specific embodiments of preferred dmSNF3 protein fragments and derivatives are discussed further below in connection with specific dmSNF3 proteins.

### dmGAT Nucleic Acid Molecules

In some embodiments, the invention provides isolated nucleic acid molecules comprising nucleotide sequences of GABA transporters, and more particularly GABA transporter nucleic acid sequences *of Drosophila,* and methods of using these sequences. As described in the Examples below, a nucleic acid sequence (SEQ ID NO:11) was isolated from *Drosophila* that encodes a GABA transporter homolog, hereinafter referred to as dmGAT.

In some embodiments, a dmGAT nucleic acid molecule comprises a nucleotide sequence of at least about 50, at least about 75, at least about 100, at least about 150, at least about 200, at least about 300, at least about 400, at least about 500, at least about 600, at least about 700, at least about 800, at least about 900, at least about 1000, at least about 1100, at least about 1200, at least about 1300, at least about 1400, at least about 1500, at least about 1600, at least about 1700, at least about at least about 1800, at least about 1900, at least about 2000, at least about 2100, at least about 2200, at least about 2300 at least about 2400, at least about 2500, at least about 2600, at least about 2700, at least about 2800, at least about 2900, at least about 3000, at least about 3100, at least about 3200, at least about 3300, at least about 3400, at least about 3500, at least about 3600, at least about 3700, at least about 3 800, or at least about 3900 contiguous nucleotides of the sequence set forth in SEQ ID NO:11, up to the entire sequence set forth in SEQ ID NO:11.

In other embodiments, a dmGAT nucleic acid molecule of the invention comprises a nucleotide sequence that encodes a polypeptide comprising at least about 6, at least about 10, at least about 20, at least about 50, at least about 75, at least about 100, at least about 15 0, at least about 200, at least about 250, at least about 300, at least about 350, at least about 400, at least about 450, at least about 500, at least about 550, at least about 600, at least about 650, or at least about 670 contiguous amino acids of the sequence set forth in SEQ ID NO:12, up to the entire amino acid sequence as set forth in SEQ ID NO:12.

Additional preferred fragments of SEQ ID NO:11 encode the transmembrane domains, which are located at approximately nucleotides 293-355, 386-448, 554-616, 857-919, 929-991, 1082-1144, 1199-1261,1343-1405,1481-1543,1580-1642,1712-1774, and 1823-1885. Another preferred domain encodes the signature motif of the sodium neurotransmitter symporter (SNF) family, which is located at approximately nucleotides 278-1939.

More specific embodiments of preferred dmGAT protein fragments and derivatives are discussed further below in connection with specific dmGAT proteins.

### Isolation, Production, and Expression of Subject Nucleic Acid Molecules

The subject nucleic acid molecules, or fragments or derivatives thereof, may be obtained from an appropriate cDNA library prepared from any eukaryotic species that encodes a subject protein, such as vertebrates, preferably mammalian *(e.g.* primate, porcine, bovine, feline, equine, and canine species, *etc.*) and invertebrates, such as arthropods, particularly insects species (preferably *Drosophila*)*, acarids, crustacea, molluscs, nematodes*, and other worms. An expression library can be constructed using known methods. For example, mRNA can be isolated to make cDNA which is ligated into a suitable expression vector for expression in a host cell into which it is introduced. Various screening assays can then be used to select for the gene or gene product *(e.g.* oligonucleotides of at least about 20 to 80 bases designed to identify the gene of interest, or labeled antibodies that specifically bind to the gene product). The gene and/or gene product can then be recovered from the host cell using known techniques.

Polymerase chain reaction (PCR) can also be used to isolate a subject nucleic acid molecule, where oligonucleotide primers representing fragmentary sequences of interest amplify RNA or DNA sequences from a source such as a genomic or cDNA library (as described by Sambrook *et al*., *supra*). Additionally, degenerate primers for amplifying homologs from any species of interest may be used. Once a PCR product of appropriate size and sequence is obtained, it may be cloned and sequenced by standard techniques, and utilized as a probe to isolate a complete cDNA or genomic clone.

Fragmentary sequences of the subject nucleic acid molecules and derivatives thereof may be synthesized by known methods. For example, oligonucleotides may be synthesized using an automated DNA synthesizer available from commercial suppliers *(e.g.* Biosearch, Novato, CA; Perkin-Elmer Applied Biosystems, Foster City, CA). Antisense RNA sequences can be produced intracellularly by transcription from an exogenous sequence, *e.g.* from vectors that contain subject antisense nucleic acid sequences. Newly generated sequences may be identified and isolated using standard methods.

An isolated subject nucleic acid molecule can be inserted into any appropriate cloning vector, for example bacteriophages such as lambda derivatives, or plasmids such as PBR322, pUC plasmid derivatives and the Bluescript vector (Stratagene, San Diego, CA). Recombinant molecules can be introduced into host cells via transformation, transfection, infection, electroporation, *etc*., or into a transgenic animal such as a fly. The transformed cells can be cultured to generate large quantities of the subject nucleic acid. Suitable methods for isolating and producing the subject nucleic acid sequences are well known in the art (Sambrook *et al., supra;* DNA Cloning: A Practical Approach, Vol. 1, 2, 3, 4, (1995) Glover, ed., MRL Press, Ltd., Oxford, U.K.).

The nucleotide sequence encoding a subject protein or fragment or derivative thereof, can be inserted into any appropriate expression vector for the transcription and translation of the inserted protein-coding sequence. Alternatively, the necessary transcriptional and translational signals can be supplied by the native subject gene and/or its flanking regions. A variety of host-vector systems may be utilized to express the protein-coding sequence such as mammalian cell systems infected with virus (e.g. vaccinia virus, adenovirus, *etc.*)*;* insect cell systems infected with virus (*e.g*. baculovirus); microorganisms such as yeast containing yeast vectors, or bacteria transformed with bacteriophage, DNA, plasmid DNA, or cosmid DNA. Expression of a subject protein may be controlled by a suitable promoter/enhancer element. In addition, a host cell strain may be selected which modulates the expression of the inserted sequences, or modifies and processes the gene product in the specific fashion desired.

To detect expression of a subject gene product, the expression vector can comprise a promoter operably linked to a subject nucleic acid molecule, one or more origins of replication, and, one or more selectable markers *(e.g.* thymidine kinase activity, resistance to antibiotics, *etc*.). Alternatively, recombinant expression vectors can be identified by assaying for the expression of a subject gene product based on the physical or functional properties of a subject protein in in *vitro* assay systems (*e.g.* immunoassays).

A subject protein, fragment, or derivative may be optionally expressed as a fusion, or chimeric protein product (i.e. it is joined via a peptide bond to a heterologous protein sequence of a different protein). A chimeric product can be made by ligating the appropriate nucleic acid sequences encoding the desired amino acid sequences to each other in the proper coding frame using standard methods and expressing the chimeric product. A chimeric product may also be made by protein synthetic techniques, e.g. by use of a peptide synthesizer.

Once a recombinant that expresses a subject gene sequence is identified, the gene product can be isolated and purified using standard methods *(e.g.* ion exchange, affinity, and gel exclusion chromatography; centrifugation; differential solubility; electrophoresis). The amino acid sequence of the protein can be deduced from the nucleotide sequence of the chimeric gene contained in the recombinant and can thus be synthesized by standard chemical methods (Hunkapiller *et al*., Nature (1984) 310:105-111). Alternatively, native subject proteins can be purified from natural sources, by standard methods (*e.g*. immunoaffinity purification).

### ISOLATED POLYPEPTIDES OF THE INVENTION

The invention further provides isolated polypeptides comprising or consisting of an amino acid sequence of any of SEQ ID NOS:2, 4, 6, 8, 10, or 12, or fragments or derivatives thereof. Compositions comprising any of these proteins may consist essentially of a subject protein, fragments, or derivatives, or may comprise additional components (*e.g*. pharmaceutically acceptable carriers or excipients, culture media, carriers used in pesticide formulations, *etc*.).

A derivative of a subject protein typically shares a certain degree of sequence identity or sequence similarity with any one of SEQ ID NOS:2, 4, 6, 8, 10, or 12, or a fragment thereof. As used herein, "percent (%) amino acid sequence identity" with respect to a subject sequence, or a specified portion of a subject sequence, is defined as the percentage of amino acids in the candidate derivative amino acid sequence identical with the amino acid in the subject sequence (or specified portion thereof), after aligning the sequences and introducing gaps, if necessary to achieve the maximum percent sequence identity, as generated by BLAST (Altschul *et al*., *supra*) using the same parameters discussed above for derivative nucleic acid sequences. A % amino acid sequence identity value is determined by the number of matching identical amino acids divided by the sequence length for which the percent identity is being reported. "Percent (%) amino acid sequence similarity" is determined by doing the same calculation as for determining % amino acid sequence identity, but including conservative amino acid substitutions in addition to identical amino acids in the computation. A conservative amino acid substitution is one in which an amino acid is substituted for another amino acid having similar properties such that the folding or activity of the protein is not significantly affected. Aromatic amino acids that can be substituted for each other are phenylalanine, tryptophan, and tyrosine; interchangeable hydrophobic amino acids are leucine, isoleucine, methionine, and valine; interchangeable polar amino acids are glutamine and asparagine; interchangeable basic amino acids are arginine, lysine and histidine; interchangeable acidic amino acids are aspartic acid and glutamic acid; and interchangeable small amino acids are alanine, serine, threonine, cysteine, and glycine.

In some embodiments, a subject protein derivative shares at least 80% sequence identity or similarity, preferably at least 85%, more preferably at least 90%, and most preferably at least 95% sequence identity or similarity with a contiguous stretch of at least 25 amino acids, preferably at least 50 amino acids, more preferably at least 100 amino acids, and in some cases, the entire length of any one of SEQ ID NOS:2, 4, 6, 8, 10, or 12.

The fragment or derivative of a subject protein is preferably "functionally active" meaning that the subject protein derivative or fragment exhibits one or more functional activities associated with a full-length, wild-type subject protein comprising the amino acid sequence of any one of SEQ ID NOS:2, 4, 6, 8,10, or 12. As one example, a fragment or derivative may have antigenicity such that it can be used in immunoassays, for immunization, for inhibition of activity of a subject protein, *etc*, as discussed further below regarding generation of antibodies to subject proteins. Preferably, a functionally active fragment or derivative of a subject protein is one that displays one or more biological activities associated with a subject protein, such as transport, ion conductance, etc. For purposes herein, functionally active fragments also include those fragments that exhibit one or more structural features of a subject protein, such as transmembrane or SNF domains. The functional activity of the subject proteins, derivatives and fragments can be assayed by various methods known to one skilled in the art (Current Protocols in Protein Science (1998) Coligan *et al*., eds., John Wiley & Sons, Inc., Somerset, New Jersey). In a preferred method, which is described in detail below, a model organism, such as *Drosophila,* is used in genetic studies to assess the phenotypic effect of a fragment or derivative (i.e. a mutant of a subject protein).

A derivative of a subject protein can be produced by various methods known in the art. The manipulations which result in their production can occur at the gene or protein level. For example, a cloned subject gene sequence can be cleaved at appropriate sites with restriction endonuclease(s) (Wells *et al*., Philos. Trans. R Soc. London SerA (1986) 317:415), followed by further enzymatic modification if desired, isolated, and ligated *in vitro*, and expressed to produce the desired derivative. Alternatively, a subject gene can be mutated *in vitro* or *in vivo,* to create and/or destroy translation, initiation, and/or termination sequences, or to create variations in coding regions and/or to form new restriction endonuclease sites or destroy preexisting ones, to facilitate further in *vitro* modification. A variety of mutagenesis techniques are known in the art such as chemical mutagenesis, *in vitro* site-directed mutagenesis (*Carter et al*., Nucl. Acids Res. (1986) 13:4331), use of TAB® linkers (available from Pharmacia and Upjohn, Kalamazoo, MI), *etc.*

At the protein level, manipulations include post translational modification, e.g. glycosylation, acetylation, phosphorylation, amidation, derivatization by known protecting/blocking groups, proteolytic cleavage, linkage to an antibody molecule or other cellular ligand, *etc.* Any of numerous chemical modifications may be carried out by known technique *(e.g.* specific chemical cleavage by cyanogen bromide, trypsin, chymotrypsin, papain, V8 protease, NaBH₄, acetylation, formylation, oxidation, reduction, metabolic synthesis in the presence of tunicamycin, *etc.*)*.* Derivative proteins can also be chemically synthesized by use of a peptide synthesizer, for example to introduce nonclassical amino acids or chemical amino acid analogs as substitutions or additions into the subject protein sequence.

Chimeric or fusion proteins can be made comprising a subject protein or fragment thereof (preferably comprising one or more structural or functional domains of the subject protein) joined at its amino- or carboxy-terminus via a peptide bond to an amino acid sequence of a different protein. Chimeric proteins can be produced by any known method, including: recombinant expression of a nucleic acid encoding the protein (comprising an amino acid sequence encoding a subject protein joined in-frame to a coding sequence for a different protein); ligating the appropriate nucleic acid sequences encoding the desired amino acid sequences to each other in the proper coding frame, and expressing the chimeric product; and protein synthetic techniques, e.g. by use of a peptide synthesizer.

Specific subject proteins are discussed below.

### dmNTT4 Proteins

In some embodiments, the invention provides isolated dmNTT4 polypeptides. In some of these embodiments, isolated dmNTT4 polypeptides comprise or consist of an amino acid sequence of SEQ ID NO:2, or fragments or derivatives thereof.

In some embodiments, a dmNTT4 polypeptide of the invention comprises an amino acid sequence of at least about 6, at least about 10, at least about 20, at least about 50, at least about 75, at least about 100, at least about 150, at least about 200, at least about 250, at least about 300, at least about 350, at least about 400, at least about 450, at least about 500, at least about 550, at least about 600, at least about G50, or at least about 670 contiguous amino acids of the sequence set forth in SEQ ID NO:2, up to the entire amino acid sequence as set forth in SEQ ID NO:2.

In other embodiments, a dmNTT4 protein derivative may consist of or comprise a sequence that shares 100% similarity with any contiguous stretch of at least 35 amino acids, preferably at least 37 amino acids, more preferably at least 40 amino acids, and most preferably at least 45 amino acids of SEQ ID NO:2. Preferred derivatives of dmNTT4 consist of or comprise an amino acid sequence that has at least 80%, preferably at least 85%, more preferably at least 90%, and most preferably at least 95% sequence identity or sequence similarity with any of amino acid residues 55-352, or 395-617, which code for the putative SNF domains, or residues 153-216, which code for the extracellular loop. Preferred fragments of dmNTT4 proteins consist or comprise at least 15, preferably at least 17, more preferably at least 20, and most preferably at least 25 contiguous amino acids of SEQ ID NO:2.

### dmKSNF Proteins

In some embodiments, the invention provides isolated dmKSNF polypeptides. In some of these embodiments, isolated dmKSNF proteins of the invention comprise or consist of an amino acid sequence of SEQ ID NO:4, or fragments or derivatives thereof.

In some embodiments, an isolated dmKSNF polypeptide of the invention comprises an amino acid sequence of at least about 6, at least about 10, at least about 20, at least about 50, at least about 75, at least about 100, at least about 150, at least about 200, at least about 250, at least about 300, at least about 350, at least about 400, at least about 450, at least about 500, at least about 550, at least about 600, or at least about 630 contiguous amino acids of the sequence set forth in SEQ ID NO:4, up to the entire amino acid sequence as set forth in SEQ ID NO:4.

In another embodiment, the dmKSNF protein derivative may consist of or comprise a sequence that shares 100% similarity with any contiguous stretch of at least 29 amino acids, preferably at least 31 amino acids, more preferably at least 34 amino acids, and most preferably at least 39 amino acids of SEQ ID NO:4. Preferred derivatives of dmKSNF consist of or comprise an amino acid sequence that has at least 80%, preferably at least 85%, more preferably at least 90%, and most preferably at least 95% or 100% sequence identity or sequence similarity with any of amino acid residues 1-41, 63-70, 92-123, 145-230, 252-257, 279-307, 329-345, 367-400, 422-440, 462-472, 494-514, 536-551, and 573-641, which are the extracellular or intracellular domains. Preferred fragments of dmKSNF proteins consist or comprise at least 20, preferably at least 22, more preferably at least 25, and most preferably at least 30 contiguous amino acids of SEQ ID NO:4.

### dmSNF Proteins

In some embodiments, the invention provides isolated dmSNF polypeptides. In some of these embodiments, isolated dmSNF proteins of the invention comprise or consist of an amino acid sequence of SEQ ID NO:6, or fragments or derivatives thereof.

In some embodiments, an isolated dmSNF polypeptide comprises at least about 6, at least about 10, at least about 20, at least about 50, at least about 75, at least about 100, at least about 150, at least about 200, at least about 250, at least about 300, at least about 350, or at least about 400 contiguous amino acids of the sequence set forth in SEQ ID NO:6, up to the entire amino acid sequence as set forth in SEQ ID NO:6.

In one preferred embodiment, a dmSNF protein derivative shares at least 70% sequence identity or similarity, preferably at least 75%, more preferably at least 80%, still more preferably at least 85%, more preferably at least 90%, and most preferably at least 95% sequence identity or similarity with a contiguous stretch of at least 25 amino acids, preferably at least 50 amino acids, more preferably at least 100 amino acids, and in some cases, the entire length of SEQ ID NO:6. Other preferred derivatives of dmSNF consist of or comprise an amino acid sequence that has the above-specified percent similarity or identity with amino acid residues 123-214, which comprise the SNF domain, or any of amino acid residues 1-119,137-158,176-221, 239-291, or 209-403, which are extra- or intracellular domains.

In another embodiment, the dmSNF protein derivative may consist of or comprise a sequence that shares 100% similarity with any contiguous stretch of at least 23 amino acids, preferably at least 25 amino acids, more preferably at least 28 amino acids, and most preferably at least 33 amino acids of SEQ ID NO:6. Preferred fragments of dmSNF proteins consist or comprise at least 19, preferably at least 21, more preferably at least 24, and most preferably at least 29 contiguous amino acids of SEQ ID NO:6.

### dmSNF2 Proteins

In some embodiments, the invention provides isolated dmSNF2 polypeptides. In some of these embodiments, isolated dmSNF2 proteins of the invention comprise or consist of an amino acid sequence of SEQ ID NO:8, or fragments or derivatives thereof.

In some embodiments, an isolated dmSNF2 polypeptide comprises at least about 6, at least about 10, at least about 20, at least about 50, at least about 75, at least about 100, at least about 150, at least about 200, at least about 250, at least about 300, at least about 350, at least about 400, at least about 500, at least about 550, or at least about 575 contiguous amino acids of the sequence set forth in SEQ ID NO:8, up to the entire amino acid sequence as set forth in SEQ ID NO:8.

In one preferred embodiment, a dmSNF2 protein derivative shares at least 70% sequence identity or similarity, preferably at least 75%, more preferably at least 80%, still more preferably at least 85%, more preferably at least 90%, and most preferably at least 95% sequence identity or similarity with a contiguous stretch of at least 25 amino acids, preferably at least 50 amino acids, more preferably at least 100 amino acids, and in some cases, the entire length of SEQ ID NO:8. Further preferred derivatives of dmSNF2 consist of or comprise an amino acid sequence that shares the above sequence identities or similarities with amino acid residues 15-548 of SEQ ID NO:8, which comprise the SNF domain, or with any of amino acid residues 1-21, 69-100, 118-189, 233-264, 282-304, 322-357, 375-395,449-475, and 519-592 of SEQ ID NO:8, which are intracellular or extracellular domains.

In another embodiment, the dmSNF2 protein derivative may consist of or comprise a sequence that shares 100% similarity with any contiguous stretch of at least 19 amino acids, preferably at least 21 amino acids, more preferably at least 24 amino acids, and most preferably at least 29 amino acids of SEQ ID NO:8.

Preferred fragments of dmSNF2 proteins consist or comprise at least 12, preferably at least 14, more preferably at least 17, and most preferably at least 22 contiguous amino acids of SEQ ID NO:8. Further preferred fragments consist of or comprise dmSNF2 transmembrane domains.

### dmSNF3 Proteins

In some embodiments, the invention provides isolated dmSNF3 polypeptides. In some of these embodiments, isolated dmSNF3 proteins of the invention comprise or consist of an amino acid sequence of SEQ ID NO:10, or fragments or derivatives thereof.

In some embodiments, an isolated dmSNF3 polypeptide comprises at least about 6, at least about 10, at least about 20, at least about 50, at least about 75, at least about 100, at least about 150, at least about 200, at least about 250, at least about 300, at least about 350, at least about 400, at least about 500, at least about 550, at least about 600, or at least about 625 contiguous amino acids of the sequence set forth in SEQ ID NO:10, up to the entire amino acid sequence as set forth in SEQ ID NO:10.

In one preferred embodiment, a dmSNF3 protein derivative shares at least 70% sequence identity or similarity, preferably at least 75%, more preferably at least 80%, still more preferably at least 85%, more preferably at least 90%, and most preferably at least 95% sequence identity or similarity with a contiguous stretch of at least 25 amino acids, preferably at least 50 amino acids, more preferably at least 100 amino acids, and in some cases, the entire length of SEQ ID NO:10. Further preferred derivatives of dmSNF3 consist of or comprise an amino acid sequence that shares the above sequence identities or similarities with amino acid residues 50-598 of SEQ ID NO:10, which comprise the SNF domain, or with amino acids 57-73; 87-103; 137-153; 239-255; 269-285; 355-371; 408-424; 448-464; 474-490; 522-538; and 564-580, which are the transmembrane domains. Further preferred are extracellular or intracellular domain-encoding regions between the transmembrane domains (*e.g.* residues 1-56, 74-86, *etc*.)

In another embodiment, the dmSNF3 protein derivative may consist of or comprise a sequence that shares 100% similarity with any contiguous stretch of at least 21 amino acids, preferably at least 23 amino acids, more preferably at least 26 amino acids, and most preferably at least 31 amino acids of SEQ ID NO:10.

Preferred fragments of dmSNF3 proteins consist or comprise at least 12 preferably at least 14, more preferably at least 17, and most preferably at least 22 contiguous amino acids of SEQ ID NO:10.

### dmGAT Proteins

In some embodiments, the invention provides isolated dmGAT polypeptides. In some of these embodiments, isolated dmGAT proteins of the invention comprise or consist of an amino acid sequence of SEQ ID NO:12, or fragments or derivatives thereof.

In some embodiments, an isolated dmGAT polypeptide comprises at least about 6, at least about 10, at least about 20, at least about 50, at least about 75, at least about 100, at least about 150, at least about 200, at least about 250, at least about 300, at least about 350, at least about 400, at least about 450, at least about 500, at least about 55 0, at least about 600, at least about 650, or at least about 670 contiguous amino acids of the sequence set forth in SEQ ID NO:12, up to the entire amino acid sequence as set forth in SEQ ID NO:12.

In one preferred embodiment, a dmGAT protein derivative shares at least 80% sequence identity or similarity, preferably at least 85%, more preferably at least 90%, and most preferably at least 95% sequence identity or similarity with a contiguous stretch of at least 25 amino acids, preferably at least 50 amino acids, more preferably at least 100 amino acids, and in some cases, the entire length of SEQ ID NO:12. In addition to sharing the above-mentioned sequence similarity with SEQ ID NO:12, preferably, the derivative dmGAT protein has the biological activity of a dmGAT protein.

In another embodiment, the dmGAT protein derivative.may consist of or comprise a sequence that shares 100% similarity with any contiguous stretch of at least 85 amino acids, preferably at least 87 amino acids, more preferably at least 90 amino acids, and most preferably at least 95 amino acids of SEQ ID NO:12. Preferred derivatives of dmGAT consist of or comprise an amino acid sequence that has at least 80%, preferably at least 85%, more preferably at least 90%, and most preferably at least 95% sequence identity or sequence similarity with any of amino acid residues 58-611, which comprise the SNF domain, or with amino acids 63-83, 94-114, 150-170, 251-271, 275-295, 326-346, 365-385, 413-433, 459-479, 492-512, 536-556, and 573-593, which are the likely transmembrane domains. Other preferred derivatives share these % identities or similarities with intra- or extracellular domains of dmGAT that reside between the transmembrane domains (i.e. residues 1-57, 84-93, etc.). Preferred fragments of dmGAT proteins consist or comprise at least 43, preferably at least 45, more preferably at least 48, and most preferably at least 53 contiguous amino acids of SEQ ID NO:12.

### GENE REGULATORY ELEMENTS OF THE SUBJECT NUCLEIC ACID MOLECULES

The invention further provides gene regulatory DNA elements, such as enhancers or promoters that control transcription of the subject nucleic acid molecules. Such regulatory elements can be used to identify tissues, cells, genes and factors that specifically control production of a subject protein. Analyzing components that are specific to a particular subject protein function can lead to an understanding of how to manipulate these regulatory processes, especially for pesticide and therapeutic applications, as well as an understanding of how to diagnose dysfunction in these processes.

Gene fusions with the subject regulatory elements can be made. For compact genes that have relatively few and small intervening sequences, such as those described herein for *Drosophila*, it is typically the case that the regulatory elements that control spatial and temporal expression patterns are found in the DNA immediately upstream of the coding region, extending to the nearest neighboring gene. Regulatory regions can be used to construct gene fusions where the regulatory DNAs are operably fused to a coding region for a reporter protein whose expression is easily detected, and these constructs are introduced as transgenes into the animal of choice. An entire regulatory DNA region can be used, or the regulatory region can be divided into smaller segments to identify sub-elements that might be specific for controlling expression a given cell type or stage of development. Reporter proteins that can be used for construction of these gene fusions include *E. coli* beta-galactosidase and green fluorescent protein (GFP). These can be detected readily *in situ*, and thus are useful for histological studies and can be used to sort cells that express a subject protein (O'Kane and Gehring PNAS (1987) 84(24):9123-9127; Chalfie *et al*., Science (1994) 263:802-805; and Cumberledge and Krasnow (1994) Methods in Cell Biology 44:143-159). Recombinase proteins, such as FLP orcre, can be used in controlling gene expression through site-specific recombination (Golic and Lindquist (1989) Cell 59(3):499-509; White *et al*., Science (1996) 271:805-807). Toxic proteins such as the reaper and hid cell death proteins, are useful to specifically ablate cells that normally express a subject protein in order to assess the physiological function of the cells (Kingston, In Current Protocols in Molecular Biology (1998) Ausubel *et al*., John Wiley & Sons, Inc. sections 12.0.3-12.10) or any other protein where it is desired to examine the function this particular protein specifically in cells that synthesize a subject protein.

Alternatively, a binary reporter system can be used, similar to that described further below, where a subject regulatory element is operably fused to the coding region of an exogenous transcriptional activator protein, such as the GAL4 or tTA activators described below, to create a subject regulatory element "driver gene". For the other half of the binary system the exogenous activator controls a separate "target gene" containing a coding region of a reporter protein operably fused to a cognate regulatory element for the exogenous activator protein, such as UAS_{G} or a tTA-response element, respectively. An advantage of a binary system is that a single driver gene construct can be used to activate transcription from preconstructed target genes encoding different reporter proteins, each with its own uses as delineated above.

Subject regulatory element-reporter gene fusions are also useful for tests of genetic interactions, where the objective is to identify those genes that have a specific role in controlling the expression of subject genes, or promoting the growth and differentiation of the tissues that expresses a subject protein. Subject gene regulatory DNA elements are also useful in protein-DNA binding assays to identify gene regulatory proteins that control the expression of subject genes. The gene regulatory proteins can be detected using a variety of methods that probe specific protein-DNA interactions well known to those skilled in the art (Kingston, *supra*) including in *vivo* footprinting assays based on protection of DNA sequences from chemical and enzymatic modification within living or permeabilized cells; and *in vitro* footprinting assays based on protection of DNA sequences from chemical or enzymatic modification using protein extracts, nitrocellulose filter-binding assays and gel electrophoresis mobility shift assays using radioactively labeled regulatory DNA elements mixed with protein extracts. Candidate gene regulatory proteins can be purified using a combination of conventional and DNA-affinity purification techniques. Molecular cloning strategies can also be used to identify proteins that specifically bind subject gene regulatory DNA elements. For example, a *Drosophila* cDNA library in an expression vector, can be screened for cDNAs that encode subject gene regulatory element DNA-binding activity. Similarly, the yeast "one-hybrid" system can be used (Li and Herskowitz, Science (1993) 262:1870-1874; Luo *et al*., Biotechniques (1996) 20(4):564-568; Vidal *et al*., PNAS (1996) 93(19):10315-10320).

### dmNTT4 regulatory DNA elements

In some embodiments, dnNTT4 regulatory elements are provided. dmNTT4 regulatory DNA elements, such as enhancers or promoters that reside within nucleotides 1 to 238, can be used to identify tissues, cells, genes and factors that specifically control dmNTT4 protein production. Preferably at least 20, more preferably at least 25, and most preferably at least 50 contiguous nucleotides within nucleotides 1 to 23 8 of SEQ ID NO:1 are used. Analyzing components that are specific to dmNTT4 protein function can lead to an understanding of how to manipulate these regulatory processes, especially for pesticide and therapeutic applications, as well as an understanding of how to diagnose dysfunction in these processes.

### dmKSNF Gene Regulatory Elements

In some embodiments, dmKSNF regulatory elements are provided. dmKSNF gene regulatory DNA elements, such as enhancers or promoters that reside within nucleotides 1 to 248, can be used to identify tissues, cells, genes and factors that specifically control dmKSNF protein production. Preferably at least 20, more preferably at least 25, and most preferably at least 50 contiguous nucleotides within nucleotides 1 to 248 of SEQ ID NO:3 are used. Analyzing components that are specific to dmKSNF protein function can lead to an understanding of how to manipulate these regulatory processes, especially for pesticide and therapeutic applications, as well as an understanding of how to diagnose dysfunction in these processes.

### dmSNF Gene Regulatory Elements

In some embodiments, dmSNF regulatory elements are provided. dmSNF gene regulatory DNA elements, such as enhancers or promoters that reside within nucleotides 1 to 497, can be used to identify tissues, cells, genes and factors that specifically control dmSNF protein production. Preferably at least 20, more preferably at least 25, and most preferably at least 50 contiguous nucleotides within nucleotides 1 to 497. of SEQ ID NO:5 are used. Analyzing components that are specific to dmSNF protein function can lead to an understanding of how to manipulate these regulatory processes, especially for pesticide and therapeutic applications, as well as an understanding of how to diagnose dysfunction in these processes.

### dmSNF2 Gene Regulatory Elements

In some embodiments, dmSNF2 regulatory elements are provided. dmSNF2 gene regulatory DNA elements, such as enhancers or promoters that reside within nucleotides 1 to 56, can be used to identify tissues, cells, genes and factors that specifically control dmSNF2 protein production. Preferably at least 20, more preferably at least 25, and most preferably at least 50 contiguous nucleotides within nucleotides 1 to 56 of SEQ ID NO:7 are used. Analyzing components that are specific to dmSNF2 protein function can lead to an understanding of how to manipulate these regulatory processes, especially for pesticide and therapeutic applications, as well as an understanding of how to diagnose dysfunction in these processes.

### dmSNF3 Gene Regulatory Elements

In some embodiments, dmSNF3 regulatory elements are provided. dmSNF3 gene regulatory DNA elements, such as enhancers or promoters that reside within nucleotides 1 to 49, can be used to identify tissues, cells, genes and factors that specifically control dmSNF3 protein production. Preferably at least 20, more preferably at least 25, and most preferably at least 40 contiguous nucleotides within nucleotides 1 to 49 of SEQ ID NO:9 are used. Analyzing components that are specific to dmSNF3 protein function can lead to an understanding of how to manipulate these regulatory processes, especially for pesticide and therapeutic applications, as well as an understanding of how to diagnose dysfunction in these processes.

### dmGAT Gene Regulatory Elements

In some embodiments, dmGAT regulatory elements are provided. dmGAT gene regulatory DNA elements, such as enhancers or promoters that reside within nucleotides 1 to 106, can be used to identify tissues, cells, genes and factors that specifically control dmGAT protein production. Preferably at least 20, more preferably at least 25, and most preferably at least 50 contiguous nucleotides within nucleotides 1 to 106 of SEQ ID NO:11 are used. Analyzing components that are specific to dmGAT protein function can lead to an understanding of how to manipulate these regulatory processes, especially for pesticide and therapeutic applications, as well as an understanding of how to diagnose dysfunction in these processes.

### ANTIBODIES SPECIFIC FOR SUBJECT PROTEINS

The present invention provides antibodies, which may be isolated antibodies, that bind specifically to a subject protein. The subject proteins, fragments thereof, and derivatives thereof may be used as an immunogen to generate monoclonal or polyclonal antibodies and antibody fragments or derivatives *(e.g.* chimeric, single chain, Fab fragments). For example, fragments of a subject protein, preferably those identified as hydrophilic, are used as immunogens for antibody production using art-known methods such as by hybridomas; production of monoclonal antibodies in germ-free animals (PCT/US90/02545); the use of human hybridomas (Cole *et al*., PNAS (1983) 80:2026-2030; Cole *et al*., in Monoclonal Antibodies and Cancer Therapy (1985) Alan R Liss, pp. 77-96), and production of humanized antibodies (Jones *et al*., Nature (1986) 321:522-525; U.S. Pat. 5,530,101). In a particular embodiment, subject polypeptide fragments provide specific antigens and/or immunogens, especially when coupled to carrier proteins. For example, peptides are covalently coupled to keyhole limpet antigen (KLH) and the conjugate is emulsified in Freund's complete adjuvant Laboratory rabbits are immunized according to conventional protocol and bled. The presence of specific antibodies is assayed by solid phase immunosorbent assays using immobilized corresponding polypeptide. Specific activity or function of the antibodies produced may be determined by convenient *in vitro,* cell-based, or *in vivo* assays: *e.g. in vitro* binding assays, *etc.* Binding affinity may be assayed by determination of equilibrium constants of antigen-antibody association (usually at least about 10⁷ M⁻¹, preferably at least about 10⁸ M⁻¹, more preferably at least about 10⁹ M⁻¹).

### Identification of Molecules that Interact with a Subject Protein

A variety of methods can be used to identify or screen for molecules, such as proteins or other molecules, that interact with a subject protein, or derivatives or fragments thereof. The assays may employ purified protein, or cell lines or model organisms such as *Drosophila* and *C. elegans*, that have been genetically engineered to express a subject protein. Suitable screening methodologies are well known in the art to test for proteins and other molecules that interact with a subj ect gene and protein (see e.g., PCT International Publication No. WO 96/34099). The newly identified interacting molecules may provide new targets for pharmaceutical or pesticidal agents. Any of a variety of exogenous molecules, both naturally occurring and/or synthetic *(e.g.,* libraries of small molecules or peptides, or phage display libraries), may be screened for binding capacity. In a typical binding experiment, a subject protein or fragment is mixed with candidate molecules under conditions conducive to binding, sufficient time is allowed for any binding to occur, and assays are performed to test for bound complexes. Assays to find interacting proteins can be performed by any method known in the art, for example, immunoprecipitation with an antibody that binds to the protein in a complex followed by analysis by size fractionation of the immunoprecipitated proteins (*e*.*g*. by denaturing or nondenaturing polyacrylamide gel electrophoresis), Western analysis, non-denaturing gel electrophoresis, two-hybrid systems (Fields and Song, Nature (1989) 340:245-246; U.S. Pat NO. 5,283,173; for review see Brent and Finley, Annu. Rev. Genet. (1977) 31:663-704), *etc.*

### Immunoassays

Immunoassays can be used to identify proteins that interact with or bind to a subject protein. Various assays are available for testing the ability of a protein to bind to or compete with binding to a wild-type subject protein or for binding to an anti- subject protein antibody. Suitable assays include radioimmunoassays, ELISA (enzyme linked immunosorbent assay), immunoradiometric assays, gel diffusion precipitin reactions, immunodiffusion assays, *in situ* immunoassays *(e.g.,* using colloidal gold, enzyme or radioisotope labels), western blots, precipitation reactions, agglutination assays *(e.g.,* gel agglutination assays, hemagglutination assays), complement fixation assays, immunofluorescenee assays, protein A assays, immunoelectrophoresis assays, *etc.*

### Identification of Potential Pesticide or Drug Targets

Once new target genes or target interacting genes are identified, they can be assessed as potential pesticide or drug targets, or as potential biopesticides. Further, transgenic plants that express subject proteins can be tested for activity against insect pests (Estruch *et al*., Nat. Biotechnol (1997) 15(2):137-141).

The subject proteins are validated pesticide targets, since disruption of the *Drosophila* the subject genes results in lethality when homozygous. The mutation to lethality of these gene indicates that drugs that agonize or antagonize the gene product may be effective pesticidal agents.

As used herein, the term "pesticide" refers generally to chemicals, biological agents, and other compounds that kill, paralyze, sterilize or otherwise disable pest species in the areas of agricultural crop protection, human and animal health. Exemplary pest species include parasites and disease vectors such as mosquitoes, fleas, ticks, parasitic nematodes, chiggers, mites, *etc.* Pest species also include those that are eradicated for aesthetic and hygienic purposes *(e.g.* ants, cockroaches, clothes moths, flour beetles, *etc.*)*,* home and garden applications, and protection of structures (including wood boring pests such as termites, and marine surface fouling organisms).

Pesticidal compounds can include traditional small organic molecule pesticides (typified by compound classes such as the organophosphates, pyrethroids, carbamates, and organochlorines, benzoylureas, *etc.*)*.* Other pesticides include proteinaceous toxins such as the *Bacillus thuringiensis* Crytoxins (Gill *et al*., Annu Rev Entomol (1992) 37:615-636) and *Photorabdus luminescens* toxins (Bowden *et al*., Science (1998) 280:2129-2132); and nucleic acids such as subject dsRNA or antisense nucleic acids that interfere with activity of a subject nucleic acid molecule. Pesticides can be delivered by a variety of means including direct application to pests or to their food source. In addition to direct application, toxic proteins and pesticidal nucleic acids (e.g. dsRNA) can be administered using biopesticidal methods, for example, by viral infection with nucleic acid or by transgenic plants that have been engineered to produce interfering nucleic acid sequences or encode the toxic protein, which are ingested by plant-eating pests.

Putative pesticides, drugs, and molecules can be applied onto whole insects, nematodes, and other small invertebrate metazoans, and the ability of the compounds to modulate *(e.g.* block or enhance) activity of a subject protein can be observed. Alternatively, the effect of various compounds on a subject protein can be assayed using cells that have been engineered to express one or more subject proteins and associated proteins.

### Assays of Compounds on Worms

In a typical worm assay, the compounds to be tested are dissolved in DMSO or other organic solvent, mixed with a bacterial suspension at various test concentrations, preferably OP50 strain of bacteria (Brenner, Genetics (1974) 110:421-440), and supplied as food to the worms. The population of worms to be treated can be synchronized larvae (Sulston and Hodgkin, in the nematode *C. elegans* (1988), *supra*) or adults or a mixed-stage population of animals.

Adult and larval worms are treated with different concentrations of compounds, typically ranging from 1 mg/ml to 0.001 mg/ml. Behavioral aberrations, such as a decrease in motility and growth, and morphological aberrations, sterility, and death are examined in both acutely and chronically treated adult and larval worms. For the acute assay, larval and adult worms are examined immediately after application of the compound and re-examined periodically (every 30 minutes) for 5-6 hours. Chronic or long-term assays are performed on worms and the behavior of the treated worms is examined every 8-12 hours for 4-5 days. In some circumstances, it is necessary to reapply the pesticide to the treated worms every 24 hours for maximal effect.

### Assays of Compounds on Insects

Potential insecticidal compounds can be administered to insects in a variety of ways, including orally (including addition to synthetic diet, application to plants or prey to be consumed by the test organism), topically (including spraying, direct application of compound to animal, allowing animal to contact a treated surface), or by injection. Insecticides are typically very hydrophobic molecules and must commonly be dissolved in organic solvents, which are allowed to evaporate in the case of methanol or acetone, or at low concentrations can be included to facilitate uptake (ethanol, dimethyl sulfoxide).

The first step in an insect assay is usually the determination of the minimal lethal dose (MLD) on the insects after a chronic exposure to the compounds. The compounds are usually diluted in DMSO, and applied to the food surface bearing 0-48 hour old embryos and larvae. In addition to MLD, this step allows the determination of the fraction of eggs that hatch, behavior of the larvae, such as how they move /feed compared to untreated larvae, the fraction that survive to pupate, and the fraction that eclose (emergence of the adult insect from puparium). Based on these results more detailed assays with shorter exposure times may be designed, and larvae might be dissected to look for obvious morphological defects. Once the MLD is determined, more specific acute and chronic assays can be designed.

In a typical acute assay, compounds are applied to the food surface for embryos, larvae, or adults, and the animals are observed after 2 hours and after an overnight incubation. For application on embryos, defects in development and the percent that survive to adulthood are determined. For larvae, defects in behavior, locomotion, and molting may be observed. For application on adults, behavior and neurological defects are observed, and effects on fertility are noted.

For a chronic exposure assay, adults are placed on vials containing the compounds for 48 hours, then transferred to a clean container and observed for fertility, neurological defects, and death.

### Assay of Compounds using Cell Cultures

Compounds that modulate (*e.g.* block or enhance) a subject protein's activity may also be assayed using cell culture. For example, various compounds added to cells expressing a subject protein may be screened for their ability to modulate the activity of subject genes based upon measurements of a biological activity of a subject protein. Assays for changes in a biological activity of a subject protein can be performed on cultured cells expressing endogenous normal or mutant subject protein. Such studies also can be performed on cells transfected with vectors capable of expressing the subject protein, or functional domains of one of the subject protein, in normal or mutant form. In addition, to enhance the signal measured in such assays, cells may be cotransfected with genes encoding a subject protein.

Alternatively, cells expressing a subject protein may be lysed, the subject protein purified, and tested in *vitro* using methods known in the art (Kanemaki M., et al., J Biol Chem, 1999 274:22437-22444).

Compounds that selectively modulate a subject protein are identified as potential pesticide and drug candidates having specificity for the subject protein.

Identification of small molecules and compounds as potential pesticides or pharmaceutical compounds from large chemical libraries requires high-throughput screening (HTS) methods (Bolger, Drug Discovery Today (1999) 4:251-253). Several of the assays mentioned herein can lend themselves to such screening methods. For example, cells or cell lines expressing wild type or mutant subject protein or its fragments, and a reporter gene can be subjected to compounds of interest, and depending on the reporter genes, interactions can be measured using a variety of methods such as color detection, fluorescence detection (*e.g.* GFP), autoradiography, scintillation analysis, *etc.*

### Subject Nucleic Acids as Biopesticides

Subject nucleic acids and fragments thereof, such as antisense sequences or double-stranded RNA (dsRNA), can be used to inhibit subject nucleic acid molecule function, and thus can be used as biopesticides. Methods of using dsRNA interference are described in published PCT application WO 99/32619. The biopesticides may comprise the nucleic acid molecule itself, an expression construct capable of expressing the nucleic acid, or organisms transfected with the expression construct. The biopesticides may be applied directly to plant parts or to soil surrounding the plants (*e.g.* to access plant parts growing beneath ground level), or directly onto the pest.

Biopesticides comprising a subject nucleic acid may be prepared in a suitable vector for delivery to a plant or animal. For generating plants that express the subject nucleic acids, suitable vectors include *Agrobacterium tumefaciens* Ti plasmid-based vectors (Horsch *et al*., Science (1984) 233:496-89; Fraley *et al*., Proc. Natl. Acad. Sci. USA (1983) 80:4803), and recombinant cauliflower mosaic virus (Hohn *et al*., 1982, In Molecular Biology of Plant Tumors, Academic Press, New York, pp 549-560; U.S. Patent No. 4,407,956 to Howell). Retrovirus based vectors are useful for the introduction of genes into vertebrate animals (Burns *et al*., Proc. Natl. Acad. Sci. USA (1993) 90:8033-37).

Transgenic insects can be generated using a transgene comprising a subject gene operably fused to an appropriate inducible promoter. For example, a tTA-responsive promoter may be used in order to direct expression of a subject protein at an appropriate time in the life cycle of the insect. In this way, one may test efficacy as an insecticide in, for example, the larval phase of the life cycle (i.e. when feeding does the greatest damage to crops). Vectors for the introduction of genes into insects include P element (Rubin and Spradling, Science (1982) 218:348-53; U.S. Pat. No. 4,670,388), "hermes" (O'Brochta *et al*., Genetics (1996) 142:907-914), "minos" (U.S. Pat. No. 5,348,874), "mariner" (Robertson, Insect Physiol. (1995) 41:99-105), and "sleeping beauty"(Ivics *et al*., Cell (1997) 91(4):501-510), "piggyBac" (Thibault *et al*., Insect Mol Biol (1999) 8(1):119-23), and "hobo" (Atkinson *et al*., Proc. Natl. Acad. Sci. U.S.A. (1993) 90:9693-9697). Recombinant virus systems for expression of toxic proteins in infected insect cells are well known and include Semliki Forest virus (DiCiommo and Bremner, J. Biol. Chem. (1998) 273:18060-66), recombinant sindbis virus (Higgs *et al*., Insect Mol. Biol. (1995) 4:97-103; Seabaugh *et al*., Virology (1998) 243:99-112), recombinant pantropic retrovirus (Matsubara *et al*., Proc. Natl. Acad. Sci. USA (1996) 93:6181-85; Jordan *et al*., Insect Mol. Biol. (1998) 7:215-22), and recombinant baculovirus (Cory and Bishop, Mol. Biotechnol. (1997) 7(3):303-13; U.S. Patent No. 5,470,735; U.S. Patent Nos. 5,352,451; U.S. Patent No. 5, 770,192; U.S. Patent No. 5,759,809; U.S. Patent No. 5,665,349; and U.S. Patent No. 5,554,592).

### Generation and Genetic Analysis of Animals and Cell Lines with Altered Expression of a Subject Gene

Both genetically modified animal models (i.e. *in vivo* models), such as *C. elegans* and *Drosophila,* and *in vitro* models such as genetically engineered cell lines expressing or mis-expressing subject pathway genes, are useful for the functional analysis of these proteins. Model systems that display detectable phenotypes, can be used for the identification and characterization of subject pathway genes or other genes of interest and/or phenotypes associated with the mutation or mis-expressian of subject pathway protein. The term "mis-expression" as used herein encompasses mis-expression due to gene mutations. Thus, a mis-expressed subject pathway protein may be one having an amino acid sequence that differs from wild-type (i.e. it is a derivative of the normal protein). A mis-expressed subject pathway protein may also be one in which one or more amino acids have been deleted, and thus is a "fragment" of the normal protein. As used herein, "mis-expression" also includes ectopic expression (*e*.*g*. by altering the normal spatial or temporal expression), over-expression *(e.g.* by multiple gene copies), underexpression, non-expression *(e.g.* by gene knockout or blocking expression that would otherwise normally occur), and further, expression in ectopic tissues. As used in the following discussion concerning *in vivo* and *in vitro* models, the term "gene of interest" refers to a subject pathway gene, or any other gene involved in regulation or modulation, or downstream effector of the subject pathway.

The *in vivo* and *in vitro* models may be genetically engineered or modified so that they 1) have deletions and/or insertions of one or more subject pathway genes, 2) harbor interfering RNA sequences derived from subject pathway genes, 3) have had one or more endogenous subject pathway genes mutated (*e*.*g*. contain deletions, insertions, rearrangements, or point mutations in subject gene or other genes in the pathway), and/or 4) contain transgenes for mis-expression of wild-type or mutant forms of such genes. Such genetically modified *in vivo* and *in vitro* models are useful for identification of genes and proteins that are involved in the synthesis, activation, control, *etc*. of subject pathway gene and/or gene products, and also downstream effectors of subject function, genes regulated by subject, etc. The newly identified genes could constitute possible pesticide targets (as judged by animal model phenotypes such as non-viability, block of normal development, defective feeding, defective movement, or defective reproduction). The model systems can also be used for testing potential pesticidal or pharmaceutical compounds that interact with the subject pathway, for example by administering the compound to the model system using any suitable method (*e*.*g*. direct contact, ingestion, injection, *etc.*) and observing any changes in phenotype, for example defective movement, lethality, *etc.* Various genetic engineering and expression modification methods which can be used are well-known in the art, including chemical mutagenesis, transposon mutagenesis, antisense RNAi, dsRNAi, and transgene-mediated mis-expression.

### Generating Loss-of-function Mutations by Mutagenesis

Loss-of-function mutations in an invertebrate metazoan subject gene can be generated by any of several mutagenesis methods known in the art (Ashburner, In Drosophila melanogaster: A Laboratory Manual (1989), Cold Spring Harbor, NY, Cold Spring Harbor Laboratory Press: pp. 299-418; Fly pushing: The Theory and Practice of Drosophila melanogaster Genetics (1997) Cold Spring Harbor Press, Plainview, NY; The nematode *C. elegans* (1988) Wood, Ed., Cold Spring Harbor Laboratory Press, Cold Spring harbor, New York). Techniques for producing mutations in a gene or genome include use of radiation (*e.g*., X-ray, UV, or gamma ray); chemicals *(e.g.,* EMS, MMS, ENU, formaldehyde, *etc.*)*;* and insertional mutagenesis by mobile elements including dysgenesis induced by transposon insertions, or transposon-mediated deletions, for example, male recombination, as described below. Other methods of altering expression of genes include use of transposons *(e.g.,* P element, EP-type "overexpression trap" element, mariner element, *piggyBac* transposon, hermes, minos, sleeping beauty, *etc.*) to misexpress genes; antisense; double-stranded RNA interference; peptide and RNA aptamers; directed deletions; homologous recombination; dominant negative alleles; and intrabodies.

Transposon insertions lying adjacent to a gene of interest can be used to generate deletions of flanking genomic DNA, which if induced in the germline, are stably propagated in subsequent generations. The utility of this technique in generating deletions has been demonstrated and is well-known in the art. One version of the technique using collections of P element transposon induced recessive lethal mutations (P lethals) is particularly suitable for rapid identification of novel, essential genes in *Drosophila* (Cooley *et al*., Science (1988) 239:1121-1128; Spralding *et al.,* PNAS (1995) 92:0824-10830). Since the sequence of the P elements are known, the genomic sequence flanking each transposon insert is determined either by plasmid rescue (Hamilton *et al*., PNAS (1991) 88:2731-2735) or by inverse polymerase chain reaction, using well-established techniques. (Rehm, http://www.fruitfly.org/methods/). The subject genes were identified from a P lethal screen. Disruption of the *Drosophila* subject gene results in lethality when homozygous, indicating that this protein is critical for cell function and the survival of insects. The mutation to lethality of this gene indicates that drugs which agonize or antagonize the encoded subject protein will be effective insecticidal agents and that this class of proteins are excellent targets for drug screening and discovery.

A more recent version of the transposon insertion technique in male *Drosophila* using P elements is known as P-mediated male recombination (Preston and Engels, Genetics (1996) 144:1611-1638).

### Generating Loss-of-function Phenotypes Using RNA-based Methods

The subject genes may be identified and/or characterized by generating loss-of-function phenotypes in animals of interest through RNA-based methods, such as antisense RNA (Schubiger and Edgar, Methods in Cell Biology (1994) 44:697-713). One form of the antisense RNA method involves the injection of embryos with an antisense RNA that is partially homologous to the gene of interest (in this case the subject gene). Another form of the antisense RNA method involves expression of an antisense RNA partially homologous to the gene of interest by operably joining a portion of the gene of interest in the antisense orientation to a powerful promoter that can drive the expression of large quantities of antisense RNA, either generally throughout the animal or in specific tissues. Antisense RNA-generated loss-of function phenotypes have been reported previously for several *Drosophila* genes including cactus, pecanex, and Krüppel (LaBonne *et al*., Dev. Biol. (1989) 136(1):1-16; Schuh and Jackle, Genome (1989) 31(1):422-425; Geisler *et al*., Cell (1992) 71(4):613-621).

Loss-of-function phenotypes can also be generated by cosuppression methods (Bingham Cell (1997) 90(3):385-387; Smyth, Curr. Biol. (1997) 7(12):793-795; Que and Jorgensen, Dev. Genet. (1998)22(1):100-109). Cosuppression is a phenomenon of reduced gene expression produced by expression or injection of a sense strand RNA corresponding to a partial segment of the gene of interest Cosuppression effects have been employed extensively in plants and *C. elegans* to generate loss-of-function phenotypes, and there is a single report of cosuppression in *Drosophila,* where reduced expression of the Adh gene was induced from a white-Adh transgene using cosuppression methods (Pal-Bhadra *et al*., Cell (1997) 90(3):479-490).

Another method for generating loss-of-function phenotypes is by double-stranded RNA interference (dsRNAi). This method is based on the interfering properties of double-stranded RNA derived from the coding regions of gene, and has proven to be of great utility in genetic studies of *C. elegans* (Fire *et al*., Nature (1998) 391:806-811), and can also be used to generate loss-of-function phenotypes in *Drosophila* (Kennerdell and Carthew, Cell (1998) 95:1017-1026; Misquitta and Patterson PNAS (1999) 96:1451-1456). In one example of this method, complementary sense and antisense RNAs derived from a substantial portion of a gene of interest, such as a subject gene, are synthesized *in vitro.* The resulting sense and antisense RNAs are annealed in an injection buffer, and the double-stranded RNA injected or otherwise introduced into animals (such as in their food or by soaking in the buffer containing the RNA). Progeny of the injected animals are then inspected for phenotypes of interest (PCT publication no. W099/32619).

In another embodiment of the method, the dsRNA can be delivered to the animal by bathing the animal in a solution containing a sufficient concentration of the dsRNA. In another embodiment of the method, dsRNA derived from the subject genes can be generated *in vivo* by simultaneous expression of both sense and antisense RNA from appropriately positioned promoters operably fused to subject sequences in both sense and antisense orientations. In yet another embodiment of the method the dsRNA can be delivered to the animal by engineering expression of dsRNA within cells of a second organism that serves as food for the animal, for example engineering expression of dsRNA in *E. coli* bacteria which are fed to *C. elegans*, or engineering expression of dsRNA in baker's yeast which are fed to *Drosophila,* or engineering expression of dsRNA in transgenic plants which are fed to plant eating insects such as *Leptinotarsa* or *Heliothis.* Recently, RNAi has been successfully used in cultured *Drosophila* cells to inhibit expression of targeted proteins (Clemens, J.C., *et al*., Proc Natl Acad Sci U S A 2000 Jun 6;97(12):6499-503). Thus, cell lines in culture can be manipulated using RNAi both to perturb and study the function of the subject gene pathway components and to validate the efficacy of therapeutic or pesticidal strategies that involve the manipulation of this pathway.

### Generating Loss-of-function Phenotypes Using Peptide and RNA Aptamers

Additional methods that can be used for generating loss-of-function phenotypes include use of peptide aptamers that act as dominant inhibitors of protein function (Kolonin and Finley, PNAS (1998) 95:14266-14271; Xu *et al*., PNAS (1997) 94:12473-12478; Hoogenboom *et al*., Immunotechnology (1998) 4:1-20), RNA aptamers (Good *et al*., Gene Therapy (1997) 4:45-54; Ellingtan *et al*., Biotechnol. Annu. Rev. (1995) 1:185-214; Bell *et al*., J. Biol. Chem. (1998) 273:14309-14314; Shi *et al*., Proc. Natl. Acad. Sci USA (1999) 96:10033-10038), and intrabodies (Chen *et al*., Hum. Gen. Ther. (1994) 5:595-601; Hassanzadch *et al*., Febs Lett. (1998) 16:75-86).

### Generating Loss of Function Phenotypes Using Intrabodies

Intracellularly expressed antibodies, or intrabodies, are single-chain antibody molecules designed to specifically bind and inactivate target molecules inside cells. Intrabodies have been used in cell assays and in whole organisms such as *Drosophila* (Chen *et al*., Hum. Gen. Ther. (1994) 5:595-601; Hassanzadeh *et al*., Febs Lett. (1998) 16(1, 2):75-80 and 81-86). Inducible expression vectors can be constructed with intrabodies that react specifically with a subject protein. These vectors can be introduced into model organisms and studied in the same manner as described above for aptamers.

### Transgenesis

Typically, transgenic animals are created that contain gene fusions of the coding regions of a subject gene (from either genomic DNA or cDNA) or genes engineered to encode antisense RNAs, cosuppression RNAs, interfering dsRNA, RNA aptamers, peptide aptamers, or intrabodies operably joined to a specific promoter and transcriptional enhancer whose regulation has been well characterized, preferably heterologous promoters/enhancers (i.e. promoters/enhancers that are non-native to a subject pathway genes being expressed).

Methods are well known for incorporating exogenous nucleic acid sequences into the genome of animals or cultured cells to create transgenic animals or recombinant cell lines. For invertebrate animal models, the most common methods involve the use of transposable elements. There are several suitable transposable elements that can be used to incorporate nucleic acid sequences into the genome of model organisms. Transposable elements are particularly useful for inserting sequences into a gene of interest so that the encoded protein is not properly expressed, creating a "knock-out" animal having a loss-of-function phenotype. Techniques are well-established for the use of P element in *Drosophila* (Rubin and Spradling, Science (1982) 218:348-53; U.S. Pat. No. 4,670,388) and Tc1 in *C. elegans* (Zwaal *et al*., Proc. Natl. Acad. Sci. U.S.A. (1993) 90:7431-7435; and *Caenorhabditis elegans*: Modern Biological Analysis of an Organism (1995) Epstein and Shakes, Eds.). Other Tc1-like transposable elements can be used such as minos, mariner and sleeping beauty. Additionally, transposable elements that function in a variety of species, have been identified, such as PiggyBac (Thibault *et al*., Insect Mol Biol (1999) 8(1):119-23), hobo, and hermes.

P elements, or marked P elements, are preferred for the isolation of loss-of-function mutations in *Drosophila* genes because of the precise molecular mapping of these genes, depending on the availability and proximity of preexisting P element insertions for use as a localized transposon source (Hamilton and Zinn, Methods in Cell Biology (1994) 44:81-94; and Wolfner and Goldberg, Methods in Cell Biology (1994) 44:33-80). Typically, modified P elements are used which contain one or more elements that allow detection of animals containing the P element. Most often, marker genes are used that affect the eye color *of Drosophila,* such as derivatives of the *Drosophila white* or *rosy* genes (Rubin and Spradling, Science (1982) 218(4570):348-353; and Klemenz *et al*., Nucleic Acids Res. (1987) 15(10):3947-3959). However, in principle, any gene can be used as a marker that causes a reliable and easily scored phenotypic change in transgenic animals. Various other markers include bacterial plasmid sequences having selectable markers such as ampicillin resistance (Steller and Pirrotta, EMBO. J. (1985) 4:167-171); and *lacZ* sequences fused to a weak general promoter to detect the presence of enhancers with a developmental expression pattern of interest (Bellen *et al*., Genes Dev. (1989) 3(9):1288-1300). Other examples of marked P elements useful for mutagenesis have been reported (Nucleic Acids Research (1998) 26:85-88; and http://flybase.bio.indiana.edu).

Preferred methods of transposon mutagenesis in *Drosophila* employ the "local hopping" method described by Tower *et al*. (Genetics (1993) 133:347-359) or generation of localized deletions from *Drosophila* lines carrying P insertions in the gene of interest using known methods (Kaiser, Bioassays (1990) 12(6);297-301; Harnessing the power of *Drosophila* genetics, In Drosophila melanogaster: Practical Uses in Cell and Molecular Biology, Goldstein and Fyrberg, Eds., Academic Press, Inc., San Diego, California). The preferred method of transposon mutagenesis in *C. elegans* employs Tc1 transposable element (Zwaal *et al, supra;* Plasterk *et al*., *supra).*

In addition to creating loss-of-function phenotypes, transposable elements can be used to incorporate the gene of interest, or mutant or derivative thereof, as an additional gene into any region of an animal's genome resulting in mis-expression (including over-expression) of the gene. A preferred vector designed specifically for misexpression of genes in transgenic *Drosophila,* is derived from pGMR (Hay *et al*., Development (1994) 120:2121-2129), is 9Kb long, and contains: an origin of replication for *E. coli;* an ampicillin resistance gene; P element transposon 3' and 5' ends to mobilize the inserted sequences; a White marker gene; an expression unit comprising the TATA region of hsp70 enhancer and the 3'untranslated region of α-tubulin gene. The expression unit contains a first multiple cloning site (MCS) designed for insertion of an enhancer and a second MCS located 500 bases downstream, designed for the insertion of a gene of interest. As an alternative to transposable elements, homologous recombination or gene targeting techniques can be used to substitute a gene of interest for one or both copies of the animal's homologous gene. The transgene can be under the regulation of either an exogenous or an endogenous promoter element, and be inserted as either a minigene or a large genomic fragment. In one application, gene function can be analyzed by ectopic expression, using, for example, *Drosophila* (Brand *et al*., Methods in Cell Biology (1994) 44:635- 654) or *C. elegans* (Mello and Fire, Methods in Cell Biology (1995) 48:451-482).

Examples of well-characterized heterologous promoters that may be used to create the transgenic animals include heat shock promoters/enhancers, which are useful for temperature induced mis-expression. In *Drosophila,* these include the *hsp70* and *hsp83* genes, and in *C. elegans*, include *hsp 16-2* and *hsp 16-41*. Tissue specific promoters/enhancers are also useful, and in *Drosophila*, include *eyeless* (Mozer and Benzer, Development (1994) 120:1049-1058), *sevenless* (Bowtell *et al*., PNAS (1991) 88(15):6853-6857), and *glass*-responsive promoters/enhancers (Quiring *et al*., Science (1994) 265:785-789) which are useful for expression in the eye; and enhancers/promoters derived from the *dpp* or *vestigal* genes which are useful for expression in the wing (Staehling-Hampton *et al*., Cell Growth Differ. (1994) 5(6):585-593; Kim *et al*., Nature (1996) 382:133-138). Finally, where it is necessary to restrict the activity of dominant active or dominant negative transgenes to regions where the pathway is normally active, it may be useful to use endogenous promoters of genes in the pathway, such as a subject protein pathway genes.

In *C. elegans*, examples of useful tissue specific promoters/enhancers include the *myo-2* gene promoter, useful for pharyngeal muscle-specific expression; the *hlh-1* gene promoter, useful for body-muscle-specific expression; and the gene promoter, useful for touch-neuron-specific gene expression. In a preferred embodiment, gene fusions for directing the mis-expression of a subject pathway gene are incorporated into a transformation vector which is injected into nematodes along with a plasmid containing a dominant selectable marker, such as *rol-6.* Transgenic animals are identified as those exhibiting a roller phenotype, and the transgenic animals are inspected for additional phenotypes of interest created by mis-expression of a subject pathway gene.

In *Drosophila,* binary control systems that employ exogenous DNA are useful when testing the mis-expression of genes in a wide variety of developmental stage-specific and tissue-specific patterns. Two examples of binary exogenous regulatory systems include the UAS/GAL4 system from yeast (Hay *et al*., PNAS (1997) 94(10):5195-5200; Ellis *et al*., Development (1993) 119(3):855-865); Brand and Perrimon (1993) Development 118(2):401-415), and the "Tet system" derived from *E. coli* (Bello et al., Development (1998) 125:2193-2202).

Dominant negative mutations, by which the mutation causes a protein to interfere with the normal function of a wild-type copy of the protein, and which can result in loss-of-function or reduced-function phenotypes in the presence of a normal copy of the gene, can be made using known methods (Hershkowitz, Nature (1987) 329:219-222).

### Assays for Change in Gene Expression

Various expression analysis techniques may be used to identify genes which are differentially expressed between a cell line or an animal expressing a wild type subject gene compared to another cell line or animal expressing a mutant subject gene. Such expression profiling techniques include differential display, serial analysis of gene expression (SAGE), transcript profiling coupled to a gene database query, nucleic acid array technology, subtractive hybridization, and proteome analysis (e.g. mass-spectrometry and two-dimensional protein gels). Nucleic acid array technology may be used to determine a global (i.e., genome-wide) gene expression pattern in a normal animal for comparison with an animal having a mutation in a subject gene. Gene expression profiling can also be used to identify other genes (or proteins) that may have a functional relation to a subject (e.g. may participate in a signaling pathway with a subject gene). The genes are identified by detecting changes in their expression levels following mutation, i.e., insertion, deletion or substitution in, or over-expression, underexpression, mis-expression or knock-out, of the subject

### Phenotypes Associated with Target Pathway Gene Mutations

After isolation of model animals carrying mutated or mis-expressed subject pathway genes or inhibitory RNAs, animals are carefully examined for phenotypes of interest. For analysis of subject pathway genes that have been mutated (i.e. deletions, insertions, and/or point mutations) animal models that are both homozygous and heterozygous for the altered subject pathway gene are analyzed. Examples of specific phenotypes that may be investigated include lethality; sterility; feeding behavior, perturbations in neuromuscular function including alterations in motility, and alterations in sensitivity to pesticides and pharmaceuticals. Some phenotypes more specific to flies include alterations in: adult behavior such as, flight ability, walking, grooming, phototaxis, mating or egg-laying; alterations in the responses of sensory organs, changes in the morphology, size or number of adult tissues such as, eyes, wings, legs, bristles, antennae, gut, fat body, gonads, and musculature; larval tissues such as mouth parts, cuticles, internal tissues or imaginal discs; or larval behavior such as feeding, molting, crawling, or puparian formation; or developmental defects in any germline or embryonic tissues. Some phenotypes more specific to nematodes include: locomotory, egg laying, chemosensation, male mating, and intestinal expulsion defects. In various cases, single phenotypes or a combination of specific phenotypes in model organisms might point to specific genes or a specific pathway of genes, which facilitate the cloning process.

Genomic sequences containing a subject pathway gene can be used to confirm whether an existing mutant insect or worm line corresponds to a mutation in one or more subject pathway genes, by rescuing the mutant phenotype. Briefly, a genomic fragment containing the subject pathway gene of interest and potential flanking regulatory regions can be subcloned into any appropriate insect (such as *Drosophila)* or worm (such as *C. elegans*) transformation vector, and injected into the animals. For *Drosophila,* an appropriate helper plasmid is used in the injections to supply transposase for transposon-based vectors. Resulting germline transformants are crossed for complementation testing to an existing or newly created panel of *Drosophila* or *C. elegans* lines whose mutations have been mapped to the vicinity of the gene of interest (Fly Pushing: The Theory and Practice of *Drosophila* Genetics, *supra*; and *Caenorhabditis elegans*: Modern Biological Analysis of an Organism (1995), Epstein and Shakes, eds.). If a mutant line is discovered to be rescued by this genomic fragment, as judged by complementation of the mutant phenotype, then the mutant line likely harbors a mutation in the subject pathway gene. This prediction can be further confirmed by sequencing the subject pathway gene from the mutant line to identify the lesion in the subject pathway gene.

### Identification of Genes That Modify a Subject Genes

The characterization of new phenotypes created by mutations or misexpression in subject genes enables one to test for genetic interactions between subject genes and other genes that may participate in the same, related, or interacting genetic or biochemical pathway(s). Individual genes can be used as starting points in large-scale genetic modifier screens as described in more detail below. Alternatively, RNAi methods can be used to simulate loss-of-function mutations in the genes being analyzed. It is of particular interest to investigate whether there are any interactions of subject genes with other well-characterized genes, particularly genes involved in DNA unwinding.

### Genetic Modifier Screens

A genetic modifier screen using invertebrate model organisms is a particularly preferred method for identifying genes that interact with subject genes, because large numbers of animals can be systematically screened making it more possible that interacting genes will be identified. In *Drosophila,* a screen of up to about 10,000 animals is considered to be a pilot-scale screen. Moderate-scale screens usually employ about 10,000 to about 50,000 flies, and large-scale screens employ greater than about 50,000 flies. In a genetic modifier screen, animals having a mutant phenotype due to a mutation in or misexpression of one or more subject genes are further mutagenized, for example by chemical mutagenesis or transposon mutagenesis.

The procedures involved in typical *Drosophila* genetic modifier screens are well nown in the art (Wolfner and Goldberg, Methods in Cell Biology (1994) 44:33-80; and Karim *et al*., Genetics (1996) 143:315-329). The procedures used differ depending upon the precise nature of the mutant allele being modified. If the mutant allele is genetically recessive, as is commonly the situation for a loss-of-function allele, then most typically males, or in some cases females, which carry one copy of the mutant allele are exposed to an effective mutagen, such as EMS, MMS, ENU, triethylamine, diepoxyalkanes, ICR-170, formaldehyde, X-rays, gamma rays, or ultraviolet radiation. The mutagenized animals are crossed to animals of the opposite sex that also carry the mutant allele to be modified. In the case where the mutant allele being modified is genetically dominant, as is commonly the situation for ectopically expressed genes, wild type males are mutagenized and crossed to females carrying the mutant allele to be modified.

The progeny of the mutagenized and crossed flies that exhibit either enhancement or suppression of the original phenotype are presumed to have mutations in other genes, called "modifier genes", that participate in the same phenotype-generating pathway. These progeny are immediately crossed to adults containing balancer chromosomes and used as founders of a stable genetic line. In addition, progeny of the founder adult are retested under the original screening conditions to ensure stability and reproducibility of the phenotype. Additional secondary screens may be employed, as appropriate, to confirm the suitability of each new modifier mutant line for further analysis.

Standard techniques used for the mapping of modifiers that come from a genetic screen in *Drosophila* include meiotic mapping with visible or molecular genetic markers; male-specific recombination mapping relative to P-element insertions; complementation analysis with deficiencies, duplications, and lethal P-element insertions; and cytological analysis of chromosomal aberrations (Fly Pushing: Theory and Practice of *Drosophila* Genetics, *supra; Drosophila:* A Laboratory Handbook, *supra*). Genes corresponding to modifier mutations that fail to complement a lethal P-element may be cloned by plasmid rescue of the genomic sequence surrounding that P-element. Alternatively, modifier genes may be mapped by phenotype rescue and positional cloning (Sambrook *et al*., *supra).*

Newly identified modifier mutations can be tested directly for interaction with other genes of interest known to be involved or implicated with a subject gene using methods described above. Also, the new modifier mutations can be tested for interactions with genes in other pathways that are not believed to be related to neuronal signaling *(e.g.* nanos in *Drosophila).* New modifier mutations that exhibit specific genetic interactions with other genes implicated in neuronal signaling, but not interactions with genes in unrelated pathways, are of particular interest.

The modifier mutations may also be used to identify "complementation groups". Two modifier mutations are considered to fall within the same complementation group if animals carrying both mutations in trans exhibit essentially the same phenotype as animals that are homozygous for each mutation individually and, generally are lethal when in trans to each other (Fly Pushing: The Theory and Practice of *Drosophila* Genetics, *supra*). Generally, individual complementation groups defined in this way correspond to individual genes.

When modifier genes are identified, homologous genes in other species can be isolated using procedures based on cross-hybridization with modifier gene DNA probes, PCR-based strategies with primer sequences derived from the modifier genes, and/or computer searches of sequence databases. For therapeutic applications related to the function of subject genes, human and rodent homologs of the modifier genes are of particular interest. For pesticide and other agricultural applications, homologs of modifier genes in insects and arachnids are of particular interest. Insects, arachnids, and other organisms of interest include, among others, Isopoda; Diplopoda; Chilopoda; Symphyla; Thysanura; Collembola; Orthoptera, such as *Scistocerca* spp; Blattoidea, such as *Blattella germanica*; Dermaptera; Isoptera; Anoplura; Mallophaga; Thysanoptera; Heteroptera; Homoptera, including *Bemisia* tabaci, and *Myzus* spp.; Lepidoptera including *Plodia interpunctella, Pectinophora gossypiella, Plutella* spp., *Heliothis* spp., and *Spodoptera* species; Coleoptera such as *Leptinotarsa, Diabrotica* spp., *Anthonomus* spp., and *Tribolium* spp.; Hymenoptera; Diptera, including *Anopheles* spp.; Siphonaptera, including Ctenocephalides felis; Arachnida; and Acarinan, including *Amblyoma americanum*; and nematodes, including *Meloidogyne* spp., and *Heterodera glycinii*.

Although the above-described *Drosophila* genetic modifier screens are quite powerful and sensitive, some genes that interact with subject genes may be missed in this approach, particularly if there is functional redundancy of those genes. This is because the vast majority of the mutations generated in the standard mutagenesis methods will be loss-of-function mutations, whereas gain-of-function mutations that could reveal genes with functional redundancy will be relatively rare. Another method of genetic screening in *Drosophila* has been developed that focuses specifically on systematic gain-of-function genetic screens (Rorth *et al*., Development (1998) 125:1049-1057). This method is based on a modular mis-expression system utilizing components of the GAL4/UAS system (described above) where a modified P element, termed an "enhanced P" (EP) element, is genetically engineered to contain a GAL4-responsive UAS element and promoter. Any other transposons can also be used for this system. The resulting transposon is used to randomly tag genes by insertional mutagenesis (similar to the method of P element mutagenesis described above). Thousands of transgenic *Drosophila* strains, termed EP lines, can be generated, each containing a specific UAS-tagged gene. This approach takes advantage of the preference of P elements to insert at the 5'-ends of genes. Consequently, many of the genes that are tagged by insertion of EP elements become operably fused to a GAL4-regulated promoter, and increased expression or mis-expression of the randomly tagged gene can be induced by crossing in a GAL4 driver gene.

Systematic gain-of-function genetic screens for modifiers of phenotypes induced by mutation or mis-expression of a subject gene can be performed by crossing several thousand *Drosophila* EP lines individually into a genetic background containing a mutant or mis-expressed subject gene, and further containing an appropriate GAL4 driver transgene. It is also possible to remobilize the EP elements to obtain novel insertions. The progeny of these crosses are then analyzed for enhancement or suppression of the original mutant phenotype as described above. Those identified as having mutations that interact with the subject gene can be tested further to verify the reproducibility and specificity of this genetic interaction. EP insertions that demonstrate a specific genetic interaction with a mutant or mis-expressed subject gene, have a physically tagged new gene which can be identified and sequenced using PCR or hybridization screening methods, allowing the isolation of the genomic DNA adj acent to the position of the EP element insertion.

### EXAMPLES

The following examples describe the isolation and cloning of the nucleic acid sequence of SEQ ID NOS:1, 3, 5, 7, 9, and 11, and how these sequences, and derivatives and fragments thereof, as well as other pathway nucleic acids and gene products can be used for genetic studies to elucidate mechanisms of a pathway involving a subject protein as well as the discovery of potential pharmaceutical or pesticidal agents that interact with the pathway.

These Examples are provided merely as illustrative of various aspects of the invention and should not be construed to limit the invention in any way.

### Example 1: Preparation of Drosophila cDNA Library

A *Drosophila* expressed sequence tag (EST) cDNA library was prepared as follows. Tissue from mixed stage embryos (0-20 hour), imaginal disks and adult fly heads were collected and total RNA was prepared. Mitochondrial rRNA was removed from the total RNA by hybridization with biotinylated rRNA specific oligonucleotides and the resulting RNA was selected for polyadenylated mRNA. The resulting material was then used to construct a random primed library. First strand cDNA synthesis was primed using a six nucleotide random primer. The first strand cDNA was then tailed with terminal transferase to add approximately 15 dGTP molecules. The second strand was primed using a primer which contained a Not1 site followed by a 13 nucleotide C-tail to hybridize to the G-tailed first strand cDNA. The double stranded cDNA was ligated with BstX1 adaptors and digested with Not1. The cDNA was then fractionated by size by electrophoresis on an agarose gel and the cDNA greater than 700 bp was purified. The cDNA was ligated with Not1, BstX1 digested pCDNA-sk+ vector (a derivative of pBluescript, Stratagene) and used to transform *E. coli* (XL1blue). The final complexity of the library was 6 X 10⁶ independent clones.

The cDNA library was normalized using a modification of the method described by Bonaldo *et al*. (Genome Research (1996) 6:791-806). Biotinylated driver was prepared from the cDNA by PCR amplification of the inserts and allowed to hybridize with single stranded plasmids of the same library. The resulting double-stranded forms were removed using strepavidin magnetic beads, the remaining single stranded plasmids were converted to double stranded molecules using Sequenase (Amersham, Arlington Hills, IL), and the plasmid DNA stored at -20°C prior to transformation. Aliquots of the normalized plasmid library were used to transform *E. coli* (XL1blue or DH10B), plated at moderate density, and the colonies picked into a 384-well master plate containing bacterial growth media using a Qbot robot (Genetix, Christchurch, UK). The clones were allowed to grow for 24 hours at 37° C then the master plates were frozen at -80° C for storage. The total number of colonies picked for sequencing from the normalized library was 240,000. The master plates were used to inoculate media for growth and preparation of DNA for use as template in sequencing reactions. The reactions were primarily carried out with primer that initiated at the 5' end of the cDNA inserts. However, a minor percentage of the clones were also sequenced from the 3' end. Clones were selected for 3' end sequencing based on either further biological interest or the selection of clones that could extend assemblies of contiguous sequences ("contigs") as discussed below. DNA sequencing was carried out using ABI377 automated sequencers and used either ABI FS, dirhodamine or BigDye chemistries (Applied Biosystems, Inc., Foster City, CA).

Analysis of sequences were done as follows: the traces generated by the automated sequencers were base-called using the program "Phred" (Gordon, Genome Res. (1998) 8:195-202), which also assigned quality values to each base. The resulting sequences were trimmed for quality in view of the assigned scores. Vector sequences were also removed. Each sequence was compared to all other fly EST sequences using the BLAST program and a filter to identify regions of near 100% identity. Sequences with potential overlap were then assembled into contigs using the programs "Phrap", "Phred" and "Consed" (Phil Green, University of Washington, Seattle, Washington; http://bozeman.mbt.washington.edu/phrap.docs/phrap.html). The resulting assemblies were then compared to existing public databases and homology to known proteins was then used to direct translation of the consensus sequence. Where no BLAST homology was available, the statistically most likely translation based on codon and hexanucleotide preference was used. The Pfam (Bateman *et al*., Nucleic Acids Res. (1999) 27:260-262) and Prosite (Hofmann *et al*., Nucleic Acids Res. (1999) 27(1):215-219) collections of protein domains were used to identity motifs in the resulting translations. The contig sequences were archived in an Oracle-based relational database (FlyTag™, Exelixis Pharmaceuticals, Inc., South San Francisco, CA).

### Example 2: Cloning of Subject Nucleic Acid Molecules

Unless otherwise noted, the PCR conditions used for cloning the subject nucleic acid molecules were as follows: A denaturation step of 94° C, 5 min; followed by 35 cycles of: 94° C, 1 minute; 55° C, 1 minute; 72° C, 1 minute; then, a final extension at 72° C, 10 minutes.

All DNA sequencing reactions were performed using standard protocols for the BigDye sequencing reagents (Applied Biosystems, Inc.) and products were analyzed using ABI 377 DNA sequencers. Trace data obtained from the ABI 377 DNA sequencers was analyzed and assembled into contigs using the Phred-Phrap programs.

Well-separated, single colonies were streaked on a plate and end-sequenced to verify the clones. Single colonies were picked and the enclosed plasmid DNA was purified using Qiagen REAL Preps (Qiagen, Inc., Valencia, CA). Samples were then digested with appropriate enzymes to excise insert from vector and determine size, for example the vector pOT2, (www.fruitfly.org/EST/pOT2vector.html) and can be excised with Xhol/EcoRI; or pBluescript (Stratagene) and can be excised with BssH II. Clones were then sequenced using a combination of primer walking and *in vitro* transposon tagging strategies.

For primer walking, primers were designed to the known DNA sequences in the clones, using the Primer-3 software (Steve Rozen, Helen J. Skaletsky (1998) Primer3. Code available at http://www-genome.wi.mit.edu/genome_software/other/primer3.html.). These primers were then used in sequencing reactions to extend the sequence until the full sequence of the insert was determined.

The GPS-1 Genome Priming System *in vitro* transposon kit (New England Biolabs, Inc., Beverly, MA) was used for transposon-based sequencing, following manufacturer's protocols. Briefly, multiple DNA templates with randomly interspersed primer-binding sites were generated. These clones were prepared by picking 24 colonies/clone into a Qiagen REAL Prep to purify DNA and sequenced by using supplied primers to perform bidirectional sequencing from both ends of transposon insertion.

Sequences were then assembled using Phred/Phrap and analyzed using Consed. Ambiguities in the sequence were resolved by resequencing several clones. This effort resulted in identification of various nucleic acid molecules, which are described in detail below.

### dmNTT4

A dmNTT4 nucleic acid molecule was identified in a contiguous nucleotide sequence of 3 977 bases in length, encompassing an open reading frame (ORF) of 2025 nucleotides encoding a predicted protein of 675 amino acids. The ORF extends from base 239-2263 of SEQ ID NO:1.

### dmKSNF

A dmKSNF nucleic acid molecule was identified in a contiguous nucleotide sequence of 2473 bases in length, encompassing an open reading frame (ORF) of 1923 nucleotides encoding a predicted protein of 641 amino acids. The ORF extends from base 249-2173 of SEQ ID NO:3.

### dmSNF

A dmSNF nucleic acid molecule was identified in a contiguous nucleotide sequence of 1.969 kilobases in length, encompassing an open reading frame (ORF) of 1209 nucleotides encoding a predicted protein of 403 amino acids. The ORF extends from base 498-1706 of SEQ ID NO:5.

### dmSNF2

A dmSNF2 nucleic acid molecule was identified in a contiguous nucleotide sequence of 2.03 kilobases in length, encompassing an open reading frame (ORF) of 1779 nucleotides encoding a predicted protein of 593 amino acids. The ORF extends from base 57-1838 of SEQ ID NO:7.

### dmSNF3

A dmSNF3 nucleic acid molecule was identified in a contiguous nucleotide sequence of 2.108 kilobases in length, encompassing an open reading frame (ORF) of 1917 nucleotides encoding a predicted protein of 639 amino acids. The ORF extends from base 50-1969 of SEQ ID NO:9.

### dmGAT

A dmGAT nucleic acid molecule was identified in a contiguous nucleotide sequence of 2.874 kilobases in length, encompassing an open reading frame (ORF) of 1908 nucleotides encoding a predicted protein of 636 amino acids. The ORF begins at base 107-109 of SEQ ID NO:11.

### Example 3: Analysis of Subject Nucleic Acid Sequences

Upon completion of cloning, the sequences were analyzed using the Pfam and Prosite, and TopPred programs (Claros and von Heijne, Comput Appl Biosci (1994) 10(6):685-686); PSORT (Nakai K., and Horton P., Trends Biochem Sci, 1999, 24:34-6), and Pfam programs..

### dmNTT4

TopPred predicted 12 transmembrane domains, at amino acids 59-79, 91-111, 132-152, 217-237, 244-264, 294-314, 329-349, 428-448, 474-494, 506-526, 552-572,, and 596-616, corresponding to nucleotides 413-475, 509-571, 632-694, 887-949, 968-1030, 1118-1180, 1223-1285, 1520-1582, 1658-1720,1754-1816,1892-1954, and 2024-2086, respectively. Pfam predicted two sodium:neurotransmitter symporter (SNF) domains (PF00209) at amino acids 55-352, and 395-617, corresponding to nucleotides 403-1294, and 1423-2089, respectively.

Nucleotide and amino acid sequences for dmNTT4 nucleic acid sequence and its encoded protein were searched against all available nucleotide and amino acid sequences in the public databases, using BLAST (Altschul *et al*., *supra*). Table 1 below summarizes the results. The 5 most similar sequences are listed.

**TABLE 1**

| **DNA BLAST** | |
|---|---|
| **GI#** | **DESCRIPTION** |
| 6437322=AC014013 | *Drosophila melanogaster*, *** SEQUENCING IN PROGRESS ***, in ordered pieces |
| 5670377= AC008231 | *Drosophila melanogaster* chromosome 2 clone BACR12013 (D946) RPCI-98 12.O.13 map 57B-57D strain y; cnbw sp, *** SEQUENCING IN PROGRESS ***, 158 unordered pieces |
| 2700336=AA697407 | *Drosophila melanogaster* cDNA clone HL02420 5prime, mRNA sequence |
| 4203539= AI389528 | *Drosophila melanogaster* cDNA clone GH20734 5prime, mRNA sequence |
| 3942415= AI293008 | *Drosophila melanogaster* cDNA clone GH16070 5prime, mRNA sequence |
| **PROTEIN BLAST** | |

| **GI#** | **DESCRIPTION** |
|---|---|
| 285126= S27043 | neurotransmitter transport protein - rat |
| 262843= AAB24776 | neurotransmitter transporter [rats, brain, Peptide, 727 aa] [*Rattus* sp.] |
| 2143866= I56506 | Na+/Cl(-)-dependent neurotransmitter transporter, brain - rat |
| 545078= AAC60673 | Na+/Cl(-)-dependent neurotransmitter transporter [rats, brain, Peptide, 727 aa] [*Rattus* sp.] |
| 4691420= BAA77223 | orphan transporter short splicing variant [*Bos taurus*] |

The protein encoded by dmNTT4 is an SNF member and is most closely related to mammalian NTT4 orphan transporters. It is predicted to have the same structure as other SNF members: a cytoplasmic N-terminus followed by 3 TM domains, an extracellular hydrophilic loop, 9 more TM domains, and a cytoplasmic carboxy-terminus. Both PROSITE and Pfam analysis tools place dmNTT4 in the SNF family (Pfam score = 730.2, e-value = 9.3e-216). The dmNTT4 protein is 41% identical to rat NTT4 transporter. Moreover, it is 59% identical (192/322) and 77% similar (250/322) over the highly conserved region of amino acid residues 54-375. Although the function and ligand of this transporter are unknown, the importance of other SNF members as drug targets suggests that dmNTT4 is also a drug and/ar pesticide target as well.

BLAST results for the dmNTT4 amino acid sequence indicate 15 amino acid residues as the shortest stretch of contiguous amino acids that is novel with respect to prior art sequences and 35 amino acids as the shortest stretch of contiguous amino acids for which there are no sequences contained within public database sharing 100% sequence similarity.

### dmKSNF

Twelve transmembrane domains were predicted, at amino acids 42-62, 71-91, 124-144, 231-251, 258-278, 308-328, 346-366, 401-421, 441-461, 473-493, 515-535, and 552-572 (corresponding to nucleotides 372-434, 462-521, 618-680, 939-1001,1020-1082,1170-1232,1284-1346,1449-1511, 1569-1631, 1665-1727, 1791-1853, and 1902-1964, respectively). PFAM also predicted an SNF (sodium neurotransmitter family) domain (PF00209) at amino acids 38-591 (nucleotides 355-2024).

Nucleotide and amino acid sequences for the dmKSNF nucleic acid sequences and their encoded proteins were searched against all available nucleotide and amino acid sequences in the public databases, using BLAST (Altschul *et al*., *supra*)*.* Table 2 below summarizes the results. The 5 most similar sequences are listed.

**TABLE 2**

| **DNA BLAST** | |
|---|---|
| **GI#** | **DESCRIPTION** |
| 6436935= AC014400 | *Drosophila melanogaster*, *** SEQUENCING IN PROGRESS ***, in ordered pieces |
| 3252835=AF006063 | *Manduca sexta* potassium coupled amino acid transporter (KAAT1) mRNA, complete cds |
| 3818342= AC005894 | *Drosophila melanogaster*, chromosome 2R, region 46A1-46B2, P1 clones DS00050, DS00191, and DS05369, complete sequence |
| 6436158= AC015177 | *Drosophila melanogaster*, *** SEQUENCING IN PROGRESS ***, in ordered pieces |
| 6552872=AC018319 | *Drosophila melanogaster*, *** SEQUENCING IN PROGRESS ***, in ordered piece |

| **PROTEIN BLAST** | |
|---|---|
| **GI#** | **DESCRIPTION** |
| 6594619= AAF18560 | potassium/sodium dependent amino acid transporter; amino acid transporter with cation-dependent substrate specificity [*Manduca sexta*] |
| 3252836= AC24190 | potassium coupled amino acid transporter [*Manduca sexta*] |
| 4689410= AD27892 | glycine transporter type-2 [*Homo sapiens*] |
| 4003525= AC95145 | glycine transporter GLYT2 [*Homo sapiens*] |
| 1083684= A48716 | glycine transporter GLYT2 - rat |

The closest homolog predicted by BLAST analysis is a potassium/sodium dependent amino acid transporter from *Manduca sexta* with 48% identity and 63% sequence homology with dmKSNF. The BLAST analysis also revealed several other transporter proteins that share significant amino acid homology dmKSNF.

BLAST results for the dmKSNF amino acid sequence indicate 20 amino acid residues as the shortest stretch of contiguous amino acids that is novel with respect to published sequences and 29 amino acids as the shortest stretch of contiguous amino acids for which there are no sequences contained within public database sharing 100% sequence similarity.

### dmSNF

PSORT predicted four transmembrane domains at amino acids 120-136, 159-175, 222-238, and 292-308 (nucleotides 855-905,972-1022,1161-1211, and 1371-1421, respectively). Pfam also predicted a SNF (sodium neurotransmitter family) domain (PF00209) at amino acids 123-214 (nucleotides 864-1139).

Nucleotide and amino acid sequences for each of the dmSNF nucleic acid sequences and their encoded proteins were searched against all available nucleotide and amino acid sequences in the public databases, using BLAST (Altschul *et al*., *supra*). Table 3 below summarizes the results. The 5 most similar sequences are listed.

**TABLE 3**

| **DNA BLAST** | |
|---|---|
| **GI#** | **DESCRIPTION** |
| 6838511 = AC008328 | *Drosophila melanogaster* chromosome 2 clone BACR09A04 (D860) RPCI-98 09.A.4 map 28B-28C strain y; cn bw sp, *** SEQUENCING IN PROGRESS *** |
| 4426013 = AI520159 | *Drosophila melanogaster* embryo pOT2 Drosophila melanogaster cDNA clone LD40221 5prime |
| 4204059 = AI390048 | *Drosophila melanogaster* head pOT2 cDNA clone GH21504 5prime |
| 3865035 = AI257510 | *Drosophila melanogaster* larval-early pupal pOT2 cDNA clone LP05906 3prime similar to S76894: Dacp-1=cuticle protein |
| 3947129 = AI297722 | *Drosophila melanogaster* larval-early pupal pOT2 cDNA clone LP12087 5prime |

| **PROTEIN BLAST** | |
|---|---|
| **GI#** | **DESCRIPTION** |
| WO 9807854-A1, Example 1A | Fragment of the human GlyT-2 transporter encoded by clone phG2-9a |
| 4689410 = AAD27892 (AF142501) | glycine transporter type-2 [*Homo sapiens*] |
| 1083684 = A48716 | glycine transporter GLYT2 - rat |
| 3994801 = AAC88229 (AR010612) | Sequence 6 from patent US 5756348 |
| 1279843 = AAB01159 (U52688) | glycine transporter [*Bos taurus*] |

The closest homolog predicted by BLAST analysis is a glycine transporter from human, described in Patent WO 9807854-A1 (Example 1A), with 35% identity and 51% similarity to dmSNF. The BLAST analysis also revealed several other cation-dependent transporter neurotransmitter proteins of the sodium:neurotransmitter symporter family(SNF), from many vertebrate and invertebrate species, that share homologies throughout the SNF domain. BLAST results for the dmSNF amino acid sequence indicate 19 amino acid residues as the shortest stretch of contiguous amino acids that is novel with respect to published sequences and 23 amino acids as the shortest stretch of contiguous amino acids for which there are no sequences contained within public database sharing 100% sequence similarity.

### dmSNF2

PSORT predicted 12 transmembrane domains, located at amino acids 22-38; 52-68; 101-117; 190-206; 216-232; 265-281; 305-321; 358-374; 396-412; 432-448; 476-492; and 503-519 (nucleotides 120-170, 210-260, 357-407, 624-674, 702-752, 849-899, 969-1019,1128-1178,1242-1292,1350-1400,1482-1532, and 1563-1613, respectively). Additionally, Pfam predicted a SNF (sodium neurotransmitter family) domain (PF00209) at amino acids 15-548 (nucleotides 99-1700).

Nucleotide and amino acid sequences for each of the dmSNF2 nucleic acid sequences and their encoded proteins were searched against all available nucleotide and amino acid sequences in the public databases, using BLAST (Altschul *et al*., *supra*)*.* Table 4 below summarizes the results. The 5 most similar sequences are listed.

**TABLE 4**

| **DNA BLAST** | |
|---|---|
| **GI#** | **DESCRIPTION** |
| 6552872 = AC018319. | *Drosophila melanogaster*, *** SEQUENCING IN PROGRESS ***, in ordered pieces |
| 2794904 = AA538904 | *Drosophila melanogaster* embryo BlueScript cDNA clone LD18557 5prime |
| 6436158 = AC015177 | *Drosophila melanogaster*, *** SEQUENCING IN PROGRESS ***, in ordered pieces |
| 6957975 = AC007330 | *Drosophila melanogaster* chromosome 2 clone BACR30G11 (D600) RPCI-98 30.G.11 map 46C-46D strain y; cn bw sp, *** SEQUENCING IN PROGRESS *** |
| 3818342 = AC005894, AC005435, AC005431, and AC005441 | *Drosophila melanogaster*, chromosome 2R, region 46A1-46B2, P1 clones DS00050, DS00191, and DS05369, complete sequence |

| **PROTEIN BLAST** | |
|---|---|
| **GI#** | **DESCRIPTION** |
| 6594619 = AAF18560 (AF013963) | potassium/sodium dependent amino acid transporter; amino acid transporter with cation-dependent substrate specificity [*Manduca sexta*] |
| 3252836 = AAC24190 (AF006063) | potassium coupled amino acid transporter *[Manduca sexta*] |
| WO 9807854-A1, Claim 1 | Human GlyT-2 transporter protein R393G mutant |
| WO 9807854-A1, Claim 1 | Human GlyT-2 transporter protein F733S mutant |
| WO 9807854-A1, Claim 1 | Human GlyT-2 transporter protein I611V mutant |

The closest homolog predicted by BLAST analysis is a potassium/sodium dependent amino acid transporter from *Manduca sexta*, with 47% identity and 62% sequence homology to dmSNF2. The BLAST analysis also revealed several other cation-dependent transporter neurotransmitter proteins of the sodium:neurotransmitter symporter family(SNF), from many vertebrate and invertebrate species, that share homologies throughout the SNF domain.

BLAST results for the dmSNF2 amino acid sequence indicate 12 amino acid residues as the shortest stretch of contiguous amino acids that is novel with respect to published sequences and 19 amino acids as the shortest stretch of contiguous amino acids for which there are no sequences contained within public database sharing 100% sequence similarity.

### dmSNF3

PSORT predicted 11 transmembrane domains, located at amino acids 57-73; 87-103; 137-153; 239-255; 269-285; 355-371; 408-424; 448-464; 474-490; 522-538; and 564-580 (nucleotides 208-268; 308-368; 458-518; 764-824; 854-914; 1112-1172; 1274-1334; 1391-1451; 1469-1529; 1613-1673; and 1739-1799, respectively). Additionally, Pfam predicted a SNF (sodium neurotransmitter family) domain (PF00209) at amino acids 50-598 (nucleotides 197-1843).

Nucleotide and amino acid sequences for each of the dmSNF3 nucleic acid sequences and their encoded proteins were searched against all available nucleotide and amino acid sequences in the public databases, using BLAST (Altschul *et al*., *supra*). Table 5 below summarizes the results. The 5 most similar sequences are listed.

**TABLE 5**

| **DNA BLAST** | |
|---|---|
| **GI#** | **DESCRIPTION** |
| 1945594 = AC001648 (DMAC001648) | *Drosophila melanogaster* (P1 DS03431 (D102)) DNA sequence, complete sequence |
| 6554436 = AC017561 | *Drosophila melanogaster*, *** SEQUENCING IN PROGRESS ***, in ordered pieces |
| 3942484 = AI293077 | *Drosophila melanogaster* head pOT2 cDNA clone GH16161 5prime |
| 4201643 = AI387632 | *Drosophila melanogaster* head pOT2 Drosophila melanogaster cDNA clone GH18205 5prime |
| 6436158 = AC015177 | *Drosophila melanogaster*, *** SEQUENCING IN PROGRESS ***, in ordered pieces |

| **PROTEIN BLAST** | |
|---|---|
| **GI#** | **DESCRIPTION** |
| 3252836 = AAC24190 (AF006063) | potassium coupled amino acid transporter [*Manduca sexta*] |
| 6594619 = AAF18560 (AF013963) | potassium/sodium dependent amino acid transporter; amino acid transporter with cation-dependent substrate specificity [*Manduca sexta*] |
| WO 9807854-A1, Disclosure | Amino acid sequence of a human GlyT-2 transporter protein |
| WO 9807854-A1, Claim 1 | Human GlyT-2 transporter protein I279N mutant |
| WO 9807854-A1, Claim 1 | Human GlyT-2 transporter protein I611V mutant |

The closest homolog predicted by BLAST analysis is a potassium coupled amino acid transporter from *Manduca sexta* with 43% identity and 58% sequence homology to dmSNF3. The BLAST analysis also revealed several other cation-dependent neurotransmitter transporter proteins of the sodium neurotransmitter symporter family(SNF), from many vertebrate and invertebrate species, that share homologies throughout the conserved SNF domain. Taken together, this suggests that dmSNF3 functions as a SNF transporter and thus could be exploited as a target to control disease vectors and insect pests.

BLAST results for the dmSNF3 amino acid sequence indicate 12 amino acid residues as the shortest stretch of contiguous amino acids that is novel with respect to published sequences and 21 amino acids as the shortest stretch of contiguous amino acids for which there are no sequences contained within public database sharing 100% sequence similarity.

### dmGAT

TopPredII predicted 12 transmembrane domains, located at amino acids 63-83, 94-114, 150-170, 251-271, 275-295, 326-346, 365-385, 413-433, 459-479, 492-512, 536-556, and 573-593 (nucleotides 293-355, 386-448, 554-616, 857-919, 929-991, 1082-1144, 1199-1261, 1343-1405, 1481-1543, 1580-1642,1712-1774, and 1823-1885, respectively). Additionally, Pfam predicted a SNF (sodium neurotransmitter family) domain (PF00209) at amino acids 58-611 (nucleotides 278-1939).

Nucleotide and amino acid sequences for each of the dmGAT nucleic acid sequences and their encoded proteins were searched against all available nucleotide and amino acid sequences in the public databases, using BLAST (Altschul *et al*., *supra*). Table 6 below summarizes the results. The 5 most similar sequences are listed.

**TABLE 6**

| **DNA BLAST** | |
|---|---|
| **GI#** | **DESCRIPTION** |
| 6436266 = AC015069 | *Drosophila melanogaster*, ***** SEQUENCING IN PROGRESS ***, in ordered pieces |
| 6957595 = AC010668 | *Drosophila melanogaster* chromosome 4 clone BACR44L03 (D1071) RPCI-98 44.L.3 map 101F-102F strain y; cn bw sp, *** SEQUENCING IN PROGRESS ***, 86 unordered pieces |
| 6980158 = AC010576 | *Drosophila melanogaster* chromosome 4 clone BACR30L15 (D1073) RPCI-98 30.L.15 map 101F-102F strain y; cn bw sp, *** SEQUENCING IN PROGRESS ***, 78 unordered pieces |
| 695377 = L40373 (MOTGABANT) | *Manduca Sexta* GABA neurotransmitter transporter mRNA, complete cds |
| 6980157 = AC010669 | *Drosophila melanogaster chromosome* 4 clone BACR21N24 (D1072) RPCI-98 21.N.24 map 101F-102F strain y; cn bw sp, SEQUENCING IN PROGRESS ***, 75 unordered pieces |

| **PROTEIN BLAST** | |
|---|---|
| **GI#** | **DESCRIPTION** |
| 695378 = AAA92342 | GABA neurotransmitter transporter [*Manduca sexta*] |
| WO 9318143-A, Claim 1 | GAT-2 GABA transporter (rat) |
| WO 9318143-A, Claim 1 | Taurine transporter (rat) |
| 6019476 = AAB70922 (AF020194) | retinal taurine transporter [*Mus musculus*] |
| 3879356 =CAA98519 | Similarity to Human Na(+)/Cl(-)-dependent GABA transporter (SW:NTG1 HUMAN) [*Caenorhabditis elegans*] |

The closest homolog predicted by BLAST analysis is a GABA neurotransmitter transporter from *Manduca sexta* which shares 79% sequence identity and 89% homology with dmGAT protein. The BLAST analysis also revealed several GABA neurotransmitter transporter proteins, from many vertebrate and invertebrate species, that share a high degree of homology with dmGAT. Additionally, dmGAT shares homology with many other members of the sodium neurotransmitter symporter family (SNF). Taken together, this suggests that dmGAT functions as a GABA transporter and thus could be exploited as a target to control disease vectors and insect pests.

BLAST results for the dmGAT amino acid sequence indicate 43 amino acid residues as the shortest stretch of contiguous amino acids that is novel with respect to published sequences and 85 amino acids as the shortest stretch of contiguous amino acids for which there are no sequences contained within public database sharing 100% sequence similarity.

### Example 4: Testing of Pesticide Compounds for Activity Against a Subject Protein

cDNAs encoding any one of the subject proteins are cloned into mammalian cell culture-compatible vectors *(e.g.* pCDNA, Invitrogen, Carlsbad, CA), and the resultant constructs are transiently transfected into mammalian cells. The transiently transfected cell lines are typically used 24 to 48 hours following transfection for electrophysiology studies. Whole cell recordings, using the voltage clamp technique, are taken on the transfected cells versus cells transfected with vector only. Cells are voltage-clamped at 60 mV and continuously superfused with ND96 (96mM NaCl, 2mM KCl, 1.8mM CaCl₂ 1mM MgCl₂, 5mM HEPES, pH 7.5) containing varying concentrations of compounds. Current and fluxes are then measured.

Cell lines transiently transfected with dmKSNF can be assayed for uptake of radioactive or fluorescent bioamines such as glycine. In case of radioactive compounds, cells are incubated in 0.5µm radioactive (e.g., ³H-, or ¹⁴C-)glycine for 1 hour, washed with saline, and then assayed for compound uptake using a scintillation counter. Appropriate controls are comparison of this uptake to uptake in cells injected with water, or noninjected cells.

Cell lines transiently transfected with dmSNF, dmSNF2, or dmSNF3 can be assayed for uptake of radioactive or fluorescent neurotransmitter. In case of radioactive compounds, cells are incubated in 0.5µm radioactive (³H-, or ¹⁴C-) neurotransmitter for 1 hour, washed with saline, and then assayed for compound uptake using a scintillation counter. Appropriate controls are comparison of this uptake to uptake in cells injected with water, or noninjected cells.

Cell lines transiently transfected with dmGAT can be assayed for uptake of radioactive or fluorescent GABA. In case of radioactive compounds, cells are incubated in 0.5µm radioactive (³H-, or ¹⁴C-) GABA for 1 hour, washed with saline, and then assayed for compound uptake using a scintillation counter. Appropriate controls are comparison of this uptake to uptake in cells injected with water, or noninjected cells.

## Claims

1. An isolated nucleic acid molecule of less than about 15 kb in size comprising a nucleic acid sequence that encodes an invertebrate receptor polypeptide and that shares at least about 75% nucleotide sequence identity with the sequence set forth in SEQ ID NO:11, or the complement thereof.

2. The nucleic acid of claim 1, wherein the nucleic acid comprises a nucleic acid sequence that encodes an invertebrate receptor polypeptide and that shares at least about 80% nucleotide sequence identity with the sequence set forth in SEQ ID NO:11, or the complement thereof.

3. The nucleic acid of claim 1, wherein the nucleic acid comprises a nucleic acid sequence that encodes an invertebrate receptor polypeptide and that shares at least about 90% nucleotide sequence identity with the sequence set forth in SEQ ID NO:11, or the complement thereof.

4. The nucleic acid of claim 1, wherein the nucleic acid comprises a nucleic acid sequence that encodes an invertebrate receptor polypeptide and that shares at least about 95% nucleotide sequence identity with the sequence set forth in SEQ ID NO:11, or the complement thereof.

5. The nucleic acid of claim 1, wherein the nucleic acid comprises a nucleic acid sequence that encodes an invertebrate γ-aminobutyric acid (GABA) transporter polypeptide, wherein said GABA transporter polypeptide comprises an amino acid sequence having at least about 85% amino acid sequence identity to the amino acid sequence set forth in SEQ ID NO:12.

6. The nucleic acid of claim 1, wherein the nucleic acid comprises a nucleic acid sequence that encodes an invertebrate γ-aminobutyric acid (GABA) transporter polypeptide, wherein said GABA transporter polypeptide comprises an amino acid sequence having at least about 90% amino acid sequence identity to the amino acid sequence set forth in SEQ ID NO:12.

7. The nucleic acid of claim 1, wherein the nucleic acid comprises a nucleic acid sequence that encodes an invertebrate γ-aminobutyric acid (GABA) transporter polypeptide, wherein said GABA transporter polypeptide comprises an amino acid sequence having at least about 95% amino acid sequence identity to the amino acid sequence set forth in SEQ ID NO:12.

8. The nucleic acid of claim 1, wherein the nucleic acid comprises a nucleic acid sequence that encodes an invertebrate γ-aminobutyric acid (GABA) transporter polypeptide, wherein said GABA transporter polypeptide comprises an amino acid sequence having at least about 95% amino acid sequence identity to at least 100 contiguous amino acid of the amino acid sequence set forth in SEQ ID NO:12.

9. The nucleic acid of claim 1, wherein the nucleic acid comprises a nucleic acid sequence that encodes an invertebrate γ-aminobutyric acid (GABA) transporter polypeptide, wherein said GABA transporter polypeptide comprises an amino acid sequence having the amino acid sequence set forth in SEQ ID NO:12.

10. A vector comprising the nucleic acid molecule of any one of claims 1-9.

11. A host cell comprising a vector of claim 10.

12. A process for producing an invertebrate γ-aminobutyric acid (GABA) transporter polypeptide, comprising culturing a host cell of claim 11 under conditions suitable for expression of said protein; and recovering said protein.

13. A purified protein comprising an amino acid having at least about 80% sequence identity to the sequence set forth in SEQ ID NO:12.

14. A purified protein comprising an amino acid having at least about 85% sequence identity to the sequence set forth in SEQ ID NO:12.

15. A purified protein comprising an amino acid having at least about 90% sequence identity to the sequence set forth in SEQ ID NO:12.

16. A purified protein comprising an amino acid having at least about 95% sequence identity to the sequence set forth in SEQ ID NO:12.

17. A purified protein comprising an amino acid having the sequence set forth in SEQ ID NO:12.

18. A method for detecting a candidate compound that interacts with an invertebrate invertebrate γ-aminobutyric acid (GABA) transporter polypeptide or fragment thereof, said method comprising contacting said protein or fragment with one or more candidate molecules; and detecting any interaction between said candidate compound and said protein or fragment; wherein the amino acid sequence of said protein comprises an amino acid sequence amino acid sequence which is at least about 80% identical to the sequence set forth in SEQ ID NO:12.

19. The method of claim 18 wherein said candidate compound is a putative pesticidal or pharmaceutical agent.

20. The method of claim 18 wherein said contacting comprises administering said candidate compound to cultured host cells that have been genetically engineered to express said protein.

21. The method of claim 18 wherein said contacting comprises administering said candidate compound to a metazoan invertebrate organism that has been genetically engineered to express said proteins.

22. The method of claim 18 wherein said candidate compound is a putative pesticide and said detecting entails observing modulations of a biological activity of said result in organism lethality.

23. The method of claim 22 wherein said organism is an insect or worm.

24. A first animal that is an insect or a worm that has been genetically modified to express or mis-express an invertebrate γ-aminobutyric acid (GABA) transporter polypeptide, or the progeny of said animal that has inherited said protein expression or mis-expression, wherein said protein comprises an amino acid sequence amino acid sequence which is at least about 80% identical to the sequence set forth in SEQ ID NO:12.

25. A method for studying invertebrate γ-aminobutyric acid (GABA) transporter polypeptide comprising detecting the phenotype caused by the expression or mis-expression of said invertebrate (GABA) transporter polypeptide in the first animal of claim 24.

26. The method of Claim 25 additionally comprising observing a second animal having the same genetic modification as said first animal which causes said expression or mis-expression of said protein, and wherein said second animal additionally comprises a mutation in a gene of interest, wherein differences, if any, between the phenotype of said first animal and the phenotype of said second animal identifies the gene of interest as capable of modifying the function of the gene encoding said protein.

27. The method of Claim 25 additionally comprising administering one or more candidate compounds to said animal or its progeny and observing any changes in invertebrate (GABA) transporter polypeptide activity of said animal or its progeny.


SEQUENCE LISTING

<110> GENOPTERA, LLC.

<120> Nucleic acids and polypeptides of
  invertebrate cell surface receptors and methods of use

<130> EXEL-003WO

<140> US 60/173,929
<141> 1999-12-30

<150> US 60/189,399
<151> 2000-03-15

<150> US 60/191,688
<151> 2000-03-23

<150> US 60/191,687
<151> 2000-03-23

<150> US 60/191,686
<151> 2000-03-23

<150> US 60/191,695
<151> 2000-03-23

<160> 12

<170> FastSEQ for Windows Version 4.0

<210> 1
<211> 3977
<212> DNA
<213> Drosophila melanogaster

<400> 1
   ttcggcacga gaaatcgagc aaagctccgt gagaaaaacg caaaaattcg tcgaacgcaa 60
   ttgatatcga gcgtttgtct ggattaaagc ggaaatcagg gataatcagt gattttccaa 120
   agcgcgagag tgtgtgtgtg aaaaatccgc tttaaagcag tctggaattt taaatcccac 180
   agccaagaca aacttcgacg gtaaccacgg acacttcgcc aggaatatcc gcacgttgat 240
   ggctgccacc aaaatcatag atgcgccgcg caacggccac gagatggcgc cgctgaacac 300
   acgggcacgg ggcgatggaa cccacggggt gaccatcgtc cttaccgccc cccagcgcaa 360
   ctccgtgcaa tcggtggaca ttccgggcaa tgagccggaa cgcgccgcct ggagcggcaa 420
   gatgcagttc ttcctcagca tcatcggata ctcggtgggc ctcggcaata tctggcggtt 480
   tccctacctt tgccaacaga atggaggcgg tgcattcctg atacccttta tggttatgct 540
   gatactggag ggcataccgc tgttcctaat cgagttggga atcgggcaga gaatgcgatt 600
   gggtgccttg ggtgtgtgga acaccatcca tccctggctg ggtggcatcg gaatctcgtc 660
   gtgcatagtg acgctatttg tggctctcta ttacaacgtg atcatcacgt gggttttctt 720
   ctaccttttc aacagttttc ggtatccgct gccctggtca tcttgtccat tgaacggcac 780
   aggatttgag ttggaggagt gcgccaaatc ctcggagacg acgtacttct ggtaccgcac 840
   caccttggat gcggcaccgt ccatggatga gcctggtggc ctcaagtggt ggatcgtgct 900
   ctgcctgatg ctgtcctgga ccattgtctt cttcatcgta atgaagggca tccagagctc 960
   cggcaaggtt gtctacttca cctcgctctt tccctacatt gtgctgacca tcttcttcat 1020
   tcgcggcatc acgttgagag gagcgggcgc tggactgatg cacatgtata cgccaaaggt 1080
   ggaaaaatta ctggagccca ccgtttggct ggatgcagcc acacaggtct tttactcctt 1140
   tggcctggcc tttggatccc tgatcgcctt cgggagctac aacacgccga agaacaactg 1200
   cgtgcgggat gtgctcctgg tgtccgtgtg caatgcagtt acggctatct acgccagcgt 1260
   ggtcatcttc gccattctgg gcttcaaggc caccgtcaat gtggaccgtt gtgtagccag 1320
   taacacggaa atcctggtga aaaacaaact gctgggcctg aatgacacac agggctacga 1380
   gcaagccatg agtctactga atggcactga gttgctccgc ctgcaattga gcgaatgcag 1440
   cttggcccac gaactggaca atgctgccga gggcaccggt ttggccttca tcgtattcac 1500
   ccaggccatc gtggagctgc ccggtgctcc attctgggcc gtccttttct tcaccatgct 1560
   gctgtcgctg ggactgggat cacagatcgg catcctggag ggcatgctgt gcaccctctt 1620
   cgacattgac attatcaagc gggttaagaa acagcatgtc actggcgtgg tgtgtctctt 1680
   ctgcttcatt gtcggtttca tcttctgcac aggtgccggg gagtactggc tcaagatgtt 1740
   cgactctttt gccggcacca ttggtctcgt ggtggtggcg ctgatggaga tgatagccgt 1800
   gatctttatc tacggacacg agcgcttcac cgaggacatt ttccagatga ctggctaccg 1860
   acctggtcgt tactggcagt ggacctggcg gtacattggt cccgtcatta tggtctgcat 1920
   cttggtgtct tcagtggtct tcatggtcat tagaaatcca acttacggtg cttggaatgc 1980
   cgatttgggt atgattgagc agaagagcta ccccaattgg gtgatgggca tcgcactatc 2040
   catgatcctg gctggcgtcc tgcccatgcc catcgtgttc ctcatgcgca gcttccagtg 2100
   cctgaaggtg gacctggaca tccaccaagg atcgattcgt cgcaatgaaa caaccgcctc 2160
   caccaaggag atgattgaca atgatgacga taatatgagt ccggatatgc caccgcaaga 2220
   ttcactagcg gcgacacgct tcacaattgg agactttgaa aattagaacg aaccgaacct 2280
   gaactcaaaa ccgaagccat ggatacgaac ctaagtgcag cgccatgtct tagagaaccc 2340
   caaagaaatc agcaaccagt taaaactagg atagctctaa aaaatagttc actaacgccc 2400
   ttcgcatggc ccagaaacac aaaacaccga acactgaaca cccagaaaaa ccgaaaaaaa 2460
   aactaaggac atggctcgac tcaagtagaa accaattgct catcctcggt taccaagcaa 2520
   ttacccgtac tcaataccac aaagaactaa tcaacgcaca gcaattaatc gtagctagca 2580
   aacaatagca aacagctctt ttaaacgaaa accggataag gccagaacaa ttatttacaa 2640
   aacctaagtt tagggaaaag actagcaaaa tcgcttgaag gctcaacagg agtagggaaa 2700
   tgagtgtaca tagatttagg cagttcatgg acatatataa acccaattgg cggctgatcg 2760
   tggctaatat gcaggacgac gaggatgacg agaacgattt tagcggtccg gcgctgggtg 2820
   gtcatgtcaa gacccagagc gatttctccg acgacgacga ggaggacaag cccatgacca 2880
   tgcggatgat gatgatgcgt ccgacaacaa caaccaagac atcgaacgtg ctaaatgtgc 2940
   caacaagcag aaagaacaac tataagaagg aggcgtacga tgtctagaaa agccacagcc 3000
   ccactcccag gcgcagctat ggaaacgaaa gcataagata tggataaaga tacagataca 3060
   ggtagagata gaaggccagg tacaagtcca gatacaggtc caggtgtgaa ccgatacaaa 3120
   atggcaagtg aaaaacaaaa accaatttaa ctaagacgag cggtaagatg ggtgcgtgcc 3180
   gagctgagcg caaacattta taaatattta ctataagtat tttaacttaa tttaaatcta 3240
   aactaagtaa ttaccagaat aaacagccga agaattgcat tcgagtgcgt aactaaaatt 3300
   gttaaatcgt attttaacac aaactgaact aagcccaaac taaaactaaa tttaacaaac 3360
   tgtgtacgag tacaatgtgc cgcaggagga taggtattat ctaaaacggg ttgcaaatgg 3420
   tttaaattcg attatgtagc atttagttgg aatgcttaaa gctttaccct tagacactcg 3480
   aacacacaaa actaaaagta tttccgactc ccacagccgc actccttttc tcccatcccc 3540
   aatatcccca acaattcccc cacaattccc ccctcccccg gagaaagctc ttcgacggca 3600
   aacgcttata gtcgacttag ccagcgcatt tccaaagagt taaactcaac atttagtttt 3660
   agtagagatc tgccgttttg ttttgacctc aagtagaact catcagacct acaattaatc 3720
   tatgtatgta atctaagagg agttacacga tttacaatta ctttaaactg attaagaacg 3780
   ttgagacaca atattaacac ttcgagttct agttgagtta aacaaatacc tatacttact 3840
   atactgatta gagaatgata catggaatgt gtgatattct aaataatatc ctcaatgttt 3900
   agatgttaag actcttcatc cacagcccca aagtcgtcca ctcaatctta ttccccgtta 3960
   aaaaaaaaaa aaaaaaa 3977

<210> 2
<211> 675
<212> PRT
<213> Drosophila melanogaster

<400> 2
Met Ala Ala Thr Lys Ile Ile Asp Ala Pro Arg Asn Gly His Glu Met
1               5                   10                  15
Ala Pro Leu Asn Thr Arg Ala Arg Gly Asp Gly Thr His Gly Val Thr
            20                  25                  30
Ile Val Leu Thr Ala Pro Gln Arg Asn Ser Val Gln Ser Val Asp Ile
        35                  40                  45
Pro Gly Asn Glu Pro Glu Arg Ala Ala Trp Ser Gly Lys Met Gln Phe
    50                  55                  60
Phe Leu Ser Ile Ile Gly Tyr Ser Val Gly Leu Gly Asn Ile Trp Arg
65                  70                  75                  80
Phe Pro Tyr Leu Cys Gln Gln Asn Gly Gly Gly Ala Phe Leu Ile Pro
                85                  90                  95

Phe Met Val Met Leu Ile Leu Glu Gly Ile Pro Leu Phe Leu Ile Glu
            100                 105                 110
Leu Gly Ile Gly Gln Arg Met Arg Leu Gly Ala Leu Gly Val Trp Asn
        115                 120                 125
Thr Ile His Pro Trp Leu Gly Gly Ile Gly Ile Ser Ser Cys Ile Val
    130                 135                 140
Thr Leu Phe Val Ala Leu Tyr Tyr Asn Val Ile Ile Thr Trp Val Phe
145                 150                 155                 160
Phe Tyr Leu Phe Asn Ser Phe Arg Tyr Pro Leu Pro Trp Ser Ser Cys
                165                 170                 175
Pro Leu Asn Gly Thr Gly Phe Glu Leu Glu Glu Cys Ala Lys Ser Ser
            180                 185                 190
Glu Thr Thr Tyr Phe Trp Tyr Arg Thr Thr Leu Asp Ala Ala Pro Ser
        195                 200                 205
Met Asp Glu Pro Gly Gly Leu Lys Trp Trp Ile Val Leu Cys Leu Met
    210                 215                 220
Leu Ser Trp Thr Ile Val Phe Phe Ile Val Met Lys Gly Ile Gln Ser
225                 230                 235                 240
Ser Gly Lys Val Val Tyr Phe Thr Ser Leu Phe Pro Tyr Ile Val Leu
                245                 250                 255
Thr Ile Phe Phe Ile Arg Gly Ile Thr Leu Arg Gly Ala Gly Ala Gly
            260                 265                 270
Leu Met His Met Tyr Thr Pro Lys Val Glu Lys Leu Leu Glu Pro Thr
        275                 280                 285
Val Trp Leu Asp Ala Ala Thr Gln Val Phe Tyr Ser Phe Gly Leu Ala
    290                 295                 300
Phe Gly Ser Leu Ile Ala Phe Gly Ser Tyr Asn Thr Pro Lys Asn Asn
305                 310                 315                 320
Cys Val Arg Asp Val Leu Leu Val Ser Val Cys Asn Ala Val Thr Ala
                325                 330                 335
Ile Tyr Ala Ser Val Val Ile Phe Ala Ile Leu Gly Phe Lys Ala Thr
            340                 345                 350
Val Asn Val Asp Arg Cys Val Ala Ser Asn Thr Glu Ile Leu Val Lys
        355                 360                 365
Asn Lys Leu Leu Gly Leu Asn Asp Thr Gln Gly Tyr Glu Gln Ala Met
    370                 375                 380
Ser Leu Leu Asn Gly Thr Glu Leu Leu Arg Leu Gln Leu Ser Glu Cys
385                 390                 395                 400
Ser Leu Ala His Glu Leu Asp Asn Ala Ala Glu Gly Thr Gly Leu Ala
                405                 410                 415
Phe Ile Val Phe Thr Gln Ala Ile Val Glu Leu Pro Gly Ala Pro Phe
            420                 425                 430
Trp Ala Val Leu Phe Phe Thr Met Leu Leu Ser Leu Gly Leu Gly Ser
        435                 440                 445
Gln Ile Gly Ile Leu Glu Gly Met Leu Cys Thr Leu Phe Asp Ile Asp
    450                 455                 460
Ile Ile Lys Arg Val Lys Lys Gln His Val Thr Gly Val Val Cys Leu
465                 470                 475                 480
Phe Cys Phe Ile Val Gly Phe Ile Phe Cys Thr Gly Ala Gly Glu Tyr
                485                 490                 495
Trp Leu Lys Met Phe Asp Ser Phe Ala Gly Thr Ile Gly Leu Val Val
            500                 505                 510
Val Ala Leu Met Glu Met Ile Ala Val Ile Phe Ile Tyr Gly His Glu
        515                 520                 525
Arg Phe Thr Glu Asp Ile Phe Gln Met Thr Gly Tyr Arg Pro Gly Arg
    530                 535                 540
Tyr Trp Gln Trp Thr Trp Arg Tyr Ile Gly Pro Val Ile Met Val Cys
545                 550                 555                 560
Ile Leu Val Ser Ser Val Val Phe Met Val Ile Arg Asn Pro Thr Tyr
                565                 570                 575
Gly Ala Trp Asn Ala Asp Leu Gly Met Ile Glu Gln Lys Ser Tyr Pro
            580                 585                 590

Asn Trp Val Met Gly Ile Ala Leu Ser Met Ile Leu Ala Gly Val Leu
        595                 600                 605
Pro Met Pro Ile Val Phe Leu Met Arg Ser Phe Gln Cys Leu Lys Val
    610                 615                 620
Asp Leu Asp Ile His Gln Gly Ser Ile Arg Arg Asn Glu Thr Thr Ala
625                 630                 635                 640
Ser Thr Lys Glu Met Ile Asp Asn Asp Asp Asp Asn Met Ser Pro Asp
                645                 650                 655
Met Pro Pro Gln Asp Ser Leu Ala Ala Thr Arg Phe Thr Ile Gly Asp
            660                 665                 670
Phe Glu Asn
        675

<210> 3
<211> 2473
<212> DNA
<213> Drosophila melanogaster

<400> 3
cgcgcctcca gcaccgccaa gtagccaaaa tccacagccc aaattacgaa aatattacag 60
aaaactaaac cctgaaactg cagttggacg ctaacccaac caaccgtaac caagggctgg 120
ccaaatgcaa tgtgctaatt actattaaca aaaaaaaaaa aaataaataa ttagtaatta 180
aataagcaaa acgcgagtag ccgtcaactg ttttcattaa ccgggaaaat taacaagtgg 240
ctagaaaaat ggaattgaaa ggagttcagc catcgaatgg cagctcaaat ggaagtggaa 300
atggagccac caatgctgca tcaacggaaa aaacagatgc cgagaagccg acggcggagc 360
gcaccaattg gggcaatggc ctggagtttc taatgtcctg catatcggtg tccgtgggat 420
tgggcaacgt ctggaggttc ccgttcacgg cctatgagaa tggaggaggc gccttcctta 480
taccctacat catagtgctc tttctgatcg gcaaacccat gtactatttg gagatgatca 540
tgggccagtt cacgagccag ggaacggtga agatttggtc ggtggtgccg ggatttgtgg 600
gcgtgggcta tggccaggcc ttcggcacca tctgcatcat ctcgtactat tcctcgctgt 660
tggcgctgac cctctactat ctctttgtgt ccttccaatc ggaattgccg tggtcctact 720
gtcgcgatga gtggaccaat tgcgtgaact cgaggccgca ggagtatgtg gacaatctgc 780
ttacgggcgt atccttggcc aatgaatcgg ccagaaatct cagcggcatt gtggccaacg 840
atgagacaga gaaactccag agcagctccg aactctattt cctgaatgta gtgatcaagg 900
agaagctgga catctcggac ggcgttggtg atcccgattg gaagctgaca cttgccctgt 960
tcgtcgcctg ggtggtcatc tttctggtga tcatgcgagg ggtgaagagt tccggcaagg 1020
cggcctactt cctggccctg ttcccctacg tggtgctctt cgtcctgctg atccgtgccg 1080
ttaccttgga gggagcacgc gatggcattc tgttctttct ggagccacag tggggtgaac 1140
tcttgaaccc caccgtttgg aaggaggccg ttgtgcagtg cttcttctcg ctggccgtgg 1200
gctccggacc cattatcatg ttcgcctcgt acaatcgatt cgatcacgga atctataggg 1260
atgccatgat cgtaaccaca ctggacacgc tgaccagcct cctgggaggt ataacgatat 1320
ttgcgatctt gggcaatctg gcgcacaatc tgcagatcga aaacatcaga gatgtggtgc 1380
gaagtggcac gggattggcc ttcatctcgt atccggatgc catatcaaag ttccaggcgg 1440
tgccgcagct tttctcggtt ctgttcttct tcatgctgtt cgtcctgggc attggatcaa 1500
ttgtggccct gcagagcacc attgtcacca tcatttgcga tcagttcaag ggatggaagt 1560
actggaaggt ggcactgacc acctctgtgt gcggtttcct tatgggtttg gtctacgtta 1620
caccgggcgg acagtggatc ctcactttgg tggacttcta tggcggaacc tatgtggtct 1680
tcatcttggc cattttcgaa ctggctggca tcgtgtgggt ttatggtctg caaaactttt 1740
gcgatgacat cgagtttatg tgcaatcgcc gagtttcact gtactggcga gtatgctggt 1800
ccttcttcac gcccgtcatg atgatcatca tattcatata ctcgatggtc accatcgaac 1860
cgatcaaata tagcgaactg tacttccccg aagccgccaa cattgccggc tggctgctgt 1920
tcgccatcgg tgctgcccag tttccactgt ggggtctttg gtacatctcg cgccatccac 1980
aaggcaccta ctggaagtct ctgaaggcct cgctgaagcc cagcgatcgc tggggtcctg 2040
ccaatccgga aattcggcgc gaatgggtga tcttcaagaa tcagaaggca gcccaaagag 2100
ccacgcagaa ggatacgtcg aaacttggtt tcttctggcg caaagtggcc aatttctgtg 2160
gcagcaacaa gtaaacaaag tccgcgtgga gcggggagca gaggtgggga tatcgtgggg 2220
atatcagaga ttacgactaa ccataaccac ttgttattta tatacgccat tgtcagcctc 2280
tatgaagcca tcacaaatgc gaagcacaat ccttcgaccg aatatatcgc atgtatcaat 2340
aaataaatag ccgttttttt ttttttaatt atatttttgt tattctacgt acaaacttca 2400
tttatgaatg tgataatatt gagcagaaaa aaaaaataaa ttattattat caaagaaaaa 2460
aaaaaaaaaa aaa 2473

<210> 4

<211> 641
<212> PRT
<213> Drosophila melanogaster

<400> 4
Met Glu Leu Lys Gly Val Gln Pro Ser Asn Gly Ser Ser Asn Gly Ser
1               5                   10                  15
Gly Asn Gly Ala Thr Asn Ala Ala Ser Thr Glu Lys Thr Asp Ala Glu
            20                  25                  30
Lys Pro Thr Ala Glu Arg Thr Asn Trp Gly Asn Gly Leu Glu Phe Leu
        35                  40                  45
Met Ser Cys Ile Ser Val Ser Val Gly Leu Gly Asn Val Trp Arg Phe
    50                  55                  60
Pro Phe Thr Ala Tyr Glu Asn Gly Gly Gly Ala Phe Leu Ile Pro Tyr
65                  70                  75                  80
Ile Ile Val Leu Phe Leu Ile Gly Lys Pro Met Tyr Tyr Leu Glu Met
                85                  90                  95
Ile Met Gly Gln Phe Thr Ser Gln Gly Thr Val Lys Ile Trp Ser Val
            100                 105                 110
Val Pro Gly Phe Val Gly Val Gly Tyr Gly Gln Ala Phe Gly Thr Ile
        115                 120                 125
Cys Ile Ile Ser Tyr Tyr Ser Ser Leu Leu Ala Leu Thr Leu Tyr Tyr
    130                 135                 140
Leu Phe Val Ser Phe Gln Ser Glu Leu Pro Trp Ser Tyr Cys Arg Asp
145                 150                 155                 160
Glu Trp Thr Asn Cys Val Asn Ser Arg Pro Gln Glu Tyr Val Asp Asn
                165                 170                 175
Leu Leu Thr Gly Val Ser Leu Ala Asn Glu Ser Ala Arg Asn Leu Ser
            180                 185                 190
Gly Ile Val Ala Asn Asp Glu Thr Glu Lys Leu Gln Ser Ser Ser Glu
        195                 200                 205
Leu Tyr Phe Leu Asn Val Val Ile Lys Glu Lys Leu Asp Ile Ser Asp
    210                 215                 220
Gly Val Gly Asp Pro Asp Trp Lys Leu Thr Leu Ala Leu Phe Val Ala
225                 230                 235                 240
Trp Val Val Ile Phe Leu Val Ile Met Arg Gly Val Lys Ser Ser Gly
                245                 250                 255
Lys Ala Ala Tyr Phe Leu Ala Leu Phe Pro Tyr Val Val Leu Phe Val
            260                 265                 270
Leu Leu Ile Arg Ala Val Thr Leu Glu Gly Ala Arg Asp Gly Ile Leu
        275                 280                 285
Phe Phe Leu Glu Pro Gln Trp Gly Glu Leu Leu Asn Pro Thr Val Trp
    290                 295                 300
Lys Glu Ala Val Val Gln Cys Phe Phe Ser Leu Ala Val Gly Ser Gly
305                 310                 315                 320
Pro Ile Ile Met Phe Ala Ser Tyr Asn Arg Phe Asp His Gly Ile Tyr
                325                 330                 335
Arg Asp Ala Met Ile Val Thr Thr Leu Asp Thr Leu Thr Ser Leu Leu
            340                 345                 350
Gly Gly Ile Thr Ile Phe Ala Ile Leu Gly Asn Leu Ala His Asn Leu
        355                 360                 365
Gln Ile Glu Asn Ile Arg Asp Val Val Arg Ser Gly Thr Gly Leu Ala
    370                 375                 380
Phe Ile Ser Tyr Pro Asp Ala Ile Ser Lys Phe Gln Ala Val Pro Gln
385                 390                 395                 400
Leu Phe Ser Val Leu Phe Phe Phe Met Leu Phe Val Leu Gly Ile Gly
                405                 410                 415
Ser Ile Val Ala Leu Gln Ser Thr Ile Val Thr Ile Ile Cys Asp Gln
            420                 425                 430
Phe Lys Gly Trp Lys Tyr Trp Lys Val Ala Leu Thr Thr Ser Val Cys
        435                 440                 445
Gly Phe Leu Met Gly Leu Val Tyr Val Thr Pro Gly Gly Gln Trp Ile
    450                 455                 460

Leu Thr Leu Val Asp Phe Tyr Gly Gly Thr Tyr Val Val Phe Ile Leu
465                 470                 475                 480
Ala Ile Phe Glu Leu Ala Gly Ile Val Trp Val Tyr Gly Leu Gln Asn
                485                 490                 495
Phe Cys Asp Asp Ile Glu Phe Met Cys Asn Arg Arg Val Ser Leu Tyr
            500                 505                 510
Trp Arg Val Cys Trp Ser Phe Phe Thr Pro Val Met Met Ile Ile Ile
        515                 520                 525
Phe Ile Tyr Ser Met Val Thr Ile Glu Pro Ile Lys Tyr Ser Glu Leu
    530                 535                 540
Tyr Phe Pro Glu Ala Ala Asn Ile Ala Gly Trp Leu Leu Phe Ala Ile
545                 550                 555                 560
Gly Ala Ala Gln Phe Pro Leu Trp Gly Leu Trp Tyr Ile Ser Arg His
                565                 570                 575
Pro Gln Gly Thr Tyr Trp Lys Ser Leu Lys Ala Ser Leu Lys Pro Ser
            580                 585                 590
Asp Arg Trp Gly Pro Ala Asn Pro Glu Ile Arg Arg Glu Trp Val Ile
        595                 600                 605
Phe Lys Asn Gln Lys Ala Ala Gln Arg Ala Thr Gln Lys Asp Thr Ser
    610                 615                 620
Lys Leu Gly Phe Phe Trp Arg Lys Val Ala Asn Phe Cys Gly Ser Asn
625                 630                 635                 640
Lys

<210> 5
<211> 1969
<212> DNA
<213> Drosophila melanogaster

<400> 5
ttcggcacga gggcagacgt gctggcaacg tgcgtcgcag tcgcagcagt ttccgattcg      60
ggttcagatc gagattcggg ttcggattaa cactcgcgca gtgccagttt tgcaggggtt     120
ttgtgcgtga ttggaaaacg tgtcgcctga aagtaataga aaagtgcact gtttgcaggt     180
gaatgcaaat tttaatttta aataaggttc aagagttcgg cgtgaatccg ctggcggcaa     240
tagccaccaa ccaattagaa gataatctga agctcgcatt tctacgactt gttcttcgtg     300
aagtgaccca gaaaaactct gctgaatact aggtcataca gccaaaaaca gaaaaacaaa     360
accgaagcaa aacaaaaacg gaacaaatcg gcatgaactt ggaacaaacc gaatctgggc     420
aattttaatt gagccagcag agtcgcgagt gtttggtttt ctcatctggc cagcaacttc     480
tcacctgatg acccaacatg aagggcatct gcggtgagca gctcaacaat tgcatgctcc     540
tgggcgactg tgagaatagt gaacatggca cagaactgcc cggtgatttt atcggggagc     600
cagtggaaac ggagtccgga ggcggaatgg gcggtagtct ggccctagcc gcccgtcgta     660
tccgaagccg ccaggtgcac agcatggccg tgcgaacggg tagtgcgtat gataccctgg     720
agcgaccata tcggcatgat aaatgtcgtg gtcgttgggc caaatcggcg gacttttact     780
ttgccagctg cacacatgcc ttcagttcgc tgatcttttc ggagctctcc acctttggaa     840
tacttcatgg cggttggctt ttgtttataa tcgcctacct gatgggaatg ctattttact     900
cgctgccaat atttttgatc caagcctttt tggacagttt ttcaagttct ggaaccatat     960
cagcatttcg cgtggcgcca atatttaaag gcattggcta cgccattctg ctcctcaatc    1020
tgggaacgct cacctattac agtatagcag ctgtagtgcc cctcatatat accgtgaatt    1080
caatacaccc agtgataccc tggatgagtt gcaacaatag ttggaatacc caggagtgtt    1140
ccttgcacga gaactacgac gtcgatttag ccgtcgccgt tatcttcacc ttggccctcg    1200
ccatgggcgt gcagtcgtct gtgatccccc tgctgtccca ggtggctgga catggactgt    1260
cctacaccgc cgtttccgga cccgctgtgg tggcctctcc ctgggccgtt cccgccgccc    1320
actggcctgc tgccgtgaac gtggcttcgt ggcctccggc agcgatccac gccgccgctc    1380
ctgccgtgct tgctgcccct gctcccgctg tggttgctgc tcatgctccc tcagtcgtcg    1440
tggccccagt ggctcacagt ggcgtctaca ctgcccagac ccgtggtgcc attcacaccg    1500
ctcctctggc cggacacatc ctccatcaat gccgctcccg cacccggaac cttgtaagga    1560
gtataccgcc ttcaatccct ttgtcgatcg gaccattgcc tctgccacgg atactgccat    1620
tgatggggaa gtcgaacttt agacccctca acacgttatg taaatacatt gtatatatag    1680
ccatatatag acagggtact cgcacctagc acctgaacag gatcctttca tacctgtccc    1740
gaaaaaaagg agtcatcaaa attctcttcc gacctaagca cccggccacg tttgtggttg    1800
cctatttctc tcgcttacct atttatttag catacatttt ccaagcatcc tgtgaaaaaa    1860
ccatcacaag ttttcttcga acggaatgcc aagtgcattc tggaaggaaa tgcttgtaca    1920

tctacataat gccaataaag aaaatgtaac taaaaaaaaa aaaaaaaaa 1969

<210> 6
<211> 403
<212> PRT
<213> Drosophila melanogaster

<400> 6
Met Lys Gly Ile Cys Gly Glu Gln Leu Asn Asn Cys Met Leu Leu Gly
1               5                   10                  15
Asp Cys Glu Asn Ser Glu His Gly Thr Glu Leu Pro Gly Asp Phe Ile
            20                  25                  30
Gly Glu Pro Val Glu Thr Glu Ser Gly Gly Gly Met Gly Gly Ser Leu
        35                  40                  45
Ala Leu Ala Ala Arg Arg Ile Arg Ser Arg Gln Val His Ser Met Ala
    50                  55                  60
Val Arg Thr Gly Ser Ala Tyr Asp Thr Leu Glu Arg Pro Tyr Arg His
65                  70                  75                  80
Asp Lys Cys Arg Gly Arg Trp Ala Lys Ser Ala Asp Phe Tyr Phe Ala
                85                  90                  95
Ser Cys Thr His Ala Phe Ser Ser Leu Ile Phe Ser Glu Leu Ser Thr
            100                 105                 110
Phe Gly Ile Leu His Gly Gly Trp Leu Leu Phe Ile Ile Ala Tyr Leu
        115                 120                 125
Met Gly Met Leu Phe Tyr Ser Leu Pro Ile Phe Leu Ile Gln Ala Phe
    130                 135                 140
Leu Gly Gln Phe Ser Ser Ser Gly Thr Ile Ser Ala Phe Arg Val Ala
145                 150                 155                 160
Pro Ile Phe Lys Gly Ile Gly Tyr Ala Ile Leu Leu Leu Asn Leu Gly
                165                 170                 175
Thr Leu Thr Tyr Tyr Ser Ile Ala Ala Val Val Pro Leu Ile Tyr Thr
            180                 185                 190
Val Asn Ser Ile His Pro Val Ile Pro Trp Met Ser Cys Asn Asn Ser
        195                 200                 205
Trp Asn Thr Gln Glu Cys Ser Leu His Glu Asn Tyr Asp Val Asp Leu
    210                 215                 220
Ala Val Ala Val Ile Phe Thr Leu Ala Leu Ala Met Gly Val Gln Ser
225                 230                 235                 240
Ser Val Ile Pro Leu Leu Ser Gln Val Ala Gly His Gly Leu Ser Tyr
                245                 250                 255
Thr Ala Val Ser Gly Pro Ala Val Val Ala Ser Pro Trp Ala Val Pro
            260                 265                 270
Ala Ala His Trp Pro Ala Ala Val Asn Val Ala Ser Trp Pro Pro Ala
        275                 280                 285
Ala Ile His Ala Ala Ala Pro Ala Val Leu Ala Ala Pro Ala Pro Ala
    290                 295                 300
Val Val Ala Ala His Ala Pro Ser Val Val Val Ala Pro Val Ala His
305                 310                 315                 320
Ser Gly Val Tyr Thr Ala Gln Thr Arg Gly Ala Ile His Thr Ala Pro
                325                 330                 335
Leu Ala Gly His Ile Leu His Gln Cys Arg Ser Arg Thr Arg Asn Leu
            340                 345                 350
Val Arg Ser Ile Pro Pro Ser Ile Pro Leu Ser Ile Gly Pro Leu Pro
        355                 360                 365
Leu Pro Arg Ile Leu Pro Leu Met Gly Lys Ser Asn Phe Arg Pro Leu
    370                 375                 380
Asn Thr Leu Cys Lys Tyr Ile Val Tyr Ile Ala Ile Tyr Arg Gln Gly
385                 390                 395                 400
Thr Arg Thr

<210> 7
<211> 2030

<212> DNA
<213> Drosophila melanogaster

<400> 7
gcacgagcag gtgtgccggc agtggcgagg cgctgctaaa gccatccttg ctgcccatgg 60
cattggaatc gtctgagctc tctccggatc ggaaggcccg cgacaactgg ggcagttcgc 120
tggagttcct gatgtcctgc atcgccctga gcgtgggatt aggcaacgtg tggcggtttc 180
catttacggc cctggaaaac ggcggagggg cattcctaat tccgtacctc gtcgtcctgt 240
ttgtggtggg aaagccgatt tactacatgg agatgctgct gggacagttc tccagtcgtg 300
gcatcgtgca ggtctttgac tttgcgcccc tcatgagagg tgtgggctat gctcagctat 360
tggccctagg cgttttggcc acgtattatg cttcagtgat ggccctaact ctgcgatact 420
ttttcgattc cttcgcatcg gagttgccct ggagcttctg tcgcgaggag tggggtgacg 480
ggtgcgtaag tgcatctggg gggcagcctc ttcagggcca actgtcgcgt aacttcagct 540
cctccacgca actctacttg caacgcatcg tgctgaatga aacggattcc ctggaggagg 600
gcatcggata tcccagtggt agtttggcct taatgctggg catttcgtgg ctaaccgtaa 660
cactaatcat cattcgggga gtgaagagtt caggaaaggc ggcctatgtc ctggccctct 720
ttccgtacgt tgtaatgttt atcctgctcg tgagagcact cacattacca ggtgcctacg 780
acggagttat gtactttctg actccacagt gggagaaact tttggagccg caggtgtggt 840
acaatgccgt cacccaggtg ttcttctccc tggccgtctg tttcggtgtg atcatcatgt 900
actcctcgta caaccgtttc ggtcacaatg tctatcggga tgccaatata gtaaccaccc 960
tggacacatt cacctcactg ctctcgggtg tgattatctt tggaatcctg ggaaacctgg 1020
ctcacgaatc gggcaccaag gacattgcca gtgtggttaa ggcaggtcct ggattggctt 1080
tcatctccta tccggatgcc attgccaagt tcaaaatgtt tcctcaagtg ttttcgctgc 1140
tcttcttcgc catgctcttc atgttgggcg ttggtagcaa tgtgggcatg gtcagctgca 1200
ttatgaccgt gctcaaagat cagttcgtga acgtcaagct gtggattata gtggtcagcc 1260
tttcggtgat aggatttctg gtgggattga tttacatcac tcccggaggt caacatatca 1320
tcacgcttat ggactttcac ggcgtcacct ttgtctccct ggtatcggcc atctttgagc 1380
tcattgccgt gggctggatc tacggcacca aacgtctctg ccaagatgct gaatacatgc 1440
tgaatatcaa gacttcaaat tactatcgga tctgttggtc cattgttact cctttggtta 1500
tgttggtcat cctggtgtac agcttgctta ccatgcgtcc actcagctat aatggtcagg 1560
aatttccatt ggtatataga gttgttggtt ggtgtgtatc gggctgtatt attggccagc 1620
tgttctactg ggcaggttat gcaaacttca agcaaccgaa aggatctttg aagagtcgca 1680
ttaacaattc cattaagccg cattccgatt gggggccttc ggatcccaaa aagctgatgg 1740
attaccaaat gtttttgcga aacaaggatg aggaaaattc caaaaatgtt aatcgccgtt 1800
gtgtttgcta cacagctgga gatcgaattt tcggttaaac caaacttact tttatattat 1860
tttatgtcac ctcaaaaact ttgcatgttt ctcatttaat actgcttaga aaattgtttg 1920
gcacaaggtt ttagttgtac ttaatatatt ttatatacac tttcttattt tcatttatac 1980
gaaatgaaat caaacaaaaa tcaattaagt cgaaaaaaaa aaaaaaaaaa 2030

<210> 8
<211> 593
<212> PRT
<213> Drosophila melanogaster

<400> 8
Met Ala Leu Glu Ser Ser Glu Leu Ser Pro Asp Arg Lys Ala Arg Asp
1               5                   10                  15
Asn Trp Gly Ser Ser Leu Glu Phe Leu Met Ser Cys Ile Ala Leu Ser
            20                  25                  30
Val Gly Leu Gly Asn Val Trp Arg Phe Pro Phe Thr Ala Leu Glu Asn
        35                  40                  45
Gly Gly Gly Ala Phe Leu Ile Pro Tyr Leu Val Val Leu Phe Val Val
    50                  55                  60
Gly Lys Pro Ile Tyr Tyr Met Glu Met Leu Leu Gly Gln Phe Ser Ser
65                  70                  75                  80
Arg Gly Ile Val Gln Val Phe Asp Phe Ala Pro Leu Met Arg Gly Val
                85                  90                  95
Gly Tyr Ala Gln Leu Leu Ala Leu Gly Val Leu Ala Thr Tyr Tyr Ala
            100                 105                 110
Ser Val Met Ala Leu Thr Leu Arg Tyr Phe Phe Asp Ser Phe Ala Ser
        115                 120                 125
Glu Leu Pro Trp Ser Phe Cys Arg Glu Glu Trp Gly Asp Gly Cys Val
    130                 135                 140

Ser Ala Ser Gly Gly Gln Pro Leu Gln Gly Gln Leu Ser Arg Asn Phe
145                 150                 155                 160
Ser Ser Ser Thr Gln Leu Tyr Leu Gln Arg Ile Val Leu Asn Glu Thr
                165                 170                 175
Asp Ser Leu Glu Glu Gly Ile Gly Tyr Pro Ser Gly Ser Leu Ala Leu
            180                 185                 190
Met Leu Gly Ile Ser Trp Leu Thr Val Thr Leu Ile Ile Ile Arg Gly
        195                 200                 205
Val Lys Ser Ser Gly Lys Ala Ala Tyr Val Leu Ala Leu Phe Pro Tyr
    210                 215                 220
Val Val Met Phe Ile Leu Leu Val Arg Ala Leu Thr Leu Pro Gly Ala
225                 230                 235                 240
Tyr Asp Gly Val Met Tyr Phe Leu Thr Pro Gln Trp Glu Lys Leu Leu
                245                 250                 255
Glu Pro Gln Val Trp Tyr Asn Ala Val Thr Gln Val Phe Phe Ser Leu
            260                 265                 270
Ala Val Cys Phe Gly Val Ile Ile Met Tyr Ser Ser Tyr Asn Arg Phe
        275                 280                 285
Gly His Asn Val Tyr Arg Asp Ala Asn Ile Val Thr Thr Leu Asp Thr
    290                 295                 300
Phe Thr Ser Leu Leu Ser Gly Val Ile Ile Phe Gly Ile Leu Gly Asn
305                 310                 315                 320
Leu Ala His Glu Ser Gly Thr Lys Asp Ile Ala Ser Val Val Lys Ala
                325                 330                 335
Gly Pro Gly Leu Ala Phe Ile Ser Tyr Pro Asp Ala Ile Ala Lys Phe
            340                 345                 350
Lys Met Phe Pro Gln Val Phe Ser Leu Leu Phe Phe Ala Met Leu Phe
        355                 360                 365
Met Leu Gly Val Gly Ser Asn Val Gly Met Val Ser Cys Ile Met Thr
    370                 375                 380
Val Leu Lys Asp Gln Phe Val Asn Val Lys Leu Trp Ile Ile Val Val
385                 390                 395                 400
Ser Leu Ser Val Ile Gly Phe Leu Val Gly Leu Ile Tyr Ile Thr Pro
                405                 410                 415
Gly Gly Gln His Ile Ile Thr Leu Met Asp Phe His Gly Val Thr Phe
            420                 425                 430
Val Ser Leu Val Ser Ala Ile Phe Glu Leu Ile Ala Val Gly Trp Ile
        435                 440                 445
Tyr Gly Thr Lys Arg Leu Cys Gln Asp Ala Glu Tyr Met Leu Asn Ile
    450                 455                 460
Lys Thr Ser Asn Tyr Tyr Arg Ile Cys Trp Ser Ile Val Thr Pro Leu
465                 470                 475                 480
Val Met Leu Val Ile Leu Val Tyr Ser Leu Leu Thr Met Arg Pro Leu
                485                 490                 495
Ser Tyr Asn Gly Gln Glu Phe Pro Leu Val Tyr Arg Val Val Gly Trp
            500                 505                 510
Cys Val Ser Gly Cys Ile Ile Gly Gln Leu Phe Tyr Trp Ala Gly Tyr
        515                 520                 525
Ala Asn Phe Lys Gln Pro Lys Gly Ser Leu Lys Ser Arg Ile Asn Asn
    530                 535                 540
Ser Ile Lys Pro His Ser Asp Trp Gly Pro Ser Asp Pro Lys Lys Leu
545                 550                 555                 560
Met Asp Tyr Gln Met Phe Leu Arg Asn Lys Asp Glu Glu Asn Ser Lys
                565                 570                 575
Asn Val Asn Arg Arg Cys Val Cys Tyr Thr Ala Gly Asp Arg Ile Phe
            580                 585                 590
Gly

<210> 9
<211> 2108
<212> DNA
<213> Drosophila melanogaster

<400> 9
   ttcggcacga gacggaacga ataatttcag ttagatttac actggcaata tggatacgac 60
   gcaaaggaac caccaaaatc tagccttcgt gggcgacgac ggacgcagta cggccagcac 120
   agtggagatt tcgaccaaca gtccagccct gcgagataat tccgatgacc aggaggcagc 180
   caaagtgcca gaggagcgtg ccacctgggg caagggcgtg gagtttctga tgtcctgcat 240
   cgccatgtcc gtgggtctgg gaaacgtgtg gcgattcccc tttactgccc tcgataatgg 300
   cggtggcgcc ttcctcatac catatctcat tgtcctgttc ctgattggca aaccgattta 360
   ctatctggaa atggtaattg gccagttttc cagtcggggc tcggttaaag tgttcgattt 420
   gtgcccggcg atgaaaggtg ttggagctgg ccaggctttc caggtgttca tgctcagcac 480
   ttactatgcc gccctggtag cgataattgg ccgatatttc attgaatcat ttcgcaaccc 540
   attgccctgg tccacatgcc gtgcggaatg gggtatccac tgcattaact cagcccccga 600
   tgcgagtaat tggtctcaac tggaaagcaa tgatcagcgc ccgcaaaatt acacaatgaa 660
   atcacaaaac gatcgggtca tcaccagttc ggaatggtac tttgttaagg aagtgctgca 720
   cgagaaacca aatatcgagg aaggaatcgg cctaccaaac tgggagttgg tcattggact 780
   ttttatcgcc tggtcctgtg tgtttttcat tattcgtcgc ggagtgaaga gttcgggaaa 840
   ggcatcctac ttcctggccc ttttcccgta cgtcatcatg ggtgttcttc tggtgcgagc 900
   tgtaactctg ccgggatcaa tagacggtat atactacttt ataaagccgc aatggggaaa 960
   gattttggac ccgaaggttt ggtacgcggc tgtaactcag tgtttctact ccttgtccgt 1020
   ttgtttcggc aacatcatca tgtactcctc gttcaacaag ttcgggcaca atgtacacag 1080
   ggatgcagca attgtaacgg gtctggacac tatgacctct ctgctagctg gattcacgat 1140
   attcggtatc ctcggccatc tggcccacga aatcggaacc gatgacatcg gctcagttgt 1200
   gaagggcggt gccggattgg ccttcatctc atatcccgat gccattgcca agttcaagaa 1260
   tctgccacaa atcttctcag tgctgttctt cctcatgctc ttcgtgttgg gcataggatc 1320
   gaatatagcc atgacctcct gctcggtgac cgccattcgg gatcggtttc ccaactttgg 1380
   gcagtggcag tgctcgctgc tcatcgctgt agtcagtttc tttattggat taatgtatat 1440
   aacaccgggc ggtcagtata tgctgactct tgtggacttt ttcggcgcct caatgattgc 1500
   tttggtactg ggaatcgccg agctgtacac cattggatgg atttatggca cagatcgcct 1560
   gtgcaaggac attgagttca tgctgggtcg caaagtgggc ctgtactgga ggctctgctg 1620
   gagcatcatt accccgctga tcatgactgt tattctgatc tatttctatg cgacctacca 1680
   acctctaaca tacaacaata tcgtctatcc aaattggtct tactcaattg gttggctaat 1740
   aacggccttc ggtattcttc agctgcccat ttggatgatc gtggccattg ttcgagatcc 1800
   aggtcagact ttgggtgcaa aaatccgtgg agcctttacg ccgaaaaaga attggggacc 1860
   cagtgaccct ctgctccggg aacagtatca caaggaaatc gagaatgagt tgaccccgaa 1920
   acgtggtcag ggaatttggg ctgccatcaa gcaaaacatc tttggttgat ccatcatcag 1980
   tgagaacaaa ctaaccacaa tcgctcatgt aaataatctt attttaagag aaaagaattt 2040
   aaaagatctt gtataaatct taagtctgcc aataaacttg ataagtacgt aaaaaaaaaa 2100
   aaaaaaaa 2108

<210> 10
<211> 639
<212> PRT
<213> Drosophila melanogaster

<400> 10
Met Asp Thr Thr Gln Arg Asn His Gln Asn Leu Ala Phe Val Gly Asp
1               5                   10                  15
Asp Gly Arg Ser Thr Ala Ser Thr Val Glu Ile Ser Thr Asn Ser Pro
            20                  25                  30
Ala Leu Arg Asp Asn Ser Asp Asp Gln Glu Ala Ala Lys Val Pro Glu
        35                  40                  45
Glu Arg Ala Thr Trp Gly Lys Gly Val Glu Phe Leu Met Ser Cys Ile
    50                  55                  60
Ala Met Ser Val Gly Leu Gly Asn Val Trp Arg Phe Pro Phe Thr Ala
65                  70                  75                  80
Leu Asp Asn Gly Gly Gly Ala Phe Leu Ile Pro Tyr Leu Ile Val Leu
                85                  90                  95
Phe Leu Ile Gly Lys Pro Ile Tyr Tyr Leu Glu Met Val Ile Gly Gln
            100                 105                 110
Phe Ser Ser Arg Gly Ser Val Lys Val Phe Asp Leu Cys Pro Ala Met
        115                 120                 125
Lys Gly Val Gly Ala Gly Gln Ala Phe Gln Val Phe Met Leu Ser Thr
    130                 135                 140

Tyr Tyr Ala Ala Leu Val Ala Ile Ile Gly Arg Tyr Phe Ile Glu Ser
145                 150                 155                 160
Phe Arg Asn Pro Leu Pro Trp Ser Thr Cys Arg Ala Glu Trp Gly Ile
                165                 170                 175
His Cys Ile Asn Ser Ala Pro Asp Ala Ser Asn Trp Ser Gln Leu Glu
            180                 185                 190
Ser Asn Asp Gln Arg Pro Gln Asn Tyr Thr Met Lys Ser Gln Asn Asp
        195                 200                 205
Arg Val Ile Thr Ser Ser Glu Trp Tyr Phe Val Lys Glu Val Leu His
    210                 215                 220
Glu Lys Pro Asn Ile Glu Glu Gly Ile Gly Leu Pro Asn Trp Glu Leu
225                 230                 235                 240
Val Ile Gly Leu Phe Ile Ala Trp Ser Cys Val Phe Phe Ile Ile Arg
                245                 250                 255
Arg Gly Val Lys Ser Ser Gly Lys Ala Ser Tyr Phe Leu Ala Leu Phe
            260                 265                 270
Pro Tyr Val Ile Met Gly Val Leu Leu Val Arg Ala Val Thr Leu Pro
        275                 280                 285
Gly Ser Ile Asp Gly Ile Tyr Tyr Phe Ile Lys Pro Gln Trp Gly Lys
    290                 295                 300
Ile Leu Asp Pro Lys Val Trp Tyr Ala Ala Val Thr Gln Cys Phe Tyr
305                 310                 315                 320
Ser Leu Ser Val Cys Phe Gly Asn Ile Ile Met Tyr Ser Ser Phe Asn
                325                 330                 335
Lys Phe Gly His Asn Val His Arg Asp Ala Ala Ile Val Thr Gly Leu
            340                 345                 350
Asp Thr Met Thr Ser Leu Leu Ala Gly Phe Thr Ile Phe Gly Ile Leu
        355                 360                 365
Gly His Leu Ala His Glu Ile Gly Thr Asp Asp Ile Gly Ser Val Val
    370                 375                 380
Lys Gly Gly Ala Gly Leu Ala Phe Ile Ser Tyr Pro Asp Ala Ile Ala
385                 390                 395                 400
Lys Phe Lys Asn Leu Pro Gln Ile Phe Ser Val Leu Phe Phe Leu Met
                405                 410                 415
Leu Phe Val Leu Gly Ile Gly Ser Asn Ile Ala Met Thr Ser Cys Ser
            420                 425                 430
Val Thr Ala Ile Arg Asp Arg Phe Pro Asn Phe Gly Gln Trp Gln Cys
        435                 440                 445
Ser Leu Leu Ile Ala Val Val Ser Phe Phe Ile Gly Leu Met Tyr Ile
    450                 455                 460
Thr Pro Gly Gly Gln Tyr Met Leu Thr Leu Val Asp Phe Phe Gly Ala
465                 470                 475                 480
Ser Met Ile Ala Leu Val Leu Gly Ile Ala Glu Leu Tyr Thr Ile Gly
                485                 490                 495
Trp Ile Tyr Gly Thr Asp Arg Leu Cys Lys Asp Ile Glu Phe Met Leu
            500                 505                 510
Gly Arg Lys Val Gly Leu Tyr Trp Arg Leu Cys Trp Ser Ile Ile Thr
        515                 520                 525
Pro Leu Ile Met Thr Val Ile Leu Ile Tyr Phe Tyr Ala Thr Tyr Gln
    530                 535                 540
Pro Leu Thr Tyr Asn Asn Ile Val Tyr Pro Asn Trp Ser Tyr Ser Ile
545                 550                 555                 560
Gly Trp Leu Ile Thr Ala Phe Gly Ile Leu Gln Leu Pro Ile Trp Met
                565                 570                 575
Ile Val Ala Ile Val Arg Asp Pro Gly Gln Thr Leu Gly Ala Lys Ile
            580                 585                 590
Arg Gly Ala Phe Thr Pro Lys Lys Asn Trp Gly Pro Ser Asp Pro Leu
        595                 600                 605
Leu Arg Glu Gln Tyr His Lys Glu Ile Glu Asn Glu Leu Thr Pro Lys
    610                 615                 620
Arg Gly Gln Gly Ile Trp Ala Ala Ile Lys Gln Asn Ile Phe Gly
625                 630                 635

<210> 11
<211> 2874
<212> DNA
<213> Drosophila melanogaster

<400> 11
tttgtgactc tgaactaagt caaaaacgag agcaaaagac gcagtgtttg attgtcctag 60
actatccgtt tgtcgtcctt aatacttatt aaaatttatt gtcgaaatgt acacaaactc 120
cgcaagcgat ggtgacggag gaggagatgt gtgtaagcac gaatctattg aaatgagtaa 180
agaattgggc cacacaacca gcttgcagtc cagctcacct acaactacgg acacgtccaa 240
taaacagcag ttggtaaaaa ttgagcaact ccccgatcgc ggatcatggt cttccaaaat 300
ggatttcata ctatcagtag tgggcctagc tataggtttg ggaaacgttt ggcgatttcc 360
ctatttgtgc tacaaaaatg gaggcggtgc atttatatta ccatacatta ttacactgtt 420
tttggcaggt attccaatgt ttttcatgga actagctctt ggtcaaatgt tgacgattgg 480
tggactcggt gtattcaaaa ttgcacccat atttaaagga attgggtatg ctgctgctgt 540
aatgtcttgt tggatgaatg tatactatat tgtcattctt gcgtgggcgg tattttactt 600
ctttatgtcg atgagagcag atgttccgtg gagaacgtgc aacaattggt ggaacacagt 660
caactgtgtc agtcaatatg aaaggaaaaa tttgcattgc tgggataaga ttatcaacgg 720
caccacgcaa aaggtttgct ccgtatctgc tcttaacata acatctttag aactcacaga 780
tccagtcaaa gagttttggg agcggcgagc tcttcaaata tcgcatggta ttgaggagat 840
tggaaatatt cgctgggagt tggctggaac tttattactt gtttggatcc tctgctactt 900
ttgtatttgg aagggagtaa aatggacagg gaaagtagta tattttactg ccttgtttcc 960
ctacgttctc ctgacgattc tactggttcg cggaattaca ttacctggcg ctttagaagg 1020
cattaaattt tatataattc caaacttttc aaagttgact aattctgagg tgtggatcga 1080
cgctgtgacg caaatatttt tctcatacgg gcttggactt ggtactttgg tggcgttagg 1140
aagttacaac aagtttacca ataacgtcta caaggacgcg cttattgtct gcacagttaa 1200
ttctagcacc agtatgtttg ctggattcgt aatattttcg gtaataggat ttatggctca 1260
cgagcaacag cgaccagtgg ctgatgtagc cgcatcagga cctggccttg catttcttgt 1320
ctatccatcg gcagttctgc agttaccagg gtctccaatg tggtcttgcc tcttcttctt 1380
catgcttctg ctcattggat tggactcaca gttttgtaca atggagggct ttatcactgc 1440
cattattgat gaatggccgc agttgctgcg gaagcggaaa gaaatattta ttgctattgt 1500
atgcgctctt agctacttag tgggcttaac ctgtattaca caaggtggca tgtatatttt 1560
tcaaatattg gattcatatg cagttagcgg cttttgctta ctgtggctca tattctttga 1620
atgtgtttca atatcttggt gctatggagt ggatcgattt tatgacggaa taaaggatat 1680
gatcggatat tatcccactg tttggtggaa gttctgctgg tgtgtcacaa cgcccgctat 1740
atgcttggga gtgtttttct ttaacatagt tcagtggact cctgttaagt atctggacta 1800
cagctatcca tggtgggcgc atgcttttgg ttggttcacg gcgttgtcat ccatgcttta 1860
tataccactt tacatgtttt ggttgtggaa acgaactcct ggtgaattga gtgagaaaat 1920
tcgagctctt gtacggatag acgaggatgt aacccggtta cgtgagaaaa tgctgagaga 1980
agcttatgcc aaggagatag aatttaattc tttatagacg tttttaagtt atctcatatt 2040
gtttataagg ttccctttgt ttggccattc aatcagctaa atttatttaa attaatttc 2100
tacaatttgg aaaaagacaa cttattttgc aagttttcct tttcttaaaa ttaacttccc 2160
tattttgaca tttgcaatga atcattgtaa cagttttata atttaattta tgttcccttt 2220
ataactcaga actagtcttt ctaatcaatc gcaaggagtc aaggtttttc aatggttatt 2280
gcagtttttt tgtcgtactt acttaaagtg gtatgccaga aatatctaga gtaattagaa 2340
tcacgaattt ctgaatattc tgcttctgaa gcattaacat atatatgaag aatccatgca 2400
gttctaaaag ccatttgtcc ctcatgctca attttgtgaa tcacgccaca gagacaaatt 2460
ttatttttat actattactt ccgttgtaag ttttagtccg cagaaataga cccaggatgt 2520
gttttgcatg acttgtcaag aatgacttct ctgatctgca tggtttcaac atgtcaacgg 2580
caaaatcaaa ctgtggcctt atcagtgaga taaatatctt ttttcaaaac tattactgag 2640
tagaatgggg tttgagattt agttgtgttt agaaataaga tatattatcc gcaatgaaat 2700
tatgtaatgt aatgtaatgt aataaggttg acaccatata tatacagaac aattttgaca 2760
atgtcttgac agtgcttcag gcattgttat gtaatttatc tataaaatgt caatatgttg 2820
cttaacgtgt caaataaatt gaatatctat ccacacaaaa aaaaaaaaaa aaaa 2874

<210> 12
<211> 636
<212> PRT
<213> Drosophila melanogaster

<400> 12
Met Tyr Thr Asn Ser Ala Ser Asp Gly Asp Gly Gly Gly Asp Val Cys
1               5                   10                  15

Lys His Glu Ser Ile Glu Met Ser Lys Glu Leu Gly His Thr Thr Ser
            20                  25                  30
Leu Gln Ser Ser Ser Pro Thr Thr Thr Asp Thr Ser Asn Lys Gln Gln
        35                  40                  45
Leu Val Lys Ile Glu Gln Leu Pro Asp Arg Gly Ser Trp Ser Ser Lys
    50                  55                  60
Met Asp Phe Ile Leu Ser Val Val Gly Leu Ala Ile Gly Leu Gly Asn
65                  70                  75                  80
Val Trp Arg Phe Pro Tyr Leu Cys Tyr Lys Asn Gly Gly Gly Ala Phe
                85                  90                  95
Ile Leu Pro Tyr Ile Ile Thr Leu Phe Leu Ala Gly Ile Pro Met Phe
            100                 105                 110
Phe Met Glu Leu Ala Leu Gly Gln Met Leu Thr Ile Gly Gly Leu Gly
        115                 120                 125
Val Phe Lys Ile Ala Pro Ile Phe Lys Gly Ile Gly Tyr Ala Ala Ala
    130                 135                 140
Val Met Ser Cys Trp Met Asn Val Tyr Tyr Ile Val Ile Leu Ala Trp
145                 150                 155                 160
Ala Val Phe Tyr Phe Phe Met Ser Met Arg Ala Asp Val Pro Trp Arg
                165                 170                 175
Thr Cys Asn Asn Trp Trp Asn Thr Val Asn Cys Val Ser Gln Tyr Glu
            180                 185                 190
Arg Lys Asn Leu His Cys Trp Asp Lys Ile Ile Asn Gly Thr Thr Gln
        195                 200                 205
Lys Val Cys Ser Val Ser Ala Leu Asn Ile Thr Ser Leu Glu Leu Thr
    210                 215                 220
Asp Pro Val Lys Glu Phe Trp Glu Arg Arg Ala Leu Gln Ile Ser His
225                 230                 235                 240
Gly Ile Glu Glu Ile Gly Asn Ile Arg Trp Glu Leu Ala Gly Thr Leu
                245                 250                 255
Leu Leu Val Trp Ile Leu Cys Tyr Phe Cys Ile Trp Lys Gly Val Lys
            260                 265                 270
Trp Thr Gly Lys Val Val Tyr Phe Thr Ala Leu Phe Pro Tyr Val Leu
        275                 280                 285
Leu Thr Ile Leu Leu Val Arg Gly Ile Thr Leu Pro Gly Ala Leu Glu
    290                 295                 300
Gly Ile Lys Phe Tyr Ile Ile Pro Asn Phe Ser Lys Leu Thr Asn Ser
305                 310                 315                 320
Glu Val Trp Ile Asp Ala Val Thr Gln Ile Phe Phe Ser Tyr Gly Leu
                325                 330                 335
Gly Leu Gly Thr Leu Val Ala Leu Gly Ser Tyr Asn Lys Phe Thr Asn
            340                 345                 350
Asn Val Tyr Lys Asp Ala Leu Ile Val Cys Thr Val Asn Ser Ser Thr
        355                 360                 365
Ser Met Phe Ala Gly Phe Val Ile Phe Ser Val Ile Gly Phe Met Ala
    370                 375                 380
His Glu Gln Gln Arg Pro Val Ala Asp Val Ala Ala Ser Gly Pro Gly
385                 390                 395                 400
Leu Ala Phe Leu Val Tyr Pro Ser Ala Val Leu Gln Leu Pro Gly Ser
                405                 410                 415
Pro Met Trp Ser Cys Leu Phe Phe Phe Met Leu Leu Leu Ile Gly Leu
            420                 425                 430
Asp Ser Gln Phe Cys Thr Met Glu Gly Phe Ile Thr Ala Ile Ile Asp
        435                 440                 445
Glu Trp Pro Gln Leu Leu Arg Lys Arg Lys Glu Ile Phe Ile Ala Ile
    450                 455                 460
Val Cys Ala Leu Ser Tyr Leu Val Gly Leu Thr Cys Ile Thr Gln Gly
465                 470                 475                 480
Gly Met Tyr Ile Phe Gln Ile Leu Asp Ser Tyr Ala Val Ser Gly Phe
                485                 490                 495
Cys Leu Leu Trp Leu Ile Phe Phe Glu Cys Val Ser Ile Ser Trp Cys
            500                 505                 510

Tyr Gly Val Asp Arg Phe Tyr Asp Gly Ile Lys Asp Met Ile Gly Tyr
        515                 520                 525
Tyr Pro Thr Val Trp Trp Lys Phe Cys Trp Cys Val Thr Thr Pro Ala
    530                 535                 540
Ile Cys Leu Gly Val Phe Phe Phe Asn Ile Val Gln Trp Thr Pro Val
545                 550                 555                 560
Lys Tyr Leu Asp Tyr Ser Tyr Pro Trp Trp Ala His Ala Phe Gly Trp
                565                 570                 575
Phe Thr Ala Leu Ser Ser Met Leu Tyr Ile Pro Leu Tyr Met Phe Trp
            580                 585                 590
Leu Trp Lys Arg Thr Pro Gly Glu Leu Ser Glu Lys Ile Arg Ala Leu
        595                 600                 605
Val Arg Ile Asp Glu Asp Val Thr Arg Leu Arg Glu Lys Met Leu Arg
    610                 615                 620
Glu Ala Tyr Ala Lys Glu Ile Glu Phe Asn Ser Leu
625                 630                 635